(19) Europäisches Patentamt
European Patent Office
Office européen des brevets

(11) EP 0 556 720 B1

(12) EUROPEAN PATENT SPECIFICATION

(45) Date of publication and mention of the grant of the patent:
17.09.1997 Bulletin 1997/38

(51) Int Cl.6: **C07D 311/30**, C07D 405/10, C07D 413/10, A61K 31/35

(21) Application number: 93102139.8

(22) Date of filing: 11.02.1993

# (54) 5-Aminoflavone derivatives

5-Aminoflavon-Derivate

Dérivés de la 5-aminoflavone

(84) Designated Contracting States:
DE FR GB IT

(30) Priority: 14.02.1992 JP 28113/92

(43) Date of publication of application:
25.08.1993 Bulletin 1993/34

(73) Proprietor: KYOWA HAKKO KOGYO CO., LTD.
Chiyoda-ku, Tokyo-to (JP)

(72) Inventors:
- Akama, Tsutomu
Sunto-gun, Shizuoka-ken (JP)
- Shida, Yasushi
Mishima-shi, Shizuoka-ken (JP)
- Ikeda, Shun-ichi
Numazu-shi, Shizuoka-ken (JP)
- Kasai, Masaji
Fujisawa-shi, Kanagawa-ken (JP)
- Ishida, Hiroyuki
Sunto-gun, Shizuoka-ken (JP)
- Kimura, Uichiroi
Sunto-gun, Shizuoka-ken (JP)
- Gomi, Katsushige
Susono-shi, Shizuoka-ken (JP)
- Saito, Hiromitsu
Mishima-shi, Shizuoka-ken (JP)
- Ueno, Kimihisa
Sunto-gun, Shizuoka-ken (JP)

(74) Representative: VOSSIUS & PARTNER
Postfach 86 07 67
81634 München (DE)

(56) References cited:
EP-A- 0 374 789

- PATENT ABSTRACTS OF JAPAN vol. 14, no. 377 (C-748)(4320), 15 August 1990
- INDIAN JOURNAL OF CHEMISTRY vol. 1, no. 11, November 1963, pp. 477-479, K.C. JOSHI et al.
- Schröder et al. "ARZNEIMITTELCHEMIE I", Georg Thieme Verlag, Stuttgart, DE, 1976, pp. 24-39

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates to novel 5-aminoflavones having antibacterial activity and antitumor activity.

As derivatives having an amino group at 5-position of flavone (2-phenyl-4H-benzopyran-4-one) and halogen at 8-position of flavone, there is disclosed a compound having anti-cellular activity (Chem. Absts., 113, 171775n (1990)). However, the compound does not have an amino group at 4'-position. As derivatives having an amino group at 5-position and 4'-position, there is disclosed a compound having anti-cellular activity (EP-A-374789). However, the compound does not have halogen at 6- and/or 8-position. As derivatives having an amino group at 5-position, there are disclosed a compound having a hydroxy group at 6-position (Chem. Abst., 41, 120f (1947)), a compound having antiviral activity and an alkoxy group at 3-posititon (EP-A-233105), a compound having anti-allergy activity and the like (GB-A-1461777) and a compound having a methyl group at 7-position [Arch. Pharm. (Weinheim), 322, 589 (1989)]. Further, there is disclosed a compound having halogen at 6- and/or 8-position and an amino group at 4'-position (Indian J. Chem., 1, 477 (1963)).

The main object of the present invention is to provide novel 5-aminoflavone derivatives having antibacterial activity and antitumor activity.

This object has been achieved by the provision of 5-aminoflavones (hereinafter referred to as compound (I); a compound having another compound number corresponds to the compound represented by the formula of the same number) represented by the formula (I):

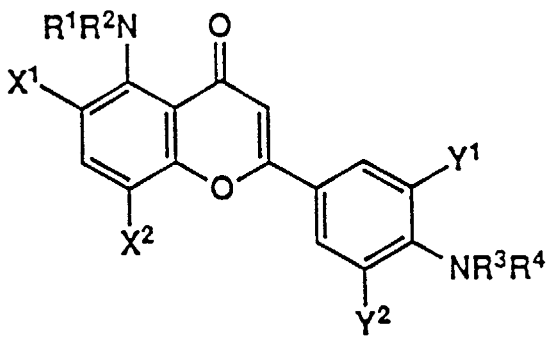

(I)

wherein $R^1$, $R^2$, $R^3$ and $R^4$ are the same or different and represent hydrogen, substituted or unsubstituted lower alkyl, lower alkenyl, halogen-substituted or unsubstituted lower alkanoyl or lower alkoxycarbonyl; and

$X^1$, $X^2$, $Y^1$ and $Y^2$ are the same or different and represent hydrogen or halogen;
provided that at least one of $X^1$ and $X^2$ represents halogen.

In the formula (I), halogen represents fluorine, chlorine, bromine and iodine. Alkyl part of lower alkyl and lower alkoxycarbonyl represents straight or branched alkyls having 1 to 8 carbon atoms, for example, methyl, ethyl, propyl, isopropyl, butyl, isobutyl, sec-butyl, tert-butyl, pentyl, neopentyl, hexyl, isohexyl, heptyl, isoheptyl and octyl. Substituted lower alkyl may have the same or different 1 to 3 substituents. Examples of the substituents are hydroxy, lower alkoxy, carboxy, lower alkoxycarbonyl, lower alkylthio, halogen, amino, mono- or di-alkylsubstituted amino, phthalimido and heterocyclic groups having nitrogen atom such as pyrrolidinyl, piperidino, piperazinyl, morpholino, pyrrolyl, imidazolyl, pyrazolyl and indolyl. Alkyl part of lower alkoxy, lower alkoxycarbonyl, lower alkylthio and alkylsubstituted amino is the same as defined for the above lower alkyl. Halogen is the same as defined for the above halogen. Lower alkenyl is straight or branched alkenyls having 2 to 6 carbon atoms, for example, vinyl, allyl, isopropenyl, 4-pentenyl and 5-hexenyl. Lower alkanoyl is straight or branched alkanoyls having 1 to 6 carbon atoms, for example, formyl, acetyl, propionyl, butyryl, valeryl, pivaloyl and pentanoyl. Lower alkanoyl may have the same or different 1 to 6 substituents of halogen. Example of halogenated alkanoyl is trifluoroacetyl.

As pharmacologically acceptable salts of the compound (I), there are pharmacologically acceptable acid or base addition salts, for example, inorganic acid salts such as hydrochloride, hydrobromide, sulfate and phosphate and organic acid salts such as oxalate, acetate, malonate, succinate, fumarate, maleate, tartrate and citrate as well as base addition salts such as sodium salt and potassium salt.

Then, a process for producing the compound (I) is explained below.

In addition, in the process described below, when the defined groups may be changed under the process conditions or are not suitable for the process, such the inconvenience can be avoided by a method usually used in organic synthetic chemistry, for example, protection and deprotection of functional groups .

Preparation 1

A compound (Ia) in which $X^1$ and/or $X^2$ are chlorine, bromine or iodine in the compound (I) can be produced according to the following Scheme.

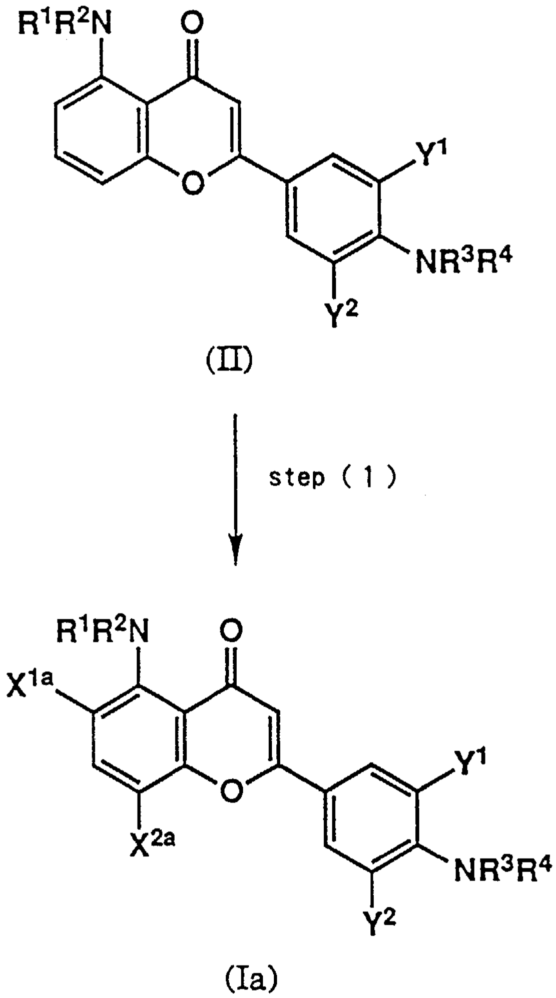


wherein $X^{1a}$ and $X^{2a}$ represent hydrogen, chlorine, bromine or iodine provided that $X^{1a}$ and $X^{2a}$ are not concurrently hydrogen, and $R^1$, $R^2$, $R^3$, $R^4$, $Y^1$ and $Y^2$ are the same as defined above.

Step (1)

A compound (Ia) is prepared by reacting a compound (II) obtained by the known method (EP-A-374789) or modified method thereof with a halogenating agent such as chlorine, bromine, iodine, N-chlorosuccinimide, N-bromosuccinimide and N-iodosuccinimide in acetic acid or dioxane. The reaction is carried out at a temperature from room temperature to the boiling point of the solvent. The reaction completes in 1 to 20 hours.

Preparation 2

A compound (Ib) in which $X^1$ and/or $X^2$ are fluorine in the compound (I) can be prepared according to the following Scheme.

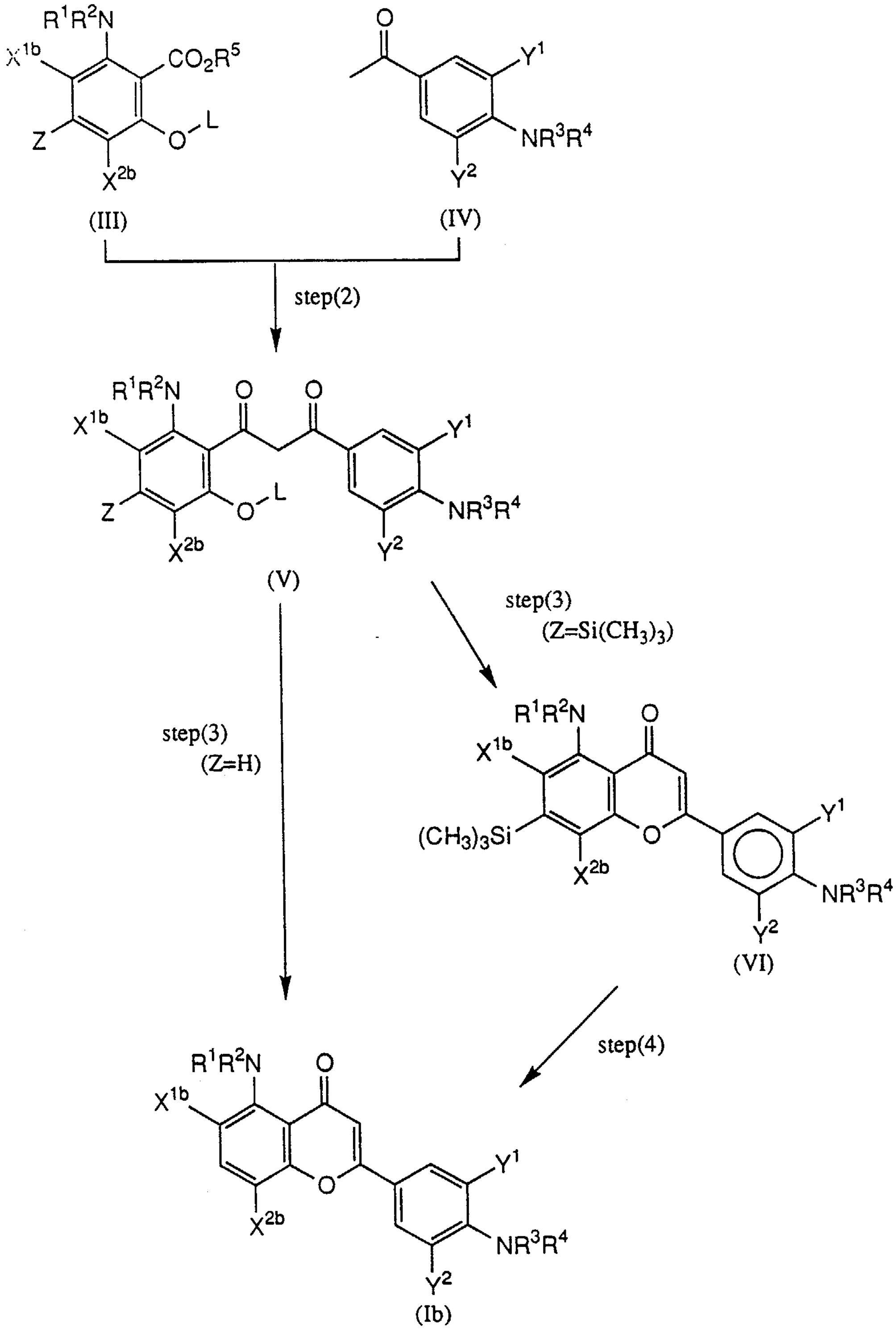


wherein $X^{1b}$ and $X^{2b}$ represent hydrogen or fluorine provided that $X^{1b}$ and $X^{2b}$ are not concurrently hydrogen, $R^5$ represents lower alkyl, Z represents hydrogen or trimethylsilyl, L represents a protecting group for hydroxy, and $R^1$, $R^2$, $R^3$, $R^4$, $Y^1$ and $Y^2$ are the same as defined above.

Lower alkyl is the same as defined for the above alkyl, and examples of L are tetrahydropyranyl and methoxymethyl .

Step (2)

A compound (V) can be obtained by condensing a compound (III) prepared by Reference Examples 1 to 5 with a compound (IV) in the presence of 1 to 3 equivalents of a base in an inert solvent. As bases, sodium hydride, potassium hydride, sodium methoxide, sodium ethoxide, potassium tert-butoxide and the like are used. As suitable inert solvents, ethers such as tetrahydrofuran and dioxane, alcohols such as methanol and ethanol, toluene and dimethylformamide can be used. They can be used sole or in combination with each other. The reaction is normally carried out under warming, preferably, at a temperature of from 40 °C to the boiling point of the solvent employed. The reaction normally completes in 1 to 12 hours.

Step (3)

When Z of a compound (III) is hydrogen, a compound (Ib) can be obtained by treating the compound (V) prepared in Step (2) with hydrochloric acid or sulfuric acid at a concentration of 0.1 to 2N in a lower alcohol such as methanol and ethanol, ether such as dioxane and tetrahydrofuran or acetic acid at a temperature of from 0 to 50 °C for 5 to 12 hours to carry out deprotection and cyclization at the same time.

Step (4)

When Z of a compound (III) is trimethylsilyl, a compound (Ib) can be obtained by treating the compound (VI) prepared by the same procedures as those in the above Step (3) with 1 to 2 equivalents of tetrabutylammonium fluoride in tetrahydrofuran at a temperature of from 0 °C to room temperature for 0.5 to 12 hours to carry out desilylation.

Some compounds thus obtained can be used as an intermediate to prepare novel derivatives (I).

For example, when a compound (Ic) wherein 5-position is an amino group ($R^1$=$R^2$=H) in the compound (I) is desired, it can be obtained by hydrolyzing a compound (I) wherein $R^1$ and/or $R^2$ are lower alkanoyl according to a conventional method. For example, when $R^1$ or $R^2$ is pivaloyl, the compound (Ic) can be obtained by reacting the compound with an acid such as hydrochloric acid, hydrobromic acid and sulfuric acid, if necessary, in a lower alcohol or a water-miscible inert solvent at a temperature of from 0 °C to the boiling point of the solvent, normally, for 0.1 to 10 hours. As lower alcohols, there are those described in Step (2). As water-miscible inert solvents, there are tetrahydrofuran, dioxane, dimethoxyethane, acetic acid and the like. The acid is used at 0.1 to 10N in an amount of 0.1 to 10 times volume relative to that of the solvent.

When a compound (Id) wherein 4'-position is an amino group ($R^3$=$R^4$=H) in the compound (I) is desired, it can be obtained by starting from a compound (I) wherein $R^3$ and/or $R^4$ are lower alkanoyl under the almost same conditions as those for the compound (Ic).

A compound (Ie) wherein one of $R^3$ and $R^4$ is hydrogen and the other is substituted or unsubstituted lower alkyl or lower alkenyl can be obtained by reacting a compound (I) wherein one of $R^3$ and $R^4$ is hydrogen and the other is acetyl, tert-butoxycarbonyl or the like with 1 to 20 equivalents of substituted or unsubstituted lower alkyl or lower alkenyl halide (referred to as alkyl halide hereinafter) in the presence of a from equal to excess base in an inert solvent at a temperature of from room temperature to 120 °C for 1 to 24 hours and removing acetyl, tert-butoxycarbonyl or the like. Halogen in alkyl halide represents chlorine, bromine or iodine. When halogen is chlorine or bromine, the reaction is promoted, in some cases, using as a catalyst from 0.05 to 1 equivalents of sodium iodide or potassium iodide. As bases, there are sodium hydride, potassium hydride, sodium carbonate, potassium carbonate and the like. As inert solvents, there are dimethylformamide, dimethyl sulfoxide, tetrahydrofuran, dioxane and the like. Then, a compound (Ie) can be obtained by treating the product with an acid such as hydrochloric acid and trifluoroacetic acid in a lower alcohol or a water-miscible inert solvent at a temperature of from 0 °C to the boiling point of the solvent, normally, for 0.1 to 10 hours. As lower alcohols, there are those described in Step (3). As inert solvents which are miscible with water, there are tetrahydrofuran, dioxane, acetic acid and the like. The acid is used at 0.1 to 10N in an amount of 0.1 to 10 times volume relative to that of the solvent.

A compound (If) wherein $R^1$ or $R^2$ is substituted or unsubstituted lower alkyl or lower alkenyl in the compound (I) can be obtained by starting from a compound (Ic) and alkyl halide according to the same procedures as those in the process for producing the above compound (Ie).

A compound (Ig) wherein $R^1$ or $R^2$ is lower alkanoyl in the compound (I) can be obtained by reacting the corresponding compound (Ic) and the corresponding lower aliphatic carboxylic acid or the reactive derivative of the carboxylic acid such as acid anhydride, acid chloride and acid bromide, if necessary, in the presence of a base. As bases, there are triethylamine, pyridine, dimethylaminopyridine, sodium carbonate, potassium carbonate and the like. As solvents for the reaction, there are dimethylformamide, dimethyl sulfoxide, tetrahydrofuran, dioxane and the like. Alternatively, carboxylic acid or the reactive derivative thereof may be used also as a solvent. The reaction completes in 0.5 to 10 hours at a temperature of from ice-cooling to the boiling point of the solvent or carboxylic acid or the reactive derivative

thereof employed.

A compound (Ih) wherein $R^3$ or $R^4$ is lower alkanoyl in the compound (I) can be obtained by starting from a compound (I) wherein $R^3$ and $R^4$ are hydrogen under the almost same conditions as those for the above compound (Ig).

A compound (Ii) wherein $R^3$ or $R^4$ is amino-substituted alkyl in the compound (I) can be obtained by reacting a compound (Ie) wherein $R^3$ or $R^4$ is the corresponding phthalimido-substituted alkyl with hydrazine monohydrate in an inert solvent at a temperature of from 0 °C to the boiling point of the solvent. As inert solvents, there are lower alcohols such as methanol and ethanol, dimethylformamide and chloroform. They may be used sole or in combination with each other. Hydrazine monohydrate is used in an -amount of 1 to 20 equivalents relative to that of the compound (Ie). Normally, the reaction completes in 0.1 to 5 hours.

Alternatively, a compound (Ii) can also be obtained by reacting a compound (Ie) wherein $R^3$ and $R^4$ are the corresponding chlorine, bromine or iodine-substituted alkyl with 1 to 10 equivalents of sodium azide in dimethylformamide at a temperature of from room temperature to 70 °C to give an azide, reacting the azide with 1 to 10 equivalents of triphenylphosphine, and then hydrolyzing the resulting product. The reaction is carried out in tetrahydrofuran, ethyl acetate, acetonitrile and the like at room temperature. The reaction is complete in 1 to 40 hours.

A compound (Ij) wherein $R^1$ or $R^2$ is amino-substituted alkyl in the compound (I) can be obtained from a compound (If) wherein $R^1$ or $R^2$ is the corresponding phthalimido, chlorine, bromine or iodine-substituted alkyl under the almost same conditions as those for the compound (Ii).

A compound (Ik) wherein $R^3$ or $R^4$ is dialkyl or nitrogen-containing heterocyclic group-substituted alkyl in the compound (I) can be obtained by reacting a compound (Ie) wherein the corresponding groups are chlorine, bromine or iodine-substituted alkyl with 1 to 10 equivalents of dialkylamine or nitrogen-containing heterocycle such as pyrrolidine, morpholine and imidazole, if necessary, in the presence of 1 to 5 equivalents of a base. As inert solvents, there are dimethylformamide, tetrahydrofuran and the like. As bases, there are sodium hydride, potassium carbonate and the like. The reaction completes in 1 to 20 hours at a temperature of from 0 °C to the boiling point of the solvent.

A compound (Im) wherein $R^1$ or $R^2$ is dialkyl or nitrogen-containing heterocyclic group-substituted alkyl in the compound (I) can be obtained from a compound (If) wherein $R^1$ or $R^2$ is the corresponding chlorine, bromine or iodine-substituted alkyl under the almost same conditions as those for the compound (Ik).

A compound (In) wherein one of $R^3$ and $R^4$ is hydrogen and the other is lower alkoxycarbonyl in the compound (I) can be obtained by starting from a compound (I) wherein one of $R^3$ and $R^4$ is hydrogen and the other is acetyl according to the known method [J. Chem. Soc. Chem. Commun., 1317 (1985)].

A compound (I) having desired functional groups at desired positions can be obtained by appropriately combining the above-described processes with each other.

Intermediates and final compounds in the above processes can be isolated and purified by purifying methods normally used in organic synthetic chemistry, for example, filtration, extraction, washing, drying, concentration, recrystallization, and various chromatographies and the like. In addition, intermediates may be subjected to the subsequent step without purification.

When the product is synthesized in the salted form, the salted compound can be subjected to known purification or isolation processes to obtain the salted product. If the product is synthetized in the unsalted form, to obtain a salt for the compound (I), it can be converted into a salt by, for example, dissolving or suspending it in an appropriate organic solvent and adding an appropriate acid.

Compound (I) and salts thereof can also be present in the form of addition products to water or various solvents, for example. Such addition products are included within the scope of the present invention.

Particular compounds (I) of the present invention are shown in Table 1.

Table 1

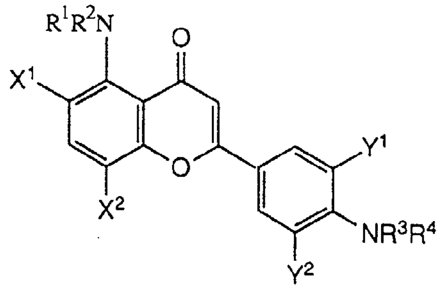

| Compound No. | $NR^1R^2$ | $NR^3R^4$ | $X^1$ | $X^2$ | $Y^1$ | $Y^2$ |
|---|---|---|---|---|---|---|
| 1 | $NH_2$ | $NH_2$ | Cl | H | H | H |
| 2 | $NH_2$ | $NH_2$ | H | Cl | H | H |
| 3 | $NH_2$ | $NH_2$ | Cl | Cl | H | H |
| 4 | $NH_2$ | $NH_2$ | Br | H | H | H |
| 5 | $NH_2$ | $NH_2$ | H | Br | H | H |
| 6 | $NH_2$ | $NH_2$ | Br | Br | H | H |
| 7 | $NHCOC(CH_3)_3$ | $NHCOC(CH_3)_3$ | F | H | H | H |
| 8 | $NH_2$ | $NH_2$ | F | H | H | H |
| 9 | $NHCOC(CH_3)_3$ | $NHCOC(CH_3)_3$ | H | F | H | H |
| 10 | $NH_2$ | $NH_2$ | H | F | H | H |
| 11 | $NHCOC(CH_3)_3$ | $NHCOC(CH_3)_3$ | F | F | H | H |
| 12 | $NH_2$ | $NH_2$ | F | F | H | H |
| 13 | $NH_2$ | $NHCOCH_3$ | F | H | H | H |
| 14 | $NH_2$ | $N(COCH_3)(CH_2)_3Pht$ | F | H | H | H |
| 15 | $NH_2$ | $NH(CH_2)_3Pht$ | F | H | H | H |
| 16 | $NH_2$ | $NH(CH_2)_3NH_2$ | F | H | H | H |
| 17 | $NH_2$ | $NHCOCH_3$ | H | F | H | H |
| 18 | $NH_2$ | $N(COCH_3)(CH_2)_3Pht$ | H | F | H | H |
| 19 | $NH_2$ | $NH(CH_2)_3Pht$ | H | F | H | H |
| 20 | $NH_2$ | $NH(CH_2)_3NH_2$ | H | F | H | H |
| 21 | $NH_2$ | $NHCOCH_3$ | F | F | H | H |
| 22 | $NH_2$ | $N(COCH_3)(CH_2)_3Pht$ | F | F | H | H |
| 23 | $NH_2$ | $NH(CH_2)_3Pht$ | F | F | H | H |
| 24 | $NH_2$ | $NH(CH_2)_3NH_2$ | F | F | H | H |
| 25 | $NHCOC(CH_3)_3$ | $NHCOC(CH_3)_3$ | F | F | F | H |

Table 1 (Continued)

| Compound No. | $NR^1R^2$ | $NR^3R^4$ | $X^1$ | $X^2$ | $Y^1$ | $Y^2$ |
|---|---|---|---|---|---|---|
| 26 | $NH_2$ | $NH_2$ | F | F | F | H |
| 27 | $NH_2$ | $NHCOCH_3$ | F | F | F | H |
| 28 | $NH_2$ | $N(COCH_3)(CH_2)_3Pht$ | F | F | F | H |
| 29 | $NH_2$ | $NH(CH_2)_3Pht$ | F | F | F | H |
| 30 | $NH_2$ | $NH(CH_2)_3NH_2$ | F | F | F | H |
| 31 | $NH(CH_2)_5CH_3$ | $N(COCH_3)(CH_2)_3Pht$ | F | F | F | H |
| 32 | $NH(CH_2)_5CH_3$ | $NH(CH_2)_3Pht$ | F | F | F | H |
| 33 | $NH(CH_2)_5CH_3$ | $NH(CH_2)_3NH_2$ | F | F | F | H |
| 34 | $NH_2$ | $NH_2$ | F | F | F | F |
| 35 | $NH_2$ | $NH_2$ | F | F | Cl | Cl |
| 36 | $NH_2$ | $NH_2$ | F | F | Br | Br |
| 37 | $NH_2$ | $NHCH_3$ | F | F | F | H |
| 38 | $NH_2$ | $N(CH_3)_2$ | F | F | F | H |
| 39 | $NH_2$ | $NH(CH_2)_2OCH_3$ | F | F | F | H |
| 40 | $NH_2$ | $NH(CH_2)_2N(CH_3)_2$ | F | F | F | H |
| 41 | $NH_2$ | 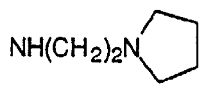 | F | F | F | H |
| 42 | $NH_2$ | 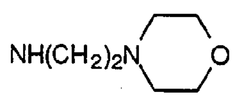 | F | F | F | H |
| 43 | $NH_2$ | $NH(CH_2)_2SCH_3$ | F | F | F | H |
| 44 | $NH_2$ | $NHCH_2CO_2CH_2CH_3$ | F | F | F | H |
| 45 | $NH_2$ | $NHCH_2CO_2Na$ | F | F | F | H |
| 46 | $NH_2$ | $NH(CH_2)_3OCH_3$ | F | F | F | H |
| 47 | $NH_2$ | $NH(CH_2)_3N(CH_3)_2$ | F | F | F | H |
| 48 | $NH_2$ | 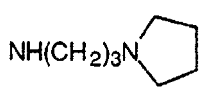 | F | F | F | H |
| 49 | $NH_2$ | $NH(CH_2)_3SCH_3$ | F | F | F | H |
| 50 | $NH_2$ | 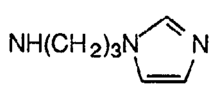 | F | F | F | H |

Table 1 (Continued)

| Compound No. | $NR^1R^2$ | $NR^3R^4$ | $X^1$ | $X^2$ | $Y^1$ | $Y^2$ |
|---|---|---|---|---|---|---|
| 51 | $NH_2$ | $NHCO_2C(CH_3)_3$ | F | F | F | H |
| 52 | $NH_2$ | 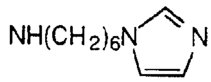 | F | F | F | H |
| 53 | $NH_2$ | $NH(CH_2)_6N(CH_3)_2$ | F | F | F | H |
| 54 | $NH_2$ | 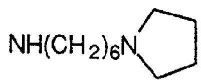 | F | F | F | H |
| 55 | $NH_2$ | 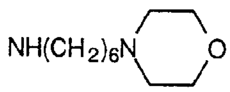 | F | F | F | H |
| 56 | $NH_2$ | $NH(CH_2)_5CO_2CH_2CH_3$ | F | F | F | H |
| 57 | $NH_2$ | $NH(CH_2)_5CO_2Na$ | F | F | F | H |
| 58 | $NH(CH_2)_5CH_3$ | $N(CH_3)_2$ | F | F | F | H |
| 59 | $NH(CH_2)_6Br$ | $N(CH_3)_2$ | F | F | F | H |
| 60 | $NH(CH_2)_6OH$ | $N(CH_3)_2$ | F | F | F | H |
| 61 | $NH(CH_2)_6OCH_3$ | $N(CH_3)_2$ | F | F | F | H |
| 62 | $NH(CH_2)_6N(CH_3)_2$ | $N(CH_3)_2$ | F | F | F | H |
| 63 | 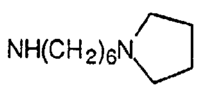 | $N(CH_3)_2$ | F | F | F | H |
| 64 | 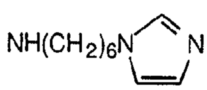 | $N(CH_3)_2$ | F | F | F | H |
| 65 | $NH(CH_2)_6NH_2$ | $N(CH_3)_2$ | F | F | F | H |
| 66 | $NH(CH_2)_4CH_3$ | $NH_2$ | F | F | F | H |
| 67 | $NH(CH_2)_5CH_3$ | $NH_2$ | F | F | F | H |
| 68 | $NH(CH_2)_6CH_3$ | $NH_2$ | F | F | F | H |
| 69 | $NH(CH_2)_4CH(Cl)CH_3$ | $NH_2$ | F | F | F | H |
| 70 | $NH(CH_2)_2CH(CH_3)_2$ | $NH_2$ | F | F | F | H |
| 71 | $NH(CH_2)_3CH(CH_3)_2$ | $NH_2$ | F | F | F | H |
| 72 | $NH(CH_2)_3NH_2$ | $NH_2$ | F | F | F | H |
| 73 | $NH(CH_2)_4NH_2$ | $NH_2$ | F | F | F | H |
| 74 | $NH(CH_2)_5CH_3$ | $NHCH_2CO_2CH_2CH_3$ | F | F | F | H |
| 75 | $NH(CH_2)_5CH_3$ | $NHCH_2CO_2H$ | F | F | F | H |

Table 1 (Continued)

| Compound No. | $NR^1R^2$ | $NR^3R^4$ | $X^1$ | $X^2$ | $Y^1$ | $Y^2$ |
|---|---|---|---|---|---|---|
| 76 | $NH(CH_2)_3CH{=}CH_2$ | $NHCH_3$ | F | F | F | H |
| 77 | $NH(CH_2)_4CH{=}CH_2$ | $NHCH_3$ | F | F | F | H |
| 78 | $NH(CH_2)_3OCH_3$ | $NHCH_3$ | F | F | F | H |
| 79 | $NH(CH_2)_4CH_3$ | $NH(CH_2)_3NH_2$ | F | F | F | H |

In Table 1, Pht represents

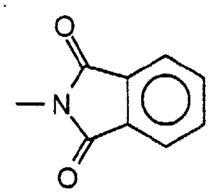

Then, antibacterial activity and antitumor activity of the representative compounds (I) are shown by experiments.

Test 1

Antibacterial activity of the compound (I) against Bacillus subtilis #10107 [Minimum Inhibition Concentration (MIC; μg/ml)] is shown in Table 2. Minimum Inhibition Concentration was determined by agar dilution method at pH 7.0.

Table 2

| Compound No. | MIC ( μg/ml) |
|---|---|
| 16 | 5.2 |
| 24 | 2.6 |

Test 2

Human uterine cervix carcinoma (HeLaS3) growth inhibition test;

Each 0.1 ml of HeLaS3 cells which had been prepared to 3 x $10^4$/ml using a medium (referred to as Medium A hereinafter) consisted of MEM medium, 2 mM glutamine and 10% bovine fetal serum was distributed in each well of 96 well-microtiter plate. The plate was cultured at 37 °C for 20 hours in a $CO_2$ gas incubator, each 0.05 ml of samples (test compounds) which had been appropriately diluted with Medium A was added thereto and the mixture was cultured at 37 °C for 72 hours in a $CO_2$ gas incubator. Supernatant was removed, each 0.1 ml of Medium A containing 0.02% neutral red was added to the residue, the mixture was cultured at 37 °C for 1 hour in a $CO_2$ gas incubator and the cells were stained. Supernatant was removed and the residue was washed once with saline. Then, the pigment was extracted with 0.001N hydrochloric acid/30% ethanol and the absorbance at 550 nm was measured with a microplatereader. Sample concentration ($IC_{50}$) at which the growth of cells is inhibited by 50% was calculated by comparing the absorbance of non-treated cells and that of cells treated with predetermined concentration of sample.

The results are shown in Table 3.

Table 3

| Compound No. | $IC_{50}$ ( μM) | Compound No. | $IC_{50}$ ( μM) |
|---|---|---|---|
| 2 | 3.2 | 53 | 6.8 |

Table 3 (continued)

| Compound No. | $IC_{50}$ ( μM) | Compound No. | $IC_{50}$ ( μM) |
|---|---|---|---|
| 3 | 33 | 54 | 6.1 |
| 5 | 6.1 | 55 | 11 |
| 10 | 21 | 62 | 7.1 |
| 12 | 10 | 63 | 7.0 |
| 16 | 5.8 | 64 | 3.0 |
| 20 | 9.3 | 65 | 7.0 |
| 24 | 3.4 | 67 | 9.4 |
| 26 | 26 | 71 | 12 |
| 30 | 14 | 74 | 12 |
| 38 | 12 | 75 | 16 |
| 41 | 5.0 | 77 | 11 |
| 52 | 4.4 | 79 | 7.5 |

Test 3

Human mammary cancer (MCF-7) growth inhibition test;

Each 0.1 ml of MCF-7 cells which had been prepared to 5 x $10^4$/ml using a medium (referred to as Medium B hereinafter) prepared by adding 10% bovine fetal serum, $10^{-8}$M estradiol (manufactured by Sigma), 100 units/ml penicillin and 100 μg/ml streptomycin to medium RPMI1640 was distributed in each well of 96 well-microtiter plate. The plate was cultured at 37 °C for 20 hours in a $CO_2$ gas incubator, each 0.05 ml of samples (test compounds) which had been appropriately diluted with Medium B was added thereto and the mixture was cultured at 37 °C for 72 hours in a $CO_2$ gas incubator. Supernatant was removed, each 0.1 ml of Medium B containing 0.02% neutral red was added to the residue, the mixture was cultured at 37 °C for 1 hour in a $CO_2$ gas incubator and the cells were stained. $IC_{50}$ was calculated according to the same manner as that in Test 2.

The results are shown in Table 4

Table 4

| Compound No. | $IC_{50}$ ( μM) | Compound No. | $IC_{50}$ ( μM) |
|---|---|---|---|
| 2 | 0.37 | 46 | 0.11 |
| 3 | 22 | 52 | 2.0 |
| 6 | 27 | 53 | 6.2 |
| 8 | 0.14 | 54 | 5.9 |
| 10 | 0.25 | 56 | 0.91 |
| 20 | 0.094 | 57 | 0.73 |
| 30 | 0.039 | 58 | 0.34 |
| 35 | 0.17 | 60 | 0.34 |
| 36 | 0.61 | 61 | 0.63 |
| 40 | 0.23 | 69 | 6.0 |
| 42 | 6.1 | 74 | 9.2 |
| 44 | 0.36 | | |

Test 4

Antitumor effects against estrogen dependent human mammary cancer MCF-7

Tumor strips (2mm x 2 mm x 2mm) of human hormone dependent mammary cancer MCF-7 were transplanted subcutaneously in the ventral part of female BALB/C-nu/nu mice (Nihon Crea), 7-9 weeks age. For promoting the growth of tumor, 12.5 μg of estradiol was intramuscularlly administered three times in the femoral region on the date of transplantation and 7 and 14 days after transplantation. 7 Days after transplantation, mice the tumor volume of which increased to 10-100 $mm^3$ were selected, and test compounds were orally administered repeatedly for five days per a week, for four weeks, at five animals per group. Length and breadth of the tumor were determined, and the tumor volumes were calculated by means of ellipsoid approximation according to the following equation:

$$\text{Tumor volume (mm}^3\text{)} = \{\text{Length (mm) x [Breadth (mm)]}^2\}/2$$

The tumor volume ($V_0$) at initial administration was determined. The tumor volume (V) was determined after initiation to calculate $V/V_0$ from which the ratio (T/C), that is, the ratio of $V/V_0$ value of treated group relative to that of control group was obtained.

The results are shown in Table 5.

Table 5

| Compound No. | Dose (mg/kg) | T/C | Date |
|---|---|---|---|
| 12 | 6.3 | 0.45 | 22 |
| 26 | 25 | 0.45 | 29 |

The following Examples and Reference Examples further illustrate the present invention in detail but are not be construed to limit the scope thereof.

Physicochemical data on respective compounds in the following Examples and Reference Examples were determined using the apparatus;

IR Nihon Bunko IR-810
HORIBA FT-200

$^1$H NMR JEOL JNM-GX270 (270MHz)
JEOl JNM-EX270 (270MHz)
HITACHI R-90H (90MHz)

MS JEOL JMS-D300
JEOL JMS-SX102
SHIMAZU QP-1000

In addition, MS means EI-MS, and dioxane means 1,4-dioxane.

Example 1

5-Amino-2-(4-aminophenyl)-6-chloro-4H-1-benzopyran-4-one (Compound 1)

2.0 g (7.93 mmol) of 5-amino-2-(4-aminophenyl)-4H-1-benzopyran-4-one (EP-A-374789) was dissolved in 50 ml of dioxane, 1.06 g (7.93 mmol) of N-chlorosuccinimide was added and the mixture was stirred under heating at reflux for 19 hours. The reaction solution was concentrated under reduced pressure, the residue was extracted with ethyl acetate, washed with an aqueous saturated solution of sodium chloride and dried over anhydrous sodium sulfate. The ethyl acetate layer was concentrated under reduced pressure and the residue was purified by silica gel column chromatography (chloroform:acetone=4:1) to give 710 mg (31%) of crude product. Further recrystallization from chloroform/methanol afforded 415 mg (18%) of Compound 1.

IR (KBr) $\nu$($cm^{-1}$) 3404, 1642, 1597, 1546, 1444, 1395, 1304, 829

NMR (270MHz, DMSO-$d_6$) $\delta$(ppm) 6.00 (2H, brs), 6.58 (1H, s), 6.67 (2H, d, J=8.9), 6.75 (1H, d, J=8.9), 7.54 (1H, d, J=8.4), 7.73 (2H, d, J=8.9)

MS(M/Z) 286/288($M^+$)

Molecular formula $C_{15}H_{11}ClN_2O_2$=286

Example 2

5-Amino-2-(4-aminophenyl)-8-chloro-4H-1-benzopyran-4-one (Compound 2)

2.0 g (7.93 mmol) of 5-amino-2-(4-aminophenyl)-4H-1-benzopyran-4-one (EP-A-374789) was dissolved in 50 ml of dioxane, 1.59 g (11.9 mmol) of N-chlorosuccinimide was added and the mixture was stirred under heating at reflux for 5 hours. The reaction solution was extracted with ethyl acetate, washed with an aqueous saturated solution of sodium chloride and dried over anhydrous sodium sulfate. The ethyl acetate layer was concentrated under reduced pressure and the residue was purified by silica gel column chromatography (chloroform:acetone=4:1) to give 0.61 g

(27%) of crude product. Further recrystallization from chloroform/methanol afforded 266 mg (12%) of Compound 2.

IR (KBr) ν($cm^{-1}$) 3436, 3308, 1640, 1580, 1544, 1471, 1396, 1186, 822

NMR (270MHz, DMSO-$d_6$) δ(ppm) 6.52 (1H, d, J=8.9), 6.58 (2H, d, J=8.4), 6.68 (1H, s), 6.75 (2H, brs), 7.42 (1H, d, J=8.9), 7.75 (2H, d, J=8.4)

MS(M/Z) 286/288 ($M^+$)

Molecular formula $C_{15}H_{11}ClN_2O_2$=286

Example 3

5-Amino-2-(4-aminophenyl)-6,8-dichloro-4H-1-benzopyran-4-one (Compound 3)

0.94 g (3.73 mmol) of 5-amino-2-(4-aminophenyl)-4H-1-benzopyran-4-one (EP-A-374789) was suspended in 50 ml of acetic acid, 24.0 g of 2.73% chlorine/acetic acid solution (chlorine: 9.23 mmol) was added and the mixture was stirred at room temperature for 18 hours. The reaction solution was poured in water, extracted with a mixed solution of chloroform and methanol, washed with an aqueous saturated solution of sodium bicarbonate and an aqueous saturated solution of sodium chloride and dried over anhydrous sodium sulfate. The organic layer was concentrated under reduced pressure and the residue was purified by silica gel column chromatography (chloroform:acetone=4:1) to give 375 mg (30%) of crude product. Further recrystallization from chloroform/methanol afforded 218 mg (18%) of Compound 3.

IR (KBr) ν($cm^{-1}$) 3454, 3300, 1627, 1607, 1536, 1514, 1444, 1393, 1296, 1250, 1187, 1129, 823

NMR (270MHz, DMSO-$d_6$) δ(ppm) 6.07 (2H, brs), 6.08 (1H, s), 6.68 (2H, d, J=9.4), 7.76 (1H, s), 7.76 (2H, d, J=8.9)

MS (M/Z) 320/322/324 ($M^+$)

Molecular formula $C_{15}H_{10}Cl_2N_2O_2$=321

Example 4

5-Amino-2-(4-aminophenyl)-6-bromo-4H-1-benzopyran-4-one (Compound 4)

2.50 g (11.9 mmol) of 5-amino-2-(4-aminophenyl)-4H-1-benzopyran-4-one (EP-A-374789) was dissolved in 75 ml of dioxane, 1.86 g (13.1 mmol) of N-bromosuccinimide was added and the mixture was stirred at room temperature for 2 hours. The reaction solution was concentrated under reduced pressure, and the residue was extracted with a mixed solution of chloroform and methanol, washed with an aqueous saturated solution of sodium chloride and dried over anhydrous sodium sulfate. The organic layer was concentrated under reduced pressure and the residue was purified by silica gel column chromatography (chloroform:acetone=40:1) to give 170 mg (4.3%) of a Compound 4.

IR (KBr) ν($cm^{-1}$) 3344, 1627, 1582, 1544, 1536, 1388, 1185, 823

NMR (270MHz, DMSO-$d_6$) δ(ppm) 6.01 (2H, brs), 6.49 (1H, d, J=8.9), 6.57 (1H, s), 6.67 (2H, d, J=8.1), 7.52 (1H, d, J=8.9), 7.58 (2H, brs), 7.77 (2H, d, J=8.4)

MS (M/Z) 330/332 ($M^+$)

Molecular formula $C_{15}H_{11}BrN_2O_2$=331

Example 5

5-Amino-2-(4-aminophenyl)-8-bromo-4H-1-benzopyran-4-one (Compound 5)

2.50 g (11.9 mmol) of 5-amino-2-(4-aminophenyl)-4H-1-benzopyran-4-one (EP-A-374789) was dissolved in 75 ml of dioxane, 1.86 g (13.1 mmol) of N-bromosuccinimide was added and the mixture was stirred at room temperature for 2 hours. The reaction solution was concentrated under reduced pressure, and the residue was extracted with a mixed solution of chloroform and methanol, washed with an aqueous saturated solution of sodium chloride and dried over anhydrous sodium sulfate. The organic layer was concentrated under reduced pressure and the residue was purified by silica gel column chromatography (chloroform:acetone=40:1) to give 767 mg (20%) of Compound 5.

IR (KBr) ν($cm^{-1}$) 3340, 1625, 1581, 1536, 1515, 1388, 1323, 1250, 1185, 824

NMR (270MHz, DMSO-$d_6$) δ(ppm) 6.00 (2H, brs), 6.50 (1H, dd, J=8.9), 6.58 (1H, s), 7.52 (1H, d, J=8.9), 7.55 (2H, brs), 7.68 (2H, d, J=8.9), 7.78 (2H, d, J=8.4)

MS (M/Z) 330/332 ($M^+$)

Molecular formula $C_{15}H_{11}BrN_2O_2$=331

Example 6

5-Amino-2-(4-aminophenyl)-6,8-dibromo-4H-1-benzopyran-4-one (Compound 6)

2.0 g (5.8 mmol) of 5-amino-2-(4-aminophenyl)-4H-1-benzopyran-4-one (EP-A-374789) was suspended in 100 ml of acetic acid, 0.45 ml (8.7 mmol) of bromine was added and the mixture was stirred at room temperature for 18 hours. The formed insolubles were filtered and triturated with methanol to give 1.23 g (51%) of hydrobromide of Compound 6.

IR (KBr) $\nu(cm^{-1})$ 3380, 1621, 1587, 1545, 1515, 1482, 1417, 1285, 1186

NMR (270MHz, DMSO-$d_6$) $\delta$(ppm) 6.05 (2H, brs), 6.68 (1H, s), 6.69 (2H, d, J=8.8), 7.79 (2H, d, J=8.6), 7.97 (1H, s)

MS (M/Z) 408/410/412 ($M^+$)

Molecular formula $C_{15}H_{10}Br_2N_2O_2$=410

Example 7

6-Fluoro-5-pivaloylamino-2-(4-pivaloylaminophenyl)-4H-1-benzopyran-4-one (Compound 7)

A solution of 32.2 g of ethyl 2-(N-ethoxycarbonyl-N-pivaloylamino)-3-fluoro-6-methoxymethoxybenzoate obtained in Reference Example 1 and 17.6 g of 4'-pivaloylaminoacetophenone dissolved in 300 ml of dioxane was added dropwise to a suspension of 6.44 g of sodium hydride (60% oil suspension) in 100 ml of dioxane over 30 minutes under heating at reflux and the mixture was heated at reflux for additional 30 minutes. The reaction solution was cooled, water was added, the aqueous layer was washed with n-hexane and extracted with ethyl acetate. The ethyl acetate layer was washed with an aqueous saturated solution of sodium chloride, dried over anhydrous sodium sulfate and subjected to silica gel column chromatography (n-hexane:ethyl acetate=2:1-1:1) to give 25.2 g (63%) of 1,3-diketone.

25.2 g of the resulting 1,3-diketone was dissolved in 300 ml of ethanol, 60 ml of concentrated hydrochloric acid was added and the mixture was stirred at room temperature for 1 hour. Then, 300 ml of water was added and the precipitated crystals were filtered to give Compound 7.

NMR (90MHz, $CDCl_3$) $\delta$(ppm) 1.37 (9H, s), 1.42 (9H, s), 6.61 (1H, s), 7.11 (1H, dd, J=9.2, 4.2), 7.31 (1H, t, J=9.5), 7.50 (2H, d, J=9.0), 7.68 (2H, d, J=9.0), 8.08 (1H, brs), 10.9 (1H, brs)

MS (M/Z) 438 ($M^+$)

Molecular formula $C_{25}H_{27}FN_2O_4$=438

Example 8

5-Amino-2-(4-aminophenyl)-6-fluoro-4H-1-benzopyran-4-one (Compound 8)

Compound 7 obtained in Example 7 was dissolved in 490 ml of dioxane, 210 ml of concentrated hydrochloric acid was added and the mixture was heated at reflux for 2.5 hours. The reaction solution was cooled on ice and the precipitated crystals were filtered to give 9.08 g (total 59%) of hydrochloride of Compound 8.

IR (KBr) $\nu(cm^{-1})$ 3425, 3310, 1619, 1595, 1561, 1554, 1485, 1394, 1298, 1248, 1187, 824

NMR (270MHz, DMSO-$d_6$) $\delta$(ppm) 6.00 (2H, brs), 6.43 (2H, dd, J=8.9, 3.5), 6.54 (1H, s), 6.67 (2H, d, J=7.9), 7.19 (2H, brs), 7.34 (1H, t, J=9.9), 7.69 (2H, d, J=8.4)

MS (M/Z) 270 ($M^+$)

Molecular formula $C_{15}H_{11}FN_2O_2$=270

Example 9

8-Fluoro-5-pivaloylamino-2 -(4-pivaloylaminophenyl)-4H-1-benzopyran-4-one (Compound 9)

A solution of 15.6 g of ethyl 6-(N-ethoxycarbonyl-N-pivaloylamino)-3-fluoro-2-methoxymethoxybenzoate obtained in Reference Example 2 and 7.71 g of 4'-pivaloylaminoacetophenone dissolved in 150 ml of dioxane was added dropwise to a suspension of 3.13 g of sodium hydride (60% oil suspension) in 40 ml of dioxane over 30 minutes under heating at reflux and the mixture was heated at reflux for additional 30 minutes. 1.56 g of sodium hydride (60% oil suspension) was added thereto and the mixture was heated at reflux for additional 1 hour. The reaction solution was cooled, water was added, the aqueous layer was washed with n-hexane and extracted with ethyl acetate. The ethyl acetate layer was washed with an aqueous saturated solution of sodium chloride, dried over anhydrous sodium sulfate and subjected to silica gel column chromatography (n-hexane:ethyl acetate=2:1) to give 15.0 g (85%) of 1,3-diketone.

15.0 g of the resulting 1,3-diketone was dissolved in 100 ml of ethanol, 20 ml of concentrated hydrochloric acid was added and the mixture was stirred at room temperature for 2 hour. The reaction solution was cooled on ice, 100

ml of water was added and the precipitated crystals were filtered to give 9.49 g (72%) of Compound 9.

NMR (90MHz, $CDCl_3$) δ(ppm) 1.35 (9H, s), 1.38 (9H, s), 6.72 (1H, s), 7.42 (1H, t, J=9.7), 7.52 (1H, brs), 7.72 (2H, d, J=9.0), 7.92 (2H, d, J=9.0), 8.68 (1H, dd, J=9.3, 4.3), 12.5 (1H, brs)

MS (M/Z) 438 ($M^+$)

Molecular formula $C_{25}H_{27}FN_2O_4$=438

Example 10

5-Amino-2-(4-aminophenyl)-8-fluoro-4H-1-benzopyran-4-one (Compound 10)

9.48 g of Compound 9 was dissolved in 140 ml of dioxane, 60 ml of concentrated hydrochloric acid was added and the mixture was heated at reflux for 4 hours. The reaction solution was cooled on ice, a 10N aqueous solution of sodium hydroxide was added to adjust to pH 7, water was added, the precipitated crystals were filtered and triturated with ether to give 5.28 g (91%) of Compound 10.

IR (KBr) ν($cm^{-1}$) 3480, 3342, 1641, 1552, 1478, 1446, 1187, 819

NMR (270MHz, DMSO-$d_6$) δ(ppm) 5.97 (2H, brs), 6.51 (1H, s), 6.65 (2H, d, J=8.9), 6.67 (1H, dd, J=9.2, 3.7), 7.25 (2H, brs), 7.37 (1H, dd, J=11.4, 9,4), 7.72 (2H, d, J=8.4)

MS (M/Z) 270 ($M^+$)

Molecular formula $C_{15}H_{11}FN_2O_2$=270

Example 11

6,8-Difluoro-5-pivaloylamino-2-(4-pivaloylaminophenyl)-4H-1-benzopyran-4-one (Compound 11)

A solution of 10.6 g of ethyl 3,5-difluoro-6-(N-ethoxycarbonyl-N-pivaloylamino)-2-methoxymethoxybenzoate obtained in Reference Example 3 and 2.09 g of 4'-pivaloylaminoacetophenone dissolved in 40 ml of dioxane was added dropwise to a suspension of 1.27 g of sodium hydride (60% oil suspension) in 20 ml of dioxane over 30 minutes under heating at reflux and the mixture was heated at reflux for additional 20 minutes. 424 mg of sodium hydride (60% oil suspension) was added and the mixture was heated at reflux for additional 1 hour. The reaction solution was cooled, water was added, the aqueous layer was washed with n-hexane and extracted with ethyl acetate. The ethyl acetate layer was washed with an aqueous saturated solution of sodium chloride, dried over anhydrous sodium sulfate and subjected to silica gel column chromatography (n-hexane:ethyl acetate=2:1) to give 2.53 g (46%) of 1,3-diketone 2.53 g of the resulting 1,3-diketone was dissolved in 50 ml of ethanol, 10 ml of concentrated hydrochloric acid was added and the mixture was stirred at room temperature for 1.5 hour. The reaction solution was cooled on ice and the precipitated crystals were filtered to give 1.42 g (64%) of Compound 11.

NMR (90MHz, $CDCl_3$) δ(ppm) 1.37 (9H, s), 1.41 (9H, s), 7.12 (1H, t, J=10), 7.53 (2H, d, J=8.8), 7.73 (2H, d, J=8.8), 8.13 (1H, brs), 10.46 (1H, brs)

MS (M/Z) 456 ($M^+$)

Molecular formula $C_{25}H_{26}F_2N_2O_4$=456

Example 12

5-Amino-2-(4-aminophenyl)-6,8-difluoro-4H-1-benzopyran-4-one (Compound 12)

1.42 g of Compound 11 was dissolved in 84 ml of dioxane, 36 ml of concentrated hydrochloric acid was added and the mixture was heated at reflux for 3 hours. The reaction solution was cooled on ice and the precipitated crystals were filtered to give 579 mg (57%) of Compound 12.

IR (KBr) ν($cm^{-1}$) 1653, 1562, 1515, 1483, 1395, 1309, 1121, 985, 832

NMR (270MHz, DMSO-$d_6$) δ(ppm) 6.05 (2H, brs), 6.58 (1H, s), 6.68 (2H, d, J=8.9), 7.03 (2H, brs), 7.67 (1H, t, J=10.9), 7.71 (2H, d, J=8.9)

MS (M/Z) 288 ($M^+$)

Molecular formula $C_{15}H_{10}F_2N_2O_2$=288

Example 13

2-(4-Acetylaminophenyl)-5-amino-6-fluoro-4H-1-benzopyran-4-one (Compound 13)

10.8 g of Compound 8 was dissolved in 100 ml of pyridine, the solution was cooled to -20 °C, 3.78 ml of acetic

anhydride was added and the mixture was stirred for 2.3 hours while rising the temperature to room temperature gradually. Then, the reaction solution was poured into 200 ml of ice water, the precipitated crystals were filtered and triturated with ether to give 10.8 g (86%) of Compound 13.

NMR (90MHz, DMSC-$d_6$) δ(ppm) 2.10 (3H, s), 6.5-6.8 (1H, m), 6.70 (1H, s), 7.23 (2H, brs), 7.38 (1H, t, J=10), 10.2 (1H, brs)

MS (M/Z) 312 ($M^+$)

Molecular formula $C_{17}H_{13}FN_2O_3$=312

Example 14

2-[4-[N-Acetyl-N-(3-phthalimidopropyl)amino]phenyl]-5-amino-6-fluoro-4H-1-benzopyran-4-one (Compound 14)

10.8 g of Compound 13 was dissolved in 150 ml of dimethylformamide, 1.38 g of sodium hydride (60% oil suspension), 9.28 g of N-(3-bromopropyl)phthalimide and 1.04 g of sodium iodide were added thereto under ice-cooling and the mixture was stirred at room temperature for 1.5 hours. Additional 1.38 g of sodium hydride (60% oil suspension) and 9.28 g of N-(3-bromopropyl)phthalimide were added thereto and the mixture was stirred at room temperature for 2.3 hours. An aqueous solution of ammonium chloride was added to the reaction solution, the solvent was distilled off under reduced pressure and the residue was extracted once with chloroform. The chloroform layer was washed with an aqueous saturated solution of sodium chloride, dried over anhydrous sodium sulfate and the solvent was distilled off under reduced pressure. The residue was subjected to silica gel column chromatography (chloroform:methanol=60: 1-40:1) to give 9.98 g (58%) of Compound 14.

NMR (90MHz, $CDCl_3$) δ(ppm) 1.91 (2H, quint., J=7.3), 1.91 (3H, s), 3.74 (2H, t, J=7.5), 3.87 (2H, t, J=7.0), 6.49 (2H, brs), 6.61 (1H, s), 6.64 (1H, dd, J=10.1, 3.7), 7.24 (1H, t, J=9.0), 7.40 (2H, d, J=8.6), 7.6-7.9 (4H, m), 7.94 (2H, d, J=8.8)

MS (M/Z) 499 ($M^+$)

Molecular formula $C_{28}H_{22}FN_3O_5$=499

Example 15

5-Amino-6-fluoro-2-[4-[(3-phthalimidopropyl)amino]phenyl]-4H-1-benzopyran-4-one (Compound 15)

6.0 g of Compound 14 was dissolved in 90 ml of dioxane, 60 ml of concentrated hydrochloric acid was added and the mixture was heated at reflux for 5 hours. The reaction solution was cooled on ice, a 10N aqueous solution of sodium hydroxide was added to adjust to pH 7.5, water was further added and the precipitated crystals were filtered to give 4.78 g (87%) of Compound 15.

NMR (90MHz, DMSO-$d_6$) δ(ppm) 1.92 (2H, quint., J=6.6), 3.0-3.3 (2H, m), 3.71 (2H, t, J=6.9), 6.4-6.7 (3H, m), 6.51 (1H, s), 7.36 (1H, t, J=9.5), 7.6-7.9 (6H, m)

MS (M/Z) 457 ($M^+$)

Molecular formula $C_{26}H_{20}FN_3O_4$=457

Example 16

5-Amino-2-[4-[(3-aminopropyl)amino]phenyl]-6-fluoro-4H-1-benzopyran-4-one (Compound 16)

4.78 g of Compound 15 was dissolved in 200 ml of a mixed solvent of dimethylformamide and methanol (1:1), 5.07 ml of hydrazine monohydrate was added and the mixture was stirred at 50-60 °C for 1 hour. The reaction solution was concentrated under reduced pressure, the residue was subjected to silica gel column chromatography (chloroform: methanol:aqueous ammonia=9:1:1) to give Compound 16 which was dissolved in ethanol, a 5.5N hydrochloric acid-isopropylalcohol solution was added, isopropyl ether was added and the precipitated crystals were filtered to give 2.69 g (64%) of hydrochloride of Compound 16.

IR (KBr) ν($cm^{-1}$) 3448, 3318, 1646, 1562, 1478, 1445, 1019

NMR (270MHz, DMSO-$d_6$) δ(ppm) 1.90 (2H, quint., J=6.9), 2.52 (2H, m), 3.24 (2H, t, J=6.9), 6.56 (1H, s), 6.68 (1H, dd, J=8.9, 3.5), 6.77 (2H, d, J=8.4), 7.38 (1H, dd, J=11.4, 8.9), 7.80 (2H, d, J=8.9), 8.13 (2H, brs)

MS (M/Z) 327 ($M^+$)

Molecular formula $C_{18}H_{18}FN_3O_2$=327

Example 17

2-(4-Acetylaminophenyl)-5-amino-8-fluoro-4H-1-benzopyran-4-one (Compound 17)

5.28 g of Compound 10 was dissolved in 50 ml of pyridine, the solution was cooled to -10 °C, 1.85 ml of acetic anhydride was added and the mixture was stirred for 2.3 hours while rising the temperature to room temperature gradually. Then, the reaction solution was poured into 200 ml of ice water, the precipitated crystals were filtered and triturated with ether to give 5.42 g (90%) of Compound 17.

NMR (90MHz, DMSO-$d_6$) δ(ppm) 2.09 (3H, s), 6.47 (1H, dd, J=9.2, 3.7), 6.76 (1H, s), 7.37 (1H, dd, J=10.8, 9.2), 7.76 (2H, d, J=9.0), 7.95 (2H, d, J=8.6)

MS (M/Z) 312 ($M^+$)

Molecular formula $C_{17}H_{13}FN_2O_3$=312

Example 18

2-[4-[N-Acetyl-N-(3-phthalimidopropyl)amino]phenyl]-5-amino-8-fluoro-4H-1-benzopyran-4-one (Compound 18)

5.42 g of Compound 17 was dissolved in 80 ml of dimethylformamide, 695 mg of sodium hydride (60% oil suspension), 5.60 g of N-(3-bromopropyl)phthalimide and 0.52 g of sodium iodide were added thereto under ice-cooling and the mixture was stirred at room temperature for 2 hours. Additional 695mg of sodium hydride (60% oil suspension) and 5.60 g of N-(3-bromopropyl)phthalimide were added thereto and the mixture was stirred at room temperature for 3 hours. An aqueous solution of ammonium chloride was added to the reaction solution, the solvent was distilled off under reduced pressure and the residue was extracted once with chloroform. The chloroform layer was washed with an aqueous saturated solution of sodium chloride, dried over anhydrous sodium sulfate and the solvent was distilled off under reduced pressure. The residue was subjected to silica gel column chromatography (chloroform:methanol=80:1-70:1) to give 5.81 g (67%) of Compound 18.

NMR (90MHz, $CDCl_3$) δ(ppm) 1.7-2.1 (2H, m), 1.91 (3H, s), 3.4-4.0 (m, 4H), 6.37 (1H, dd, J=9.0, 3.5), 6.65 (1H, s), 7.22 (1H, dd, J=10.3, 9.0), 7.40 (2H, d, J=8.6), 7.5-7.9 (4H, m), 7.98 (2H, d, J=8.6)

MS (M/Z) 499 ($M^+$)

Molecular formula $C_{28}H_{22}FN_3O_5$=499

Example 19

5-Amino-8-fluoro-2-[4-[(3-phthalimidopropyl)amino]phenyl]-4H-1-benzopyran-4-one (Compound 19)

5.78 g of Compound 18 was dissolved in 90 ml of dioxane, 60 ml of concentrated hydrochloric acid was added and the mixture was heated at reflux for 4 hours. The reaction solution was cooled on ice, a 10N aqueous solution of sodium hydroxide was added to adjust to pH 7, water was further added and the precipitated crystals were filtered to give 4.23 g (80%) of Compound 19.

NMR (90MHz, DMSO-$d_6$) δ(ppm) 1.92 (2H, quint., J=6.4), 3.0-3.4 (2H, m), 3.71 (2H, t, J=7.3), 6.43 (1H, dd, J=9.1, 3.8), 6.68 (2H, d, J=9.0), 7.34 (1H, dd, J=10.6, 9.1), 7.73 (2H, d, J=8.8), 7.84 (4H, brs)

MS (M/Z) 457 ($M^+$)

Molecular formula $C_{26}H_{20}FN_3O_4$=457

Example 20

5-Amino-2-[4-[(3-aminopropyl)amino]phenyl]-8-fluoro-4H-1-benzopyran-4-one (Compound 20)

4.22 g of Compound 19 was dissolved in 200 ml of a mixed solvent of dimethylformamide and methanol (1:1), 9.0 ml of hydrazine monohydrate was added and the mixture was stirred at 50-60 °C for 30 minutes. The reaction solution was concentrated under reduced pressure, the residue was subjected to silica gel column chromatography (chloroform: methanol:aqueous ammonia=9:1:1) to give Compound 20 which was dissolved in ethanol, a 5.5N hydrochloric acid-isopropylalcohol solution was added and the precipitated crystals were filtrered to give 1.47 g (40%) of Compound 20.

IR (KBr) ν($cm^{-1}$) 1632, 1608, 1384, 1254, 1194, 824

NMR (270MHz, DMSO-$d_6$) δ(ppm) 1.88 (2H, quint., J=6.9), 2.90 (2H, m), 3.23 (2H, t, J=6.9), 6.49 (1H, dd, J=9.2, 3.7), 6.61 (1H, s), 6.75 (2H, d, J=8.9), 7.37 (1H, t, J=9.9), 7.77 (2H, d, J=8.4), 8.06 (2H, brs)

MS (M/Z) 327 ($M^+$)

Molecular formula $C_{18}H_{18}FN_3O_2$=327

Example 21

2-(4-Acetylaminophenyl)-5-amino-6,8-difluoro-4H-1-benzopyran-4-one (Compound 21)

720 mg of Compound 12 was dissolved in 10 ml of pyridine, the solution was cooled on ice, 0.24 ml of acetic anhydride was added and the mixture was stirred at 0 °C for 40 minutes. Then, the reaction solution was added to 20 ml of ice water and the precipitated crystals were filtered to give 696 mg (84%) of Compound 21.

NMR (90MHz, DMSO-$d_6$) δ(ppm) 2.09 (3H, s), 6.79 (1H, s), 7.03 (2H, brs), 7.70 (1H, t, J=11.4), 7.75 (2H, d, J=9.9), 7.95 (2H, d, J=8.8), 10.2 (1H, brs)

MS (M/Z) 330

Molecular formula $C_{17}R_{12}F_2N_2O_3$=330

Example 22

5-Amino-2-[4-[N-acetyl-N-(3-phthalimidopropyl)amino]phenyl]-6,8-difluroro-4H-1-benzopyran-4-one (Compound 22)

690 mg of Compound 21 was dissolved in 10 ml of dimethylformamide, 83.6 mg of sodium hydride (60% oil suspension), 673 mg of N-(3-bromopropyl)phthalimide and 62.7 mg of sodium iodide were added under ice-cooling and the mixture was stirred at room temperature for 1 hour. Additional 83.6 mg of sodium hydride (60% oil suspension) and 673 mg of N-(3-bromopropyl)phthalimide were added and the mixture was stirred at room temperature for 1.3 hours. Additional 83.6 mg of sodium hydride (60% oil suspension) and 673 mg of N-(3-bromopropyl)phthalimide were added and the mixture was stirred at room temperature for 3.5 hours. An aqueous solution of ammonium chloride was added to the reaction solution, the solvent was distilled off under reduced pressure and extracted once with chloroform. The chloroform layer was washed with an aqueous saturated solution of sodium chloride, dried over anhydrous sodium sulfate and the solvent was distilled off under reduced pressure. The residue was subjected to silica gel column chromatography (chloroform:methanol=80:1-60:1) to give 0.84 g (78%) of Compound 22.

NMR (90MHz, $CDCl_3$) δ(ppm) 1.7-2.1 (2H, m), 1.91 (3H, s), 3.74 (2H, t, J=7.3), 3.86 (2H, t, J=6.7), 6.22 (2H, brs), 6.64 (1H, s), 7.21 (1H, t, J=10.4), 7.41 (2H, d, J=8.6), 7.6-7.9 (4H, m), 7.98 (2H, d, J=8.6)

MS (M/Z) 517 ($M^+$)

Molecular formula $C_{28}H_{21}F_2N_3O_5$=517

Example 23

5-Amino-6,8-difluoro-2-[4-[(3-phthalimidopropyl)amino]phenyl]-4H-1-benzopyran-4-one (Compound 23)

0.83 g of Compound 22 was dissolved in 12 ml of dioxane, 8 ml of concentrated hydrochloric acid was added and the mixture was heated at reflux for 4 hours. The reaction solution was cooled on ice, a 10N aqueous solution of sodium hydroxide was added to adjust to pH 8 and the precipitated crystals were filtered to give 605 mg (79%) of Compound 23.

NMR (90MHz, DMSO-$d_6$) δ(ppm) 1.93 (2H, quint., J=6.7), 3.19 (2H, t, J=6.7), 3.71 (2H, t, J=6.8), 6.58 (1H, s), 6.68 (2H, d, J=8.8), 7.63 (1H, t, J=11.4), 7.73 (2H, d, J=8.8), 7.84 (4H, brs)

MS (M/Z) 475

Molecular formula $C_{26}H_{19}F_2N_3O_4$=475

Example 24

5-Amino-2-[4-[(3-aminopropyl)amino]phenyl]-6,8-difluoro-4H-1-benzopyran-4-one (Compund 24)

600 mg of Compound 23 was dissolved in 75 ml of a mixed solvent of dimethylformamide and methanol (1:1), 0.613 ml of hydrazine monohydrate was added and the mixture was stirred at 50-60 °C for 1.5 hours. Additional 0.307 ml of hydrazine monohydrate was added and the mixture was stirred at 50-60 °C for 10 minutes. The reaction solution was concentrated under reduced pressure, the residue was subjected to silica gel column chromatography (chloroform: methanol:aqueous ammonia=9:1:1) to give Compound 24 which was dissolved in ethanol, a 5.5N hydrochloric acid-isopropylalcohol solution was added and the precipitated crystals were filtered to give 331 mg (69%) of Compound 24.

IR (KBr) ν ($cm^{-1}$) 3446, 3328, 1650, 1633, 1609, 1567, 1483, 1394, 1313, 1191, 1121, 986

NMR (270MHz, DMSO-$d_6$) δ(ppm) 1.90 (2H, quint., J=7.3), 2.91 (2H, m), 3.23 (2H, t, J=6.9), 6.62 (1H, s), 6.76 (2H, d, J=8.9), 7.66 (1H, t, J=11.1), 7.77 (2H, d, J=8.9), 8.12 (2H, brs)

MS (M/Z) 345 ($M^+$)

Molecular formula $C_{18}H_{17}F_2N_3O_2$=345

Example 25

6,8-Difluoro-2-(3-fluoro-4-pivaloylaminophenyl)-5-pivaloylamino-4H-1-benzopyran-4-one (Compound 25)

A solution of 4.19 g of ethyl 3,5-difluoro-6-(N-ethoxycarbonyl-N-pivaloylamino)-2-methoxymethoxybenzoate obtained in Reference Example 3 and 1.98 g of 3'-fluoro-4'-pivaloylaminoacetophenone dissolved in 23 ml of dioxane was added dropwise to a suspension of 737 mg of sodium hydride (60% oil suspension) in 10 ml of dioxane under heating at reflux and the mixture was heated at reflux for 2.3 hours. The reaction solution was cooled, water was added, the aqueous layer was washed with n-hexane and extracted with ethyl acetate. The ethyl acetate layer was washed with an aqueous saturated solution of sodium chloride, dried over anhydrous sodium sulfate and subjected to silica gel column chromatography (chloroform:acetone=40:1-30:1) to give 3.37 g (75%) of 1,3-diketone.

3.37 g of the resulting 1,3-diketone was dissolved in 80 ml of ethanol, 20 ml of concentrated hydrochloric acid was added and the mixture was stirred at room temperature for 6.6 hours. The reaction solution was cooled on ice, 100 ml of water was added and the precipitated crystals were filtered to give 2.72 g (91%) of Compound 25.

NMR (90MHz, $CDCl_3$) δ(ppm) 1.36 (9H, s), 1.38 (9H, s), 6.68 (1H, s), 7.35 (1H, t, J=9.8), 7.5-7.9 (2H, m), 8.60 (1H, t, J=8.4)

MS (M/Z) 474 ($M^+$)

Molecular formula $C_{25}H_{25}F_3N_2O_4$=474

Example 26

5-Amino-2-(4-amino-3-fluorophenyl)-6,8-difluoro-4H-1-benzopyran-4-one (Compound 26)

3.90 g of Compound 25 was dissolved in 180 ml of dioxane, 180 ml of concentrated hydrochloric acid was added and the mixture was heated at reflux for 3 hours. The reaction solution was cooled on ice, the precipitated crystals were filtered and recrystallized from methanol/chloroform to give 1.31 g (52%) of Compound 26.

IR (KBr) ν($cm^{-1}$) 3313, 1625, 1600, 1560, 1525, 1484, 1390, 1308, 1120

NMR (270MHz, DMSO-$d_6$) δ(ppm) 6.69 (1H, s), 6.88 (1H, t, J=8.7), 7.5-7.8 (3H, m)

MS (M/Z) 306 ($M^+$)

Molecular formula $C_{15}H_9F_3N_2O_2$=306

Example 27

2-(4-Acetylamino-3-fluorophenyl)-5-amino-6,8-difluoro-4H-1-benzopyran-4-one (Compound 27)

1.66 g of Compound 26 was dissolved in 20 ml of pyridine, the solution was cooled on ice, 0.46 ml of acetic anhydride was added and the mixture was stirred at room temperature for 2.2 hours. Then, the reaction solution was added to 100 ml of ice water and the precipitated crystals were filtered to give 1.82 g (96%) of Compound 27.

NMR (90MHz, DMSO-$d_6$) δ(ppm) 2.15 (3H, s), 6.89 (1H, s), 7.03 (2H, brs), 7.5-8.0 (3H, m), 8.26 (1H, t, J=8.4), 9.99 (1H, brs)

MS (M/Z) 348 ($M^+$)

Molecular formula $C_{17}H_{11}F_3N_2O_3$=348

Example 28

2-[4-[N-Acetyl-N-(3-phthalimidopropyl)amino]-3-fluorophenyl]-5-amino-6,8-difluoro-4H-1-benzopyran-4-one (Compound 28)

1.81 g of Compound 27 was dissolved in 60 ml of dimethylformamide, 208 mg of sodium hydride (60% oil suspension), 1.67 g of N-(3-bromopropyl)phthalimide and 156 mg of sodium iodide were added and the mixture was stirred at room temperature for 2.5 hours. Additional 104 mg of sodium hydride (60% oil suspension) and 697 mg of N-(3-bromopropyl)phthalimide were added and the mixture was stirred at room temperature for 1.8 hours. Additional 62.4 mg of sodium hydride (60% oil suspension) and 418 mg of N-(3-bromopropyl)phthalimide were added and the mixture was stirred at room temperature for 2 hours. An aqueous solution of ammonium chloride was added to the reaction solution, the solvent was distilled off under reduced pressure and extracted once with chloroform. The chloroform layer was washed with an aqueous saturated solution of sodium chloride, dried over anhydrous sodium sulfate and the solvent was distilled off under reduced pressure. The residue was subjected to silica gel column chromatography (chloroform:methanol=100:1-60:1) and recrystallized from chloroform/n-hexane to give 1.72 g (62%) of Compound 28.

NMR (90MHz, $CDCl_3$) δ(ppm) 1.7-2.1 (2H, m), 1.90 (3H, s), 3.6-4.0 (4H, m), 6.23 (2H, brs), 6.63 (1H, s), 7.22 (1H, t, J=10.4), 7.49 (1H, t, J=7.41), 7.6-7.9 (6H, m)

MS (M/Z) 535 ($M^+$)

Molecular formula $C_{28}H_{20}F_3N_3O_5$=535

Example 29

5-Amino-6,8-difluoro-2-[3-fluoro-4-[(3-phthalimidopropyl)amino]phenyl]-4H-1-benzopyran-4-one (Compound 29)

1.0 g of Compound 28 was dissolved in 12 ml of dioxane, 8 ml of concentrated hydrochloric acid was added and the mixture was heated at reflux for 4.3 hours. The reaction solution was cooled on ice, a 10N aqueous solution of sodium hydroxide was added to adjust to pH 8 and the precipitated crystals were filtered to give 805 mg (87%) of Compound 29.

NMR (90MHz, DMSO-$d_6$) δ(ppm) 1.94 (2H, quint., J=6.6), 3.1-3.4 (2H, m), 3.69 (2H, t, J=6.6), 6.33 (1H, m), 6.69 (1H, s), 6.84 (1H, t, J=8.8), 7.00 (2H, brs), 7.4-7.8 (3H, m), 7.84 (4H, s)

MS (M/Z) 493 ($M^+$)

Molecular formula $C_{26}H_{18}F_3N_3O_4$=493

Example 30

5-Amino-2-[4-(3-aminopropylamino)-3-fluorophenyl]-6,8-difluoro-4H-1-benzopyran-4-one (Compound 30)

800 mg of Compound 29 was dissolved in 100 ml of a mixed solvent of dimethylformamide and methanol (1:1), 0.787 ml of hydrazine monohydrate was added and the mixture was stirred at 50-60 °C for 1 hour. The reaction solution was concentrated under reduced pressure, the residue was subjected to silica gel column chromatography (chloroform: methanol:aqueous ammonia=9:1:1) to give Compound 30 which was dissolved in ethanol, a 5.5N hydrochloric acid-isopropylalcohol solution was added and the precipitated crystals were filtered to give 321 mg (49%) of hydrochloride of Compound 30.

IR (KBr) ν($cm^{-1}$) 1652, 1618, 1558, 1538, 1481, 1390, 1309, 1118, 987

NMR (270MHz, DMSO-$d_6$) δ(ppm) 2.00 (2H, quint., J=7.1), 3.00 (2H, m), 3.4-3.6 (2H, m), 6.87 (1H, s), 7.07 (1H, t, J=8.9), 7.7-7.9 (3H, m)

MS (M/Z) 363 ($M^+$)

Molecular formula $C_{18}H_{16}F_3N_3O_2$=363

Example 31

2-[4-[N-acetyl-N-(3-phthalimidopropyl)amino]-3-fluorophenyl]-6,8-difluoro-5-hexylamino-4H-1-benzopyran-4-one (Compound 31)

450 mg of Compound 28 was dissolved in 15 ml of dimethylformamide, 0.62 ml of 1-iodohexane and 34 mg of sodium hydride (60% oil suspension) were added under ice-cooling and the mixture was stirred at room temperature for 2 hours. An aqeuous solution of ammonium chloride was added, the reaction solution was concentrated under reduced pressure and extracted once with chloroform. The chloroform layer was washed with an aqueous saturated solution of sodium chloride, dried over anhydrous sodium sulfate and concentrated under reduced pressure. The residue was subjected to silica gel column chromatography (n-hexane:ethyl acetate=1:1-1:3) to give 184 mg (35%) of Compound 31.

NMR (90MHz, $CDCl_3$) δ(ppm) 0.7-2.1 (11H, m), 1.90 (3H, s), 3.3-4.0 (6H, m), 6.59 (1H, s), 7.17 (1H, dd, J=13.4, 10.3), 7.48 (1H, t, J=7.9), 7.6-7.9 (6H, m), 8.73 (1H, brt)

MS (M/z) 619 ($M^+$)

Molecular formula $C_{34}H_{32}F_3N_3O_5$=619

Example 32

6,8-Difluoro-2-[3-fluoro-4-[(3-phthalimidopropyl)amino]phenyl]-5-hexylamino-4H-1-benzopyran-4-one (Compound 32)

200 mg of Compound 31 was dissolved in 4 ml of ethanol, 2 ml of concentrated hydrochloric acid was added and the mixture was heated at reflux for 4 hours. The reaction solution was cooled on ice, a 10N aqueous solution of sodium

hydroxide was added to adjust to pH 8, the precipitated crystals were filtered and subjected to preparative thin layer chromatography (chloroform:acetone=20:1) to give 63 mg (34%) of Compound 32.

NMR (90MHz, $CDCl_3$) δ(ppm) 0.8-1.8 (11H, m), 2.03 (2H, quint., J=6.4), 3.2-3.6 (4H, m), 3.84 (2H, t, J=6.4), 4.80 (1H, m), 6.42 (1H, s), 6.72 (1H, t, J=8.7), 7.10 (1H, dd, J=13.5, 10.4), 7.48 (1H, dd, J=10.4, 2.3), 7.6-8.0 (5H, m), 8.81 (1H, brt)

MS (M/z) 577 ($M^+$)

Molecular formula $C_{32}H_{30}F_3N_3O_4$=577

Example 33

2-[4-(3-Aminopropylamino)-3-fluorophenyl]-6,8-difluoro-5-hexylamino-4H-1-benzopyran-4-one (Compound 33)

60 mg of Compound 32 was dissolved in 30 ml of a mixed solvent of dimethylformamide and methanol, 0.050 ml of hydrazine monohydrate was added and the mixture was stirred at 60-70 °C for 4.3 hours. The reaction solution was concentrated under reduced pressure, the residue was subjected to preparative thin layer chromatography (chloroform: methanol:aqueous ammonia=9:1:1) to give Compound 33 which was dissolved in chloroform. A 5.5N hydrochloric acid-isopropylalcohol solution was added and the solvent was distilled off under reduced pressure to form the slurry with isopropyl ether from which 33.6 mg (67%) of hydrochloride of Compound 33 was obtained.

IR (KBr) ν($cm^{-1}$) 2920, 1651, 1610, 1527, 1277, 982, 860

NMR (270MHz, DMSO-$d_6$) δ(ppm) 0.86 (3H, t, J=6.7), 1.2-1.7 (10H, m), 1.8-2.0 (2H, m), 2.8-3.0 (2H, m), 3.3-3.5 (2H, m), 6.78 (1H, s), 6.90 (1H, t, J=8.7), 7.6-7.8 (3H, m)

MS (M/Z) 447 ($M^+$)

Molecular formula $C_{24}H_{28}F_3N_3O_2$=447

Example 34

5-Amino-2-(4-amino-3,5-difluorophenyl)-6,8-difluoro-4H-1-benzopyran-4-one (Compound 34)

600 mg of sodium hydride (60% oil suspension) was suspended in 20 ml of a mixed solvent of dioxane-toluene (1:1), a solution of 2.18 g (5.5 mmol) of ethyl 3,5-difluoro-6-(N-pivaloylamino)-2-(2-tetrahydropyranyloxy)benzoate (Compound E) obtained in Reference Example 4 and 1.28 g (5.0 mmol) of 3',5'-difluoro-4'-pivaloylaminoacetophenone dissolved in 20 ml of the above mixed solvent was added dropwise and the mixture was heated at reflux for 2 hours. The reaction solution was cooled, water was added, the aqueous layer was washed with n-hexane and extracted with ethyl acetate. The organic layer was washed with an aqueous saturated solution of sodium chloride, dried over anhydrous sodium sulfate and the solvent was distilled off under reduced pressure. The residue was purified by silica gel column chromatography (n-hexane:ethyl acetate=2:1-1:1) to give 1.07 g (36%) of 1,3-diketone.

1.06 g (1.78 mmol) of the resulting 1,3-diketone was dissolved in 9 ml of ethanol, 1 ml of concentrated hydrochloric acid was added, the mixture was stirred at room temperature for 5 hours, additional 1 ml of concentrated hydrochloric acid was added and the mixture was stirred at room temperature for 19 hours. Water was added to the reaction solution and the precipitated crystals were filtered to give 858 mg (98%) of 6,8-difluoro-2-(3,5-difluoro-4-pivaloylaminophenyl)-5-pivaloylamino-4H-1-benzopyran-4-one.

NMR (90MHz, $CDCl_3$) δ(ppm) 1.39 (18H, s), 6.62 (1H, s), 7.2-7.5 (3H, m), 7.75 (1H, brs), 10.4 (1H, brs)

MS (M/Z) 492 ($M^+$)

Molecular formula $C_{25}H_{24}F_4N_2O_4$=492

10 ml of dioxane and 5 ml of concentrated hydrochloric acid were added to the resulting 6,8-difluoro-2-(3,5-difluoro-4-pivaloylaminophenyl)-5-pivaloylamino-4H-1-benzopyran-4-one and the mixture was heated at reflux for 4 hours. Additional 5 ml of concentrated hydrochloric acid was added and the mixture was heated at reflux for 4 hours. A 10N aqueous solution of sodium hydroxide was added thereto to adjust to pH 9, the mixture was extracted with a mixed solvent of chloroform and methanol, the organic layer was washed with an aqueous saturated solution of sodium chloride, dried over anhydrous sodium sulfate and the solvent was distilled off under reduced pressure. The residue was purified by silica gel column chromatography (chloroform:methanol=20:1) and recrystallized from chloroform-methanol to give 224 mg (41%) of Compound 34.

NMR (270MHz, DMSO-$d_6$) δ(ppm) 6.17 (2H, brs), 6.80 (1H, s), 7.06 (2H, brs), 7.60 (2H, dd, J=7.7, 2.7), 7.71 (1H, t, J=11.1).

MS (M/Z) 324 ($M^+$)

Molecular formula $C_{15}H_8F_4N_2O_2$=324

Example 35

5-Amino-2-(4-amino-3,5-dichlorophenyl)-6,8-difluoro-4H-1-benzopyran-4-one (Compound 35)

780 mg of sodium hydride (60% oil suspension) was suspended in 6 ml of a mixed solvent of dioxane and toluene (1:1), a solution of 2.97 g (6.5 mmol) of ethyl 3,5-difluoro-6-pivaloylamino-2-(2-tetrahydropyranyloxy)-4-trimethylsilylbenzoate (Compound F) obtained in Reference Example 5 and 1.71 g (6.0 mmol) of 3',5'-dichloro-4'-pivaloylaminoacetophenone dissolved in 18 ml of the above mixed solvent was added dropwise under heating at reflux and the mixture was heated at reflux for 40 minutes. The reaction solution was cooled, water was added and the mixture was extracted with ethyl acetate. The organic layer was washed with an aqueous saturated solution of sodium chloride, dried over anhydrous sodium sulfate and the solvent was distilled off under reduced pressure to give 3.85 g of 1,3-diketone.

This was subjected to the subsequent reaction without purification. That is, the above compound was dissolved in 40 ml of ethanol, 10 ml of concentrated hydrochloric acid was added and the mixture was stirred at room temperature for 18 hours. Water was added to the reaction solution and the precipitated crystals were filtered to give 2.69 g of 2-(3,5-dichloro-4-pivaloylaminophenyl)-6,8-difluoro-5-pivaloylamino-7-trimethylsilyl-4H-l-benzopyran-4-one.

The above compound was dissolved in 50 ml of tetrahydrofuran, 1.7 ml of tetrabutylammonium fluoride (1M solution in tetrahydrofuran) was added under ice-cooling and the mixture was stirred at 0 °C for 20 minutes. Water was added thereto, the mixture was extracted with a mixed solvent of chloroform and methanol, the organic layer was washed once with water and once with an aqueous saturated solution of sodium chloride and dried over anhydrous sodium sulfate. The solvent was distilled off under reduced pressure to give 2.61 g (three stages 84%) of 2-(3,5-dichloro-4-pivaloylaminophenyl)-6,8-difluoro-5-pivaloylamino-4H-1-benzopyran-4-one.

NMR (90MHz, DMSO-$d_6$) δ(ppm) 1.28 (18H ,s), 7.25 (1H, s), 8.02 (1H, t, J=10.3), 8.16 (2H, s), 9.51 (1H, brs), 10.0 (1H, brs)

MS (M/Z) 524/526 ($M^+$)

Molecular formula $C_{25}H_{24}Cl_2F_2N_2O_4$=525

10 ml of concentrated sulfuric acid was added to the resulting 1.07 g (2.04 mmol) of 2-(3,5-dichloro-4-pivaloylaminophenyl)-6,8-difluoro-5-pivaloylamino-4H-1-benzopyran-4-one and the mixture was stirred at 100 °C for 16 hours. The reaction solution was poured into ice water and the mixture was extracted with a mixed solvent of chloroform and methanol. The organic layer was washed with an aqueous saturated solution of sodium chloride, dried over anhydrous sodium chloride and the solvent was distilled off under reduced pressure. The residue was purified by silica gel column chromatography (chloroform:methanol=50:1-6:1) and triturated with chloroform to give 546 mg (75%) of Compound 35.

NMR (270 MHz, DMSO-$d_6$) δ(ppm) 6.38 (2H, brs), 6.83 (1H, s), 7.06 (2H, brs), 7.72 (1H, t, J=11.1), 7.87 (2H, s)

MS (M/Z) 356/358/360 ($M^+$)

Molecular formula $C_{15}H_8Cl_2F_2N_2O_2$=357

Example 36

5-Amino-2-(4-amino-3,5-dibromophenyl)-6,8-difluoro-4H-1-benzopyran-4-one (Compound 36)

1.32 g of sodium hydride (60% oil suspension) was suspended in 15 ml of dioxane, a solution of 5.03 g (11 mmol) of ethyl 3,5-difluoro-6-pivaloylamino-2-(2-tetrahydropyranyloxy)-4-trimethylsilylbenzoate (Compound F) obtained in Reference Example 5 and 2.93 g (10 mmol) of 4'-amino-3',5'-dibromoacetophenone dissolved in 35 ml of dioxane was added dropwise under heating at reflux and the mixture was heated at reflux for 1 hour. The reaction solution was cooled, water was added and the mixture was extracted with ethyl acetate. The organic layer was washed with an aqueous saturated solution of sodium chloride, dried over anhydrous sodium sulfate and the solvent was distilled off under reduced pressure to give 7.07 g of 1,3-diketone.

This was subjeced to the subsequent reaction without purification. That is, the above compound was dissolved in 80 ml of ethanol, 20 ml of concentrated hydrochloric acid was added and the mixture was stirred at room temperature for 18 hours. Water was added to the reaction solution and the precipitated crystals were filtered to give 2.42 g (two stages 46%) of 2-(4-amino-3,5-dibromophenyl)-6,8-difluoro-5-pivaloylamino-4H-1-benzopyran-4-one.

20 ml of concentrated sulfuric acid was added to the resulting 1.00 g (1.89 mmol) of 2-(4-amino-3,5-dibromophenyl)-6,8-difluoro-5-pivaloylamino-4H-1-benzopyran-4-one and the mixture was stirred at 100 °C for 1.3 hours. Ice and a 10N aqueous solution of sodium hydroxide were added to the reaction solution to adjust to pH 9 and the precipitated crystals were filtered. The crystals were purified by silica gel column chromatography (chloroform:acetonitrile=40:1) and triturated with chloroform to give 378 mg (45%) of Compound 36.

NMR (270MHz, DMSO-$d_6$) δ(ppm) 6.17 (2H, brs), 6.84 (1H, s), 7.06 (2H, brs), 7.72 (1H, t, J=11.1), 8.06 (2H, s)

MS (M/Z) 444/446/448 ($M^+$)

Molecular formula $C_{15}H_8Br_2F_2N_2O_2$=446

Example 37

5-Amino-6,8-difluoro-2-(3-fluoro-4-methylaminophenyl)-4H-1-benzopyran-4-one (Compound 37)

500 mg (1.44 mmol) of Compound 27 obtained in Example 27 was dissolved in 30 ml of dimethylformamide under argon atmosphere and the solution was cooled on ice. 0.27 ml of iodomethane and 63 mg of sodium hydride (60% oil suspension) were added, the mixture was stirred at the same temperature for 1 hour and the temperature was risen to room temperature to stir for 2 hours. Water was added to the reaction solution and the mixture was extracted twice with ethyl acetate. The organic layer was washed once with water and once with an aqueous saturated solution of sodium chloride, dried over anhydrous magnesium sulfate, the solvent was distilled off and the tan residue was purified by silica gel column chromatography (n-hexane:ethyl acetate=3:2) to give 2-[4-(N-acetyl-N-methylamino)-3-fluorophenyl]-5-amino-6,8-difluoro-4H-1-benzopyran-4-one.

NMR (90MHz, $CDCl_3$) δ(ppm) 1.96 (3H, brs), 3.29 (3H, s), 6.23 (2H, brs), 6.63 (1H, s), 7.23 (1H, t, J=10.4), 7.40 (1H, t, J=7.9), 7.70 (1H, brs), 7.80 (1H, brs)

MS (M/Z) 362 ($M^+$)

Molecular formula $C_{18}H_{13}F_3N_2O_3$=362

500 mg (1.53 mmol) of the resulting 2-[4-(N-acetyl-N-methylamino)-3-fluorophenyl]-5-amino-6,8-difluoro-4H-1-benzopyran-4-one was dissolved in a mixed solvent of 10 ml of dioxane and 5 ml of concentrated hydrochloric acid and the solution was heated at reflux for 2.5 hours. The reaction solution was cooled, water was added to adjust to weak alkaline, the precipitated crystals were filtered and dried under reduced pressure. The resulting tan residue was purified twice by silica gel column chromatography (chloroform) to give 180 mg of Compound 37.

NMR (270 MHz, $CDCl_3$) 2.97 (3H, d, J=5.3), 4.45 (1H, brs), 6.19 (2H, brs), 6.48 (1H, s), 6.72 (1H, t, J=8.7), 7.16 (1H, t, J=10.6), 7.53 (1H, dd, J=12.7, 2), 7.63 (1H, dd, J=8.4, 1.8)

MS (M/Z) 320 ($M^+$)

Molecular formula $C_{16}H_{11}F_3N_2O_2$=320

Example 38

5-Amino-6,8-difluoro-2-[4-(N,N-dimethylamino)-3-fluorophenyl]-4H-1-benzopyran-4-one (Compound 38)

3.36 g of sodium hydride (60% oil suspension) was suspended in 50 ml of a mixed solvent of dioxane and toluene (1:1), a solution of 17.8 g (46 mmol) of ethyl 3,5-difluoro-6-pivaloylamino-2-(2-tetrahydropyranyloxy)benzoate obtained in Reference Example 4 and 7.61 g (42 mmol) of 4'-(N,N-dimethylamino)-3'-fluoroacetophenone dissolved in 160 ml of the above mixed solvent was added dropwise under heating at reflux and the mixture was heated at reflux for 1.5 hours. The reaction solution was cooled, water was added, the aqueous layer was washed with n-hexane and extracted with ethyl acetate. The organic layer was washed with an aqueous saturated solution of sodium chloride, dried over anhydrous sodium sulfate and the solvent was distilled off under reduced pressure to give 1,3-diketone.

The resulting 1,3-diketone was dissolved in 160 ml of ethanol, 40 ml of concentrated hydrochloric acid was added and the mixture was stirred at room temperature for 2 hours and at 40 °C for 30 minutes. The reaction solution was cooled on ice, a 10N aqueous solution of sodium hydroxide was added to adjust to pH 9 and the precipitated crystals were filtered to give 6.59 g (two stages 38%) of 6,8-difluoro-2-[4-(N,N-dimethylamino)-3-fluorophenyl]-5-pivaloylamino-4H-1-benzopyran-4-one.

NMR (90MHz, $CDCl_3$) δ(ppm) 1.38 (9H, s), 3.04 (6H, d, J=1.5), 6.58 (1H, s), 6.86 (1H, t, J=9.0), 7.32 (1H, t, J=10.0), 7.4-7.7 (2H, m), 10.6 (1H, brs)

MS (M/Z) 418 ($M^+$)

Molecular formula $C_{22}H_{21}F_3N_2O_3$=418

4.00 g (9.57 mmol)of the resulting 6,8-difluoro-2-[4-(N,N-dimethylamino)-3-fluorophenyl]-5-pivaloylamino-4H-1-benzopyran-4-one was dissolved in 60 ml of dioxane, 40 ml of concentrated hydrochloric acid was added and the mixture was heated at reflux for 6 hours. The reaction solution was cooled on ice, a 10N aqueous solution of sodium hydroxide was added to adjust to pH 9 and the mixture was extracted with chloroform. The organic layer was washed with an aqueous saturated solution of sodium chloride, dried over anhydrous sodium sulfate and the solvent was distilled off under reduced pressure. The residue was purified by silica gel column chromatography (chloroform:acetone=30:1) and recrystallized from chloroform/n-hexane to give 2.52 g (79%) of Compound 38.

NMR (270MHz, $CDCl_3$) δ(ppm) 3.05 (6H, d, J=1.5), 6.24 (2H, s), 6.51 (1H, s), 6.97 (1H, t, J=8.7), 7.18 (1H, t, J=10.6), 7.5-7.8 (2H, m)

MS (M/Z) 334 ($M^+$)

Molecular formula $C_{17}H_{13}F_3N_2O_2$=334

Example 39

5-Amino-6,8-difluoro-2-[3-fluoro-4-(2-methoxyethylamino)phenyl]-4H-1-benzopyran-4-one (Compound 39)

1.20 g of sodium hydride (60% oil suspension) was suspended in 30 ml of a mixed solvent of dioxane and toluene (1:1), a solution of 6.35 g (17 mmol) of ethyl 3,5-difluoro-6-pivaloylamino-2-(2-tetrahydropyranyloxy)benzoate (Compound E) obtained in Reference Example 4 and 3.80 g (15 mmol) of 4'-[N-acetyl-N-(2-methoxyethyl)amino]-3'-fluoroacetophenone dissolved in 100 ml of the above mixed solvent was added dropwise under heating at reflux and the mixture was heated at reflux for 1.5 hours. The reaction solution was cooled water was added, the aqueous layer was washed with n-hexane and extracted with five times with ethyl acetate. The organic layer was washed with an aqueous saturated solution of sodium chloride, dried over anhydrous sodium sulfate and the solvent was distilled off under reduced pressure. The residue was purified by silica gel column chromatography (n-hexane:ethyl acetate=2:1-1:2) to give 4.12 g (45%) of 1,3-diketone.

4.11 g (6.94 mmol) of the resulting 1,3-diketone was dissolved in 16 ml of ethanol, 4.0 ml of concentrated hydrochloric acid was added and the mixture was stirred at 40 °C for 45 minutes. The reaction solution was cooled on ice, water was added and the mixture was extracted with chloroform. The organic layer was washed with an aqueous saturated solution of sodium chloride, dried over anhydrous sodium sulfate and the solvent was distilled off under reduced pressure. The residue was purified by silica gel column chromatography (chloroform:methanol=40:1-30:1) to give 3.32 g (98%) of 2-[4-[N-acetyl-N-(2-methoxyethyl)amino]-3-fluorophenyl]-6,8-difluoro-5-pivaloylamino-4H-1-benzopyran-4-one.

NMR (90MHz, $CDCl_3$) δ(ppm) 1.39 (9H, s), 1.93 (3H, brs), 3.29 (3H, s), 3.5-4.1 (4H, m), 6.74 (1H, s), 7.39 (1H, t, J=10.0), 7.4-7.9 (3H, m), 10.4 (1H, brs)

MS (M/Z) 490 ($M^+$)

Molecular formula $C_{25}H_{25}F_3N_2O_5$=490

1.0 g (2.0 mmol) of the resulting 2-[4-[N-acetyl-N-(2-methoxyethyl)amino]-3-fluorophenyl]-6,8-difluoro-5-pivaloylamino-4H-1-benzopyran-4-one was dissolved in 10 ml of ethanol, 10 ml of concentrated hydrochloric acid was added and the mixture was heated at reflux for 4 hours. The reaction solution was cooled on ice, a 10N aqueous solution of sodium hydroxide was added to adjust to pH 9 and the mixture was extracted with a mixed solvent of chloroform and methanol. The organic layer was washed with an aqueous saturated solution of sodium chloride, dried over anhydrous sodium sulfate and the solvent was distilled off under reduced pressure. The residue was purified by silica gel column chromatography (chloroform:methanol=100:1-50:1) and recrystallized from n-hexane/ethyl acetate to give 278 mg (37%) of Compound 39.

NMR (270MHz, DMSO-$d_6$) δ(ppm) 3.29 (3H, s), 3.38 (2H, q, J=5.4), 3.52 (2H, t, J=5.7), 6.31 (1H, brs), 6.72 (1H, s), 6.89 (1H, t, J=8.9), 7.04 (2H, brs), 7.6-7.8 (3H, m)

MS (M/Z) 364 ($M^+$)

Molecular formula $C_{18}H_{15}F_3N_2O_3$=364

Example 40

5-Amino-2-[4-[2-(N',N'-dimethylamino)ethylamino]-3-fluorophenyl]-6,8-difluoro-4H-1-benzopyran-4-one (Compound 40)

1.30 g (3.74 mmol) of Compound 27 obtained in Example 27 was dissolved in 40 ml of dimethylformamide under argon atmosphere, 608 mg of sodium hydride (60% oil suspension) and 1.09 g of 2-dimethylaminoethyl chloride hydrochloride were added under ice-cooling and the mixture was stirred at room temperature for 1 hour and at 50-60 °C for 30 minutes. The reaction solution was cooled on ice, water was added and the mixture was extracted with ethyl acetate. The organic layer was washed with an aqueous saturated solution of sodium chloride, dried over anhydrous sodium sulfate and the solvent was distilled off under reduced pressure. The residue was purified by silica gel column chromatography (chloroform:methanol:triethylamine=100:10:1) and recrystallized from ethyl acetate/isopropyl ether to give 493 mg (31%) of 2-[4-[N-acetyl-N-[2-(N',N'-dimethylamino)ethyl]amino]-3-fluorophenyl]-5-amino-6,8-difluoro-4H-1-benzopyran-4-one.

NMR (90MHz, $CDCl_3$) δ(ppm) 1.90 (3H, brs), 2.19 (6H, s), 3.43 (2H, t, J=6.8), 3.80 (2H, m), 6.22 (2H, brs), 6.64 (1H, s), 7.22 (1H, t, J=10.4), 7.48 (1H, t, J=8.1), 7.7-7.9 (2H. m)

MS (M/Z) 419 ($M^+$)

Molecular formula $C_{21}H_{20}F_3N_3O_3$=419

485 mg (1.16 mmol) of the resulting 2-[4-[N-acetyl-N-[2-(N',N'-dimethylamino)ethyl]amino]-3-fluorophenyl]-5-amino-6,8-difluoro-4H-1-benzopyran-4-one was dissolved in 10 ml of dioxane, 5 ml of concentrated hydrochloric acid was added and the mixture was heated at reflux for 2.5 hours. The reaction solution was cooled on ice, a 10N aqueous

solution of sodium hydroxide was added to adjust to pH 9 and the mixture was extracted with chloroform. The organic layer was washed with an aqueous saturated solution of sodium chloride, dried over anhydrous sodium sulfate and the solvent was distilled off under reduced pressure. The residue was purified by silica gel column chromatography (chloroform:methanol:aqueous ammonia=50:1:1) to give Compound 40 which was converted into hydrochloride according to the same manner as that in Example 16 to give 204 mg (42%).

NMR (270MHz, DMSO-$d_6$) δ(ppm) 2.83 (6H, d, J=3.5), 3.2-3.4 (2H, m), 3.5-3.7 (2H, m), 6.63 (lH, brs), 6.78 (1H, s), 7.02 (1H, t, J=8.9), 7.05 (2H, brs), 7.6-7.8 (3H, m), 10.3 (lH, brs)

MS (M/Z) 377 ($M^+$)

Molecular formula $C_{19}H_{18}F_3N_3O_2$=377

Example 41

5-Amino-6,8-difluoro-2-[3-fluoro-4-[2-(pyrrolidin-1-yl)ethylamino]phenyl]-4H-1-benzopyran-4-one (Compound 41)

1.00 g (2.87 mmol) of Compound 27 obtained in Example 27 was dissolved in 25 ml of dimethylformamide under argon atmosphere, 0.98 g of 1-(2-chloroethyl)pyrrolidine hydrochloride and 0.97 g of potassium tert-butoxide were added under ice-cooling and the mixture was stirred at 70-80 °C for 3 hours. Additional 0.49 g of 1-(2-chloroethyl) pyrrolidine hydrochloride and 0.32 g of potassium tert-butoxide were added and the mixture was stirred at the same temperature for 15 hours. The reaction solution was cooled on ice, water was added and the mixture was extracted with ethyl acetate. The organic layer was washed with an aqueous saturated solution of sodium chloride, dried over anhydrous sodium sulfate and the solvent was distilled off under reduced pressure. The residue was purified by silica gel column chromatography (chloroform:methanol=40:1-9:1) to give 0.37 g (29%) of 2-[4-[N-acetyl-N-[2-(N',N'-dimethylamino)ethyl]amino]-3-fluorophenyl]-5-amino-6,8-difluoro-4H-1-benzopyran-4-one.

NMR (90MHz, $CDCl_3$) δ(ppm) 1.6-1.9 (4H, m), 1.91 (3H, s), 2.4-2.9 (6H, m), 3.7-4.1 (2H, m), 6.22 (2H, brs), 6.63 (1H, s), 7.22 (1H, dd, J=10.7, 10.4), 7.52 (1H, t, J=8.9), 7.5-7.8 (2H, m)

MS (M/Z) 445 ($M^+$)

Molecular formula $C_{23}H_{22}F_3N_3O_3$=445

0.37 g (0.83 mmol) of the resulting 2-[4-[N-acetyl-N-[2-(N',N'-dimethylamino)ethyl]amino]-3-fluorophenyl]-5-amino-6,8-difluoro-4H-1-benzopyran-4-one was dissolved in 20 ml of dioxane, 10 ml of concentrated hydrochloric acid was added and the mixture was heated at reflux for 2.5 hours. The reaction solution was cooled on ice, a 10N aqueous solution of sodium hydroxide was added to adjust to pH 9 and the mixture was extracted with chloroform. The organic layer was washed with an aqueous saturated solution of sodium chloride, dried over anhydrous sodium sulfate and the solvent was distilled off under reduced pressure. The residue was purified by silica gel column chromatography (chloroform:methanol=40:1-15:1) and recrystallized from ethyl acetate/n-hexane to give 137 mg (41%) of Compound 41. The resulting Compound 41 was converted into hydrochloride according to the same manner as that in Example 16.

NMR (270MHz, $CDCl_3$) δ(ppm) 1.81 (4H, m), 2.57 (4H, t, J=6.4), 2.80 (2H, t, J=5.7), 3.30 (2H, q, J=5.7), 5.05 (1H, brs), 6.20 (2H, brs), 6.47 (1H, s), 6.72 (1H, t, J=8.6), 7.16 (1H, t, J=10.4), 7.53 (1H, dd, J=12.7, 2.0), 7.61 (1H, dd, J=8.6, 2.0) (free base)

MS (M/Z) 403 ($M^+$)

Molecular formula $C_{21}H_{20}F_3N_3O_2$=403

Example 42

5-Amino-6,8-difluoro-2-[3-fluoro-4-(2-morpholinoethylamino)phenyl]-4H-1-benzopyran-4-one (Compound 42)

1.50 g (4.31 mmol) of Compound 27 obtained in Example 27 was dissolved in 30 ml of dimethylformamide under argon atmosphere, 517 mg of sodium hydride (60% oil suspension) and 1.60 g of 1-(2-chloroethyl)morpholine hydrochloride were added under ice-cooling and the mixture was stirred at 50-60 °C for 1.5 hours. The reaction solution was cooled on ice, water was added and the mixture was extracted with ethyl acetate. The organic layer was washed with an aqueous saturated solution of sodium chloride, dried over anhydrous sodium sulfate and the solvent was distilled off under reduced pressure. The residue was purified by silica gel column chromatography (chloroform:methanol=30:1) to give 1.47 g (74%) of 2-[4-[N-acetyl-N-(2-morpholinoethyl)amino]-3-fluorophenyl]-5-amino-6,8-difluoro-4H-1-benzopyran-4-one.

NMR (90MHz, $CDCl_3$) δ(ppm) 1.92 (3H, s), 2.3-2.6 (4H, m), 2.53 (2H, t, J=6.4), 3.60 (4H, t, J=4.6), 3.7-4.0 (2H, m), 6.22 (2H, brs), 6.64 (1H, s), 7.23 (1H, t, J=10.3), 7.50 (1H, t, J=7.9), 7.6-7.8 (2H, m)

MS (M/Z) 461 ($M^+$)

Molecular formula $C_{23}H_{22}F_3N_3O_4$=461

700 mg (1.52 mmol) of the resulting 2-[4-[N-acetyl-N-(2-morpholinoethyl)amino]-3-fluorophenyl]-5-amino-6,8-difluoro-4H-1-benzopyran-4-one was dissolved in 12 ml of dioxane, 8 ml of concentrated hydrochloric acid was added and the mixture was heated at reflux for 3.5 hours. The reaction solution was cooled on ice, a 10N aqueous solution of sodium hydroxide was added to adjust to pH 9 and the mixture was extracted with chloroform. The organic layer was washed with an aqueous saturated solution of sodium chloride, dried over anhydrous sodium sulfate and the solvent was distilled off under reduced pressure. The residue was purified by silica gel column chromatography (chloroform:methanol=20:1) and recrystallized from ethyl acetate to give Compound 42 which was converted into hydrochloride according to the same manner as that in Example 16 to give 315 mg (46%).

NMR (270MHz, DMSO-$d_6$) δ(ppm) 3.0-4.1 (12H, m), 6.79 (1H, s), 7.04 (1H, t, J=8.7), 7.6-7.8 (3H, m), 11.1 (1H, brs)

MS (M/Z) 419 ($M^+$)

Molecular formula $C_{21}H_{20}F_3N_3O_3$=419

Example 43

5-Amino-6,8-difluoro-2-[3-fluoro-4-(2-methylthioethylamino)phenyl]-4H-1-benzopyran-4-one (Compound 43)

1.00 g (2.87 mmol) of Compound 27 obtained in Example 27 was dissolved in 30 ml of dimethylformamide under argon atmosphere, 1.43 ml of chloroethyl methyl sulfide and 354 mg of potassium tert-butoxide were added under ice-cooling and the mixture was stirred at 50-60 °C for 1 hour. Additional 0.29 ml of chloroethyl methyl sulfide and 322 mg of potassium tert-butoxide were added and the mixture was stirred at the same temperature for 17 hours. The reaction solution was cooled on ice, water was added and the mixture was extracted with ethyl acetate. The organic layer was washed with an aqueous saturated solution of sodium chloride, dried over anhydrous sodium sulfate and the solvent was distilled off under reduced pressure. The residue was purified by silica gel column chromatography (chloroform: methanol=30:1) to give 380 mg (32%) of 2-[4-[N-acetyl-N-(2-methylthioethyl)amino]-3-fluorophenyl]-5-amino-6,8-difluoro-4H-1-benzopyran-4-one.

NMR (90MHz, $CDCl_3$) δ(ppm) 1.92 (3H, brs), 2.12 (3H, s), 2.67 (2H, brt, J=7.0), 3.91 (2H, brt, J=7.0), 6.22 (2H, brs), 6.64 (1H, s), 7.23 (1H, t, J=10.4), 7.47 (1H, t, J=8.0), 7.6-7.9 (2H, m)

MS (M/Z) 422 ($M^+$)

Molecular formula $C_{20}H_{17}F_3N_2O_3S$=422

380 mg (0.92 mmol) of the resulting 2-[4-[N-acetyl-N-(2-methylthioethyl)amino]-3-fluorophenyl]-5-amino-6,8-difluoro-4H-1-benzopyran-4-one was dissolved in 6 ml of dioxane, 4 ml of concentrated hydrochloric acid was added and the mixture was heated at reflux for 4 hours. The reaction solution was cooled on ice, a 10N aqueous solution of sodium hydroxide was added to adjust to pH 9 and the mixture was extracted with ethyl acetate. The organic layer was washed with an aqueous saturated solution of sodium chloride, dried over anhydrous sodium sulfate and the solvent was distilled off under reduced pressure. The residue was purified by silica gel column chromatography (chloroform:acetone=20:1) and recrystallized from ethyl acetate/n-hexane to give 58 mg (16%) of Compound 43.

NMR (270MHz, $CDCl_3$) δ(ppm) 2.16 (3H, s), 2.82 (2H, t, J=6.4), 3.47 (2H, t, J=6.4), 6.49 (1H, s), 6.76 (1H, t, J=8.4), 7.17 (1H, t, J=10.6), 7.55 (1H, dd, J=12.4, 2.0), 7.62 (1H, dd, J=8.4, 2.0)

MS (M/Z) 380 ($M^+$)

Molecular formula $C_{18}H_{15}F_3N_2O_2S$=380

Example 44

5-Amino-6,8-difluoro-2-(4-ethyoxycarbonylmethylamino-3-fluorophenyl)-4H-1-benzopyran-4-one (Compound 44)

3.48 g (10 mmol) of Compound 27 obtained in Example 27 was dissolved in 100 ml of dimethylformamide under argon atmosphere, 1.32 ml of ethyl bromoacetate and 1.23 g of potassium tert-butoxide were added under ice-cooling and the mixture was stirred at room temperature for 25 minutes. The reaction solution was cooled on ice, water was added and the mixture was extracted with ethyl acetate. The organic layer was washed with an aqueous saturated solution of sodium chloride, dried over anhydrous sodium sulfate and the solvent was distilled off under reduced pressure. The residue was triturated with ethyl acetate to give 3.20 g (57%) of 2-[4-(N-acetyl-N-ethoxycarbonylmethylamino)-3-fluorophenyl]-5-amino-6,8-difluoro-4H-1-benzopyran-4-one.

NMR (90MHz, $CDCl_3$) δ(ppm) 1.28 (3H, t, J=7.3), 1.99 (3H, s), 4.20 (2H, q, J=7.3), 4.38 (2H, brs), 5.19 (2H, brs), 6.63 (1H, s), 7.22 (1H, t, J=10.4), 7.5-7.9 (3H, m)

MS (M/Z) 434 ($M^+$)

Molecular formula $C_{21}H_{17}F_3N_2O_5$=434

2.0 g (4.6 mmol) of the resulting 2-[4-(N-acetyl-N-ethoxycarbonylmethylamino)-3-fluorophenyl]-5-amino-6,8-difluoro-4H-1-benzopyran-4-one was dissolved in 18 ml of ethanol, 12 ml of concentrated hydrochloric acid was added

and the mixture was heated at reflux for 5 hours. The reaction solution was cooled on ice, a 10N aqueous solution of sodium hydroxide was added to adjust to pH 9 and the precipitated crystals were filtered. The crystals were purified by silica gel column chromatography (chloroform:methanol=30:1) and recrystallized from ethyl acetate/n-hexane to give 821 mg (45%) of Compound 44.

NMR (270MHz, $CDCl_3$) δ(ppm) 1.33 (3H, t, J=7.2), 4.01 (2H, s), 4.29 (2H, q, J=7.2), 6.51 (1H, s), 6.64 (1H, t, J=8.4), 7.18 (1H, t, J=10.6), 7.5-7.7 (2H, m)

MS (M/Z) 392 ($M^+$)

Molecular formula $C_{19}H_{15}F_3N_2O_4$=392

Example 45

Sodium salt of 5-amino-2-(4-carboxymethylamino-3-fluorophenyl)-6,8-difluoro-4H-1-benzopyran-4-one (Compound 45)

30 ml of ethanol and 4.3 ml of a 2N aqueous solution of sodium hydroxide were added to 560 mg (1.43 mmol) of Compound 44 obtained in Example 44 and the mixture was stirred at 50 °C for 10 minutes. The reaction solution was cooled to room temperature and the precipitated crystals were filtered to give 533 mg (97%) of Compound 45.

NMR (270MHz, DMSO-$d_6$) δ(ppm) 3.36 (2H, s), 5.90 (1H, brs), 6.68 (1H, t, J=8.9), 7.05 (2H, brs), 7.6-7.8 (3H, m)

FAB-MS (M/Z) 365 ($M^+$+1)

Molecular formula $C_{17}H_{11}F_3N_2O_4$=364

Example 46

5-Amino-6,8-difluoro-2-[3-fluoro-4-(3-methoxypropylamino)phenyl]-4H-1-benzopyran-4-one (Compound 46)

943 mg of 1-chloro-3-methoxypropane and 1.30 g of sodium iodide were dissolved in 20 ml of dimethylformamide under argon atmosphere and the mixture was stirred at 90 °C for 2 hours. The reaction solution was cooled on ice and 1.51 g (4.35 mmol) of Compound 27 obtained in Example 27 and 89 mg of sodium hydride (60% oil suspension) were added thereto. The mixture was stirred at room temperature for 1 hour, 943 mg of 1-chloro-3-methoxypropane and 191 mg of sodium hydride (60% oil suspension) were added, the temperature was risen to 60 °C and the mixture was stirred for 2 days. Water was added to the reaction solution and the mixture was extracted twice with ethyl acetate. The organic layer was washed once with water and once with an aqueous saturated solution of sodium chloride and dried over anhydrous sodium sulfate. The solvent was distilled off under reduced pressure and the residue was purified by silica gel column chromatography (chloroform-chloroform:methanol=100:1) to give 1.18 g (65%) of 2-[4-[N-acetyl-N-(3-methoxypropyl)amino]-3-fluorophenyl]-5-amino-6,8-difluoro-4H-1-benzopyran-4-one.

NMR (90MHz, $CDCl_3$) δ(ppm) 1.6-2.1 (2H, m), 1.90 (3H, s), 3.27 (3H, s), 3.41 (2H, t, J=6.0), 3.81 (2H, t, J=7.4), 4.0-4.9 (2H, brs), 6.63 (1H, s), 7.22 (1H, t, J=10.4), 7.40 (1H, t, J=7.7), 7.69-7.80 (2H, m)

MS (M/Z) 420 ($M^+$)

Molecular formula $C_{21}H_{19}F_3N_2O_4$=420

501 mg (1.19 mmol) of the resulting 2-[4-[N-acetyl-N-(3-methoxypropyl)amino]-3-fluorophenyl]-5-amino-6,8-difluoro-4H-1-benzopyran-4-one was dissolved in 6 ml of dioxane, 6 ml of concentrated hydrochloric acid was added and the mixture was stirred for 3 hours under heating at reflux. The reaction solution was cooled on ice, water was added, pH of the solution was adjusted to 8, the precipitated crystals were filtered and purified by silica gel column chromatography (n-hexane:ethyl acetate=6:1) to give 279 mg (62%) of Compound 46.

NMR (270MHz, $CDCl_3$) δ(ppm) 1.95 (2H, quint., J=6.1), 3.34 (2H, t, J=6.2), 3.38 (3H, s), 3.54 (2H, t, J=5.7), 4.80 (1H, brs), 6.19 (2H, brs), 6.47 (1H, s), 6.73 (1H, t, J=8.4), 7.16 (1H, t, J=10.4), 7.52 (1H, dd, J=12.9, 2.0), 7.60 (1H, dd, J=8.4, 2.0)

MS (M/Z) 378 ($M^+$)

Molecular formula $C_{19}H_{17}F_3N_2O_3$=378

Example 47

5-Amino-6,8-difluoro-2-[4-[3-(N',N'-dimethylamino)propylamino]-3-fluorophenyl]-4H-1-benzopyran-4-one (Compound 47)

736 mg of sodium hydride (60% oil suspension) was suspended in a mixed solvent of 10 ml of dioxane and 10 ml of toluene under argon atmosphere and the suspension was heated at reflux. 4.03 g (8.8 mmol) of Compound F obtained in Reference Example 5 and 2.46 g (8.8 mmol) of 4'-[N-acetyl-N-[3-(N',N'-dimethylamino)propyl]amino]-3'-fluoroacetophenone dissolved in a mixed solvent of 25 ml of dioxane and 25 ml of toluene were added dropwise thereto and the mixture was stirred for 1.5 hours. The reaction solution was cooled, water was added, the mixture was extracted with ethyl acetate, the organic layer was washed with an aqueous saturated solution of sodium chloride, dried over anhydrous sodium sulfate and the solvent was distilled off under reduced pressure. The residue was purified by silica gel column chromatography (chloroform:methanol:aqueous ammonia=9:1:1) to give 3.40 g of the product as a mixture of two materials.

3.40 g of the resulting mixture was dissolved in 32 ml of ethanol, 8 ml of concentrated hydrochloric acid was added and the mixture was stirred at room temperature for 14 hours. Water and aqueous ammonia were added to the reaction solution and the mixture was extracted with a mixed solvent of chloroform and methanol (9:1). The organic layer was washed with an aqueous saturated solution of sodium chloride, dried over anhydrous sodium sulfate and the solvent was distilled off under reduced pressure. The residue was purified by silica gel column chromatography (chloroform: methanol:aqueous ammonia=40:1:1) to give 2.39 g of the product as a mixture of two materials.

1.07 g of the resulting mixture was dissolved in 8 ml of dioxane, 8 ml of concentrated hydrochloric acid was added and the mixture was stirred for 1 hour under heating at reflux. The reaction solution was cooled, water was added, pH of the solution was adjusted to weak alkaline and the mixture was extracted twice with ethyl acetate. The organic layer was washed once with water and once with an aqueous saturated solution of sodium chloride, dried over anhydrous sodium sulfate and the solvent was distilled off under reduced pressure. The residue was purified by silica gel column chromatography (chloroform:methanol:aqueous ammonia=50:1:1) and recrystallized from ethyl acetate/n-hexane to give 358 mg of yellow crystals.

352 mg of the above crystals were dissolved in 20 ml of tetrahydrofuran, 1.5 ml of tetrabutylammonium fluoride solution (1.0M) in tetrahydrofuran was added, the mixture was stirred at room temperature for 30 minutes, water was added and the mixture was extracted three times with ethyl acetate. The organic layer was washed with an aqueous saturated solution of sodium chloride, dried over anhydrous sodium sulfate and the solvent was distilled off under reduced pressure. The residue was purified by silica gel column chromatography (chloroform:methanol:aqueous ammonia=50:1:1) to 313 mg of Compound 47.

NMR (270MHz, $CDCl_3$) δ(ppm) 1.83 (2H, quint., J=6.4), 2.27 (6H, s), 2.44 (2H, t, J=6.4), 3.31 (2H, brq., J=6.4), 5.71 (1H, brs), 6.20 (2H, brs), 6.47 (1H, s), 6.71 (1H, t, J=8.4), 7.16 (1H, t, J=10.6), 7.51 (1H, dd, J=12.6, 2.2), 7.60 (1H, dd, J=8.7, 1.7)

MS (M/Z) 391 ($M^+$)

Molecular formula $C_{20}H_{20}F_3N_3O_2$=391

Example 48

5-Amino-6,8-difluoro-2-[3-fluoro-4-[3-(pyrrolidin-1-yl)propylamino]phenyl]-4H-1-benzopyran-4-one (Compound 48)

840 mg of sodium hydride (60% oil suspension) was suspended in a mixed solvent of 10 ml of dioxane and 10 ml of toluene under argon atmosphere and the suspension was heated at reflux. A solution of 3.85 g (10 mmol) of Compound E obtained in Reference Example 4 and 3.06 g (10 mmol) of 4'-[N-acetyl-N-[3-(pyrrolidin-1-yl)propyl]amino]-3'-fluoroacetophenone dissolved in a mixed solvent of 25 ml of dioxane and 25 ml of toluene was added dropwise thereto over 20 minutes and the mixture was stirred. After 2 hours, 80 mg of sodium hydride (60% oil suspension) was added and the mixture was stirred for additional 30 minutes. The reaction solution was cooled on ice, water was added, the mixture was washed with n-hexane and extracted with ethyl acetate. The organic layer was washed with an aqueous saturated solution of sodium chloride, dried over anhydrous magnesium sulfate and the solvent was distilled off under reduced pressure.

The resulting residue was dissolved in 50 ml of ethanol, 10 ml of concentrated hydrochloric acid was added and the mixture was stirred at room temperature for 7 hours and at 40 °C for 2 hours. Water was added to the reaction solution to adjust to pH 9 and the mixture was extracted with ethyl acetate. The organic layer was washed once with water and once with an aqueous saturated solution of sodium chloride, and dried over anhydrous magnesium sulfate. The solvent was distilled off under reduced pressure and the resulting residue was purified by silica gel column chromatography (chloroform:methanol:triethylamine=20:1:0.1) to give 2.42 g (45%) of 2-[4-[N-acetyl-N-[3-(pyrrolidin-1-yl) propyl]amino]-3-fluorophenyl]-6,8-difluoro-5-pivaloylamino-4H-1-benzopyran-4-one.

NMR (90MHz, $CDCl_3$) δ(ppm) 1.40 (9H, s), 1.1-2.1 (9H, m), 2.4-2.8 (4H, m), 3.1-3.6 (2H, m), 3.77 (2H, t, J=7.0), 6.73 (1H, s), 7.30-7.53 (2H, m), 7.60-7.82 (2H, m)

MS (M/Z) 543 ($M^+$)

Molecular formula $C_{29}H_{32}F_3N_3O_4$=543

1.00 g (1.84 mmol) of the resulting 2-[4-[N-acetyl-N-[3-(pyrrolidin-1-yl)propyl]amino]-3-fluorophenyl]-6,8-difluoro-5-pivaloylamino-4H-1-benzopyran-4-one was dissolved in 8 ml of dioxane, 8 ml of concentrated hydrochloric acid was added and the mixture was stirred for 1 hour under heating at reflux. The reaction solution was cooled, water was

added to adjust to pH 8 and the mixture was extracted with ethyl acetate. The organic layer was washed with an aqueous saturated solution of sodium chloride, dried over anhydrous magnesium sulfate and the solvent was distilled off under reduced pressure. The residue was purified by silica gel column chromatography (chloroform:methanol:acetic acid=9:2:0.1) to give 738 mg (96%) of Compound 48.

NMR (270MHz, $CDCl_3$) δ(ppm) 1.82-1.93 (6H, m), 2.56 (4H, m), 2.67 (2H, t, J=6.3), 3.2-3.4 (2H, m), 6.19 (3H, brs), 6.46 (1H, s), 6.69 (1H, t, J=8.6), 7.15 (1H, t, J=10.6), 7.51 (1H, dd, J=12.5, 2.0), 7.59 (1H, dd, J=8.6, 2.3)

MS (M/Z) 417 ($M^+$)

Molecular formula $C_{22}H_{22}F_3N_3O_2$=417

Example 49

5-Amino-6,8-difluoro-2-[3-fluoro-4-(3-methylthiopropylamino)phenyl]-4H-1-benzopyran-4-one (Compound 49)

840 mg of sodium hydride (60% oil suspension) was suspended in a mixed solvent of 10 ml of dioxane and 10 ml of toluene under argon atmosphere and the suspension was heated at reflux. A solution of 3.85 g (10 mmol) of Compound E obtained in Reference Example 4 and 2.83 g (10 mmol) of 4'-[N-acetyl-N-(3-methylthiopropyl)amino]-3'-fluoroacetophenone dissolved in a mixed solvent of 25 ml of dioxane and 25 ml of toluene was added dropwise and the mixture was stirred for 1 hour. The reaction solution was cooled on ice, water was added and the mixture was washed with n-hexane and extracted with ethyl acetate. The organic layer was washed with an aqueous saturated solution of sodium chloride, dried over anhydrous magnesium sulfate and the solvent was distilled off under reduced pressure.

The resulting residue was dissolved in 50 ml of ethanol, 10 ml of concentrated hydrochloric acid was added and the mixture was stirred at room temperature for 7 hours and at 40 °C for 2 hours. Water was added to the reaction solution and the mixture was extracted with ethyl acetate. The organic layer was washed once with water and once with an aqueous saturated solution of sodium chloride, dried over anhydrous magnesium sulfate. The solvent was distilled off under reduced pressure and the resulting residue was purified by silica gel column chromatography (n-hexane:ethyl acetate=2:1) to give 2.51 g (48%) of 2-[4-[N-acetyl-N-(3-methylthiopropyl)amino]-3-fluorophenyl]-6,8-difluoro-5-pivaloylamino-4H-1-benzopyran-4-one.

NMR (90MHz, $CDCl_3$) δ(ppm) 1.39 (9H, s), 1.7-2.1 (2H, m), 1.92 (3H, brs), 2.06 (3H, s), 2.52 (2H, t, J=7.5), 3.82 (2H, brt), 6.74 (1H, s), 7.28-7.52 (2H, m), 7.7-7.8 (2H, m), 10.4 (1H, brs)

MS (M/Z) 520 ($M^+$)

Molecular formula $C_{26}H_{27}F_3N_2O_4S$=520

1.00 g (1.92 mmol) of the resulting 2-[4-[N-acetyl-N-(3-methylthiopropyl)amino]-3-fluorophenyl]-6,8-difluoro-5-pivaloylamino-4H-1-benzopyran-4-one was dissolved in 8 ml of dioxane, 8 ml of concentrated hydrochloric acid was added and the mixture was stirred for 3 hours under heating at reflux. The reaction solution was cooled, water was added to adjust to pH 8 and the precipitated crystals were filtered. The crystals were purified by silica gel column chromatography (n-hexane:ethyl acetate=2:1) and recrystallized twice from ethyl acetate/n-hexane to give 422 mg (56%) of Compound 49.

NMR (270MHz, $CDCl_3$) δ(ppm) 1.98 (2H, quint., J=6.9), 2.14 (3H, s), 2.64 (2H, t, J=6.9), 3.36-3.43 (2H, m), 5.23 (1H, brs), 6.20 (2H, brs), 6.47 (1H, s), 6.77 (1H, t, J=8.4), 7.16 (1H, t, J=10.6), 7.53 (1H, dd, J=12.9, 2.2), 7.61 (1H, dd, J=7.9, 2.0)

MS (M/Z) 394 ($M^+$)

Molecular formula $C_{19}H_{17}F_3N_2O_2S$=394

Example 50

5-Amino-6,8-difluoro-2-[3-fluoro-4-[3-(imidazol-1-yl)propylamino]phenyl]-4H-1-benzopyran-4-one (Compound 50)

1.02 g (2.92 mmol) of Compound 27 obtained in Example 27 was dissolved in 25 ml of dimethylformamide under argon atmosphere, 129 mg of sodium hydride (60% oil suspension) and 2.9 ml of 1-bromo-3-chloropropane were added under ice-cooling and the mixture was stirred at room temperature for 1 hour. Water was added to the reaction solution and the mixture was extracted twice with ethyl acetate. The organic layer was washed once with water and once with an aqueous saturated solution of sodium chloride, dried over anhydrous sodium sulfate and the solvent was distilled off under reduced pressure. Recrystallization from ethyl acetate/acetonitrile afforded 434 mg (35%) of 2-[4-[N-acetyl-N-(3-chloropropyl)amino]-3-fluorophenyl]-5-amino-6,8-difluoro-4H-1-benzopyran-4-one.

NMR (90MHz, $CDCl_3$) δ(ppm) 1.91 (3H, brs), 2.04 (2H, quint., J=7.0), 3.57 (2H, t, J=6.5), 3.7-4.0 (2H, m), 6.22 (2H, brs), 6.64 (1H, s), 7.23 (1H, t, J=10.4), 7.40 (1H, t, J=7.9), 7.72-7.82 (2H, m)

MS (M/Z) 424 ($M^+$)

Molecular formula $C_{20}H_{16}ClF_3N_2O_3$=424

508 mg (1.20 mmol) of the resulting 2-[4-[N-acetyl-N-(3-chloropropyl)amino]-3-fluorophenyl]-5-amino-6,8-difluoro-4H-1-benzopyran-4-one was dissolved in 30 ml of dioxane, 30 ml of concentrated hydrochloric acid was added and the mixture was stirred for 3 hours under heating at reflux. The reaction solution was cooled on ice, water was added to adjust to pH 8 and the precipitated crystals were filtered. The crystals were purified by silica gel column chromatography (n-hexane:ethyl acetate=4:1) to give 224 mg (49%) of 5-amino-2-[4-(3-chloropropylamino)-3-fluorophenyl]-6,8-difluoro-4H-1-benzopyran-4-one.

NMR (90MHz, $CDCl_3$) δ(ppm) 2.13 (2H, quint., J=6.3), 3.47 (2H, q, J=6.4), 3.68 (2H, J=6.0), 4.45 (1H, m), 6.19 (2H, brs), 6.47 (1H, s), 6.78 (1H, t, J=8.7), 7.15 (1H, t, J=10.5), 7.45-7.67 (2H, m)

MS (M/Z) 382 ($M^+$)

Molecular formula $C_{18}H_{14}ClF_3N_2O_2$=382

190 mg (0.50 mmol) of the resulting 5-amino-2-[4-(3-chloropropylamino)-3-fluorophenyl]-6,8-difluoro-4H-1-benzopyran-4-one was dissolved in 1.5 ml of dimethylformamide under argon atmosphere under ice-cooling, 17 mg of imidazole and 10 mg of sodium hydride (60% oil suspension) were added and the mixture was stirred for 40 minutes. The temperature of the reaction was risen to room temperature, additional 10 mg of imidazole and 6.8 mg of sodium hydride (60% oil suspension) were added and the mixture was stirred overnight. The reaction mixture was heated to 40 °C, 10 mg of imidazole and 6.8 mg of sodium hydride (60% oil suspension) were added and the mixture was stirred for 30 hours. Water was added to the reaction solution, the mixture was extracted with ethyl acetate, the organic layer was washed with an aqueous saturated solution of sodium chloride, dried over anhydrous sodium sulfate and the solvent was distilled off under reduced pressure. The residue was purified by preparative thin layer chromatography (chloroform:methanol=20:1) to give 18.3 mg (53%) of Compound 50.

NMR (270MHz, DMSO-$d_6$) δ(ppm) 2.02 (2H, quint., J=6.9), 3.15 (2H, q, J=6.9), 4.07 (2H, t, J=6.9), 6.53 (1H, brs), 6.74 (1H, s), 6.79 (1H, t, J=8.9), 6.92 (1H, s), 7.06 (2H, brs), 7.22 (1H, s), 7.6-7.8 (4H, m)

MS (M/Z) 414 ($M^+$)

Molecular formula $C_{21}H_{17}F_3N_4O_2$=414

Example 51

5-Amino-2-[4-(tert-butoxycarbonylamino)-3-fluorophenyl]-6,8-difluoro-4H-1-benzopyran-4-one (Compound 51)

3.03 g (8.70 mmol) of Compound 27 obtained in Example 27 was dissolved in 200 ml of dimethylformamide, 109 mg of dimethylaminopyridine and 3.0 ml of di-tert-butyl carbonate were added and the mixture was stirred at room temperature for 2 hours. Water was added to the reaction solution and the precipitated crystals were filtered to give 3.44 g (88%) of 2-[4-[N-acetyl-N-(tert-butoxycarbonyl)amino]-3-fluorophenyl]-5-amino-6,8-difluoro-4H-1-benzopyran-4-one.

NMR (90MHz, $CDCl_3$) δ(ppm) 1.41 (9H, s), 2.66 (3H, s), 6.20 (2H, brs), 6.63 (1H, s), 7.10-7.33 (2H, m), 7.65-7.76 (2H, m)

MS (M/Z) 448 ($M^+$)

Molecular formula $C_{22}H_{19}F_3N_2O_5$=448

3.43 g (7.65 mmol) of the resulting 2-[4-[N-acetyl-N-(tert-butoxycarbonyl)amino]-3-fluorophenyl]-5-amino-6,8-difluoro-4H-1-benzopyran-4-one was dissolved in 400 ml of acetonitrile, 2.2 ml of 2-(N,N-diethylamino)ethylamine was added and the mixture was stirred at room temperature for 4 hours. The precipitated crystals were dissolved in ethyl acetate, the solvent was distilled off under reduced pressure and the resulting residue was recrystallized from ethyl acetate to give 2.00 g (64%) of Compound 51.

NMR (90MHz, $CDCl_3$) δ(ppm) 1.55 (9H, s), 6.20 (2H, brs), 6.56 (1H, s), 6.90 (1H, brs), 7.19 (1H, t, J=10.4), 7.56-7.71 (2H, m), 8.30 (1H, t, J=8.4)

MS (M/Z) 406 ($M^+$)

Molecular formula $C_{20}H_{17}F_3N_2O_4$=406

Example 52

5-Amino-6,8-difluoro-2-[3-fluoro-4-[6-(imidazol-1-yl)hexylamino]phenyl]-4H-1-benzopyran-4-one (Compound 52)

5.00 g (14.4 mmol) of Compound 27 obtained in Example 27 was dissolved in 150 ml of dimethylformamide under argon atmosphere, 10.9 ml of 1,6-dibromohexane and 575 mg of sodium hydride (60% oil suspension) were added under ice-cooling and the mixture was stirred at room temperature for 2 hours. Water was added to the reaction solution and the mixture was extracted with ethyl acetate. The organic layer was washed once with water and once with an aqueous saturated solution of sodium chloride and dried over anhydrous sodium sulfate. The solvent was distilled off under reduced pressure and the residue was purified by silica gel column chromatography (chloroform:acetone=20:

1) to give 6.11 g (83%) of 2-[4-[N-acetyl-N-(6-bromohexyl)amino]-3-fluorophenyl]-5-amino-6,8-difluoro-4H-1-benzopyran-4-one.

NMR (90MHz, $CDCl_3$) δ(ppm) 1.2-2.0 (8H, m), 1.90 (3H, s), 3.38 (2H, t, J=6.6), 3.6-3.9 (2H, m), 6.64 (1H, s), 7.23 (1H, t, J=10.4), 7.38 (1H, t, J=8.1), 7.6-7.9 (2H, m)

MS (M/Z) 510/512 ($M^+$)

Molecular formula $C_{23}H_{22}BrF_3N_2O_3$=511

6.10 g (11.9 mmol) of the resulting 2-[4-[N-acetyl-N-(6-bromohexyl)amino]-3-fluorophenyl]-5-amino-6,8-difluoro-4H-1-benzopyran-4-one was dissolved in 60 ml of dioxane, 40 ml of 47% aqueous HBr was added and the mixture was heated at reflux for 3 hours. The reaction solution was cooled on ice, water was added and the mixture was extracted with ethyl acetate. The organic layer was washed once with 5% aqueous sodium bicarbonate and once with an aqueous saturated solution of sodium chloride and dried over anhydrous sodium sulfate. The solvent was distilled off under reduced pressure, and the residue was purified by silica gel column chromatography (chloroform:methanol=100:1) to give 3.53 g (63%) of 5-amino-2-[4-(6-bromohexylamino)-3-fluorophenyl]-6,8-difluoro-4H-1-benzopyran-4-one.

NMR (90MHz, $CDCl_3$) δ(ppm) 1.2-2.1 (8H, m), 3.24 (2H, brt, J=6.0), 3.42 (2H, t, J=6.5), 4.30 (1H, brs), 6.10 (2H, brs), 6.46 (1H, s), 6.72 (1H, t, J=8.7), 7.15 (1H, t, J=10.3), 7.4-7.7 (2H, m)

MS (M/Z) 468/470 ($M^+$)

Molecular formula $C_{21}H_{20}BrF_3N_2O_2$=469

3.53 g (7.53 mmol) of the resulting 5-amino-2-[4-(6-bromohexylamino)-3-fluorophenyl]-6,8-difluoro-4H-1-benzopyran-4-one was dissolved in 100 ml of tetrahydrofuran under argon atmosphere, 5.12 g of imidazole and 3.01 g of sodium hydride (60% oil suspension) were added and the mixture was heated at reflux for 2 hours. The reaction solution was cooled on ice, poured into aqueous ammonium chloride and extracted with ethyl acetate. The organic layer was washed with an aqueous saturated solution of sodium chloride and dried over anhydrous sodium sulfate. The solvent was distilled off under reduced pressure and the residue was purified by silica gel column chromatography (chloroform: methanol:triethylamine=90:10:1) to give Compound 52 which was converted into hydrochloride according to the same manner as that in Example 16 to give 3.13 g (84%).

NMR (270MHz, DMSO-$d_6$) δ(ppm) 1.2-1.5 (4H, m), 1.58 (2H, quint., J=7.4), 1.82 (2H, quint., J=7.4), 3.17 (2H, t, J=6.9), 4.20 (2H, t, J=7.4), 6.72 (1H, s), 6.82 (1H, t, J=8.9), 7.6-7.8 (5H, m), 9.19 (1H, s)

MS (M/Z) 456 ($M^+$)

Molecular formula $C_{24}H_{23}F_3N_4O_2$=456

Example 53

5-Amino-6,8-difluoro-2-[4-[6-(N',N'-dimethylamino)hexylamino]-3-fluorophenyl]-4H-1-benzopyran-4-one (Compound 53)

500 mg (1.07 mmol) of 5-amino-2-[4-(6-bromohexylamino)-3-fluorophenyl]-6,8-difluoro-4H-1-benzopyran-4-one obtained in Example 52 was dissolved in 15 ml of dimethylformamide, 1.47 g of potassium carbonate and 869 mg of dimethylamine hydrochloride were added and the mixture was stirred at room temperature for 16 hours. The reaction solution was poured into an aqueous ammonium chloride solution and extracted with ethyl acetate. The organic layer was washed with an aqueous saturated solution of sodium chloride, dried over anhydrous sodium sulfate and the solvent was distilled off under reduced pressure. The residue was purified by silica gel column chromatography (chloroform:methanol:triethylamine=90:10:1) and recrystallized from chloroform/methanol/n-hexane to give Compound 53 which was converted into hydrochloride according to the same manner as that in Example 16 to give 117 mg (23%).

NMR (270MHz, DMSO-$d_6$) δ(ppm) 1.3-1.5 (4H, m), 1.5-1.8 (4H, m), 2.73 (6H, s), 3.00 (2H, brt, J=8.2), 3.19 (2H, q, J=6.9), 6.42 (1H, brt), 6.72 (1H, s), 6.84 (1H, t, J=8.9), 7.05 (2H, brs), 7.6-7.8 (3H, m)

MS (M/Z) 433 ($M^+$)

Molecular formula $C_{23}H_{26}F_3N_3O_2$=433

Example 54

5-Amino-6,8-difluoro-2-[3-fluoro-4-[6-(pyrrolidin-1-yl)hexylamino]phenyl]-4H-1-benzopyran-4-one (Compound 54)

500 mg (1.07 mmol) of 5-amino-2-[4-(6-bromohexylamino)-3-fluorophenyl]-6,8-difluoro-4H-1-benzopyran-4-one obtained in Example 52 was dissolved in 15 ml of tetrahydrofuran, 0.891 ml of pyrrolidine was added and the mixture was heated at reflux for 1.5 hours. Water was added to the reaction solution and the mixture was extracted with a mixed solvent of chloroform and methanol. The organic layer was washed with an aqueous saturated solution of sodium chloride, dried over anhydrous sodium sulfate and the solvent was distilled off under reduced pressure. The residue

was purified by silica gel column chromatography (chloroform:methanol:triethylamine=90:10:1) and recrystallized from chloroform/n-hexane to give Compound 54 which was converted into hydrochloride according to the same manner as that in Example 16 to give 88 mg (17%).

NMR (270MHz, $CDCl_3$) δ(ppm) 1.3-1.5 (4H, m), 1.59 (2H, quint., J=7.3), 1.69 (2H, quint., J=7.3), 1.82 (4H, quint., J=3.3), 2.50 (2H, t, J=7.8), 2.58 (4H, m), 3.23 (2H, q, J=6.9), 4.38 (1H, m), 6.20 (2H,brs), 6.47 (1H, s), 6.72 (1H, t, J=8.6), 7.16 (1H, t, J=10.4), 7.53 (1H, dd, J=12.9, 2.0), 7.60 (1H, dd, J=8.6, 2.0)

MS (M/Z) 459 ($M^+$)

Molecular formula $C_{25}H_{28}F_3N_3O_2$=459

Example 55

5-Amino-6,8-difluoro-2-[3-fluoro-4-(6-morpholinohexylamino)phenyl]-4H-1-benzopyran-4-one (Compound 55)

500 mg (1.07 mmol) of 5-amino-2-[4-(6-bromohexylamino)-3-fluorophenyl]-6,8-difluoro-4H-1-benzopyran-4-one obtained in Example 52 was dissolved in 15 ml of dimethylformamide, 0.93 ml of morpholine was added and the mixture was stirred at 50-60 °C for 1.5 hours. Water was added to the reaction solution and the mixture was extracted with ethyl acetate. The organic layer was washed with an aqueous saturated solution of sodium chloride, dried over anhydrous sodium sulfate and the solvent was distilled off under reduced pressure. The residue was purified by silica gel column chromatography (chloroform:methanol:triethylamine=200:10:1) and recrystallized from ethyl acetate/n-hexane to give Compound 55 which was converted into hydrochloride according to the same manner as that in Example 16 to give 349 mg (62%).

NMR (270MHz, $CDCl_3$) δ(ppm) 1.3-1.5 (4H, m), 1.56 (2H, quint., J=7.1), 1.70 (2H, quint., J=7.0), 2.39 (2H, t, J=7.6), 2.50 (4H, brs), 3.23 (2H, q, J=6.6), 3.75 (4H, t, J=4.6), 4.37 (1H, m), 6.20 (2H, s), 6.47 (1H, s), 6.72 (1H, t, J=8.6), 7.16 (1H, t, J=10.6), 7.53 (1H, dd, J=12.9, 2.0), 7.60 (1H, dd, J=8.6, 2.0)

MS (M/Z) 475 ($M^+$)

Molecular formula $C_{25}H_{28}F_3N_3O_3$=475

Example 56

5-Amino-6,8-difluoro-2-[4-(5-ethoxycarbonylpentylamino)-3-fluorophenyl]-4H-1-benzopyran-4-one (Compound 56)

1.00 g (2.87 mmol) of Compound 27 obtained in Example 27 was dissolved in 10 ml of dimethylformamide under argon atmosphere, 355 mg of potassium tert-butoxide and 1.02 ml of ethyl 6-bromohexanoate were added under ice-cooling and the mixture was stirred at room temperature for 4.5 hours. An aqueous ammonium chloride solution was added to the reaction solution and the mixture was extracted with ethyl acetate. The organic layer was washed with an aqueous saturated solution of sodium chloride, dried over anhydrous sodium sulfate and purified by silica gel column chromatography (chloroform:methanol=30:1) to give 1.26 g (85%) of 2-[4-[N-acetyl-N-(5-ethoxycarbonylpentyl)amino]-3-fluorophenyl]-5-amino-6,8-difluoro-4H-1-benzopyran-4-one.

NMR (90MHz, $CDCl_3$) δ(ppm) 1.2-1.8 (6H, m), 1.24 (3H, t, J=7.0), 1.90 (3H, brs), 2.27 (2H, t, J=7.0), 3.71 (2H, t, J=6.8), 4.10 (2H, q, J=7.0), 6.22 (2H, brs), 6.64 (1H, s), 7.23 (1H, t, J=10.4), 7.37 (1H, t, J=8.1), 7.6-7.9 (2H, m)

MS (M/Z) 490 ($M^+$)

Molecular formula $C_{25}H_{25}F_3N_2O_5$=490

1.20 g (2.45 mmol) of the resulting 2-[4-[N-acetyl-N-(5-ethoxycarbonylpentyl)amino]-3-fluorophenyl]-5-amino-6,8-difluoro-4H-1-benzopyran-4-one was dissolved in 10 ml of ethanol, 5 ml of concentrated hydrochloric acid was added and the mixture was heated at reflux for 2.5 hours. The reaction solution was cooled on ice, water was added and the mixture was extracted with ethyl acetate. The organic layer was washed with an aqueous saturated solution of sodium chloride, dried over anhydrous sodium sulfate and the solvent was distilled off under reduced pressure. The residue was purified by silica gel column chromatography (chloroform:acetone=20:1) and recrystallized from ethyl acetate/n-hexane to give 386 mg (35%) of Compound 36.

NMR (270MHz, $CDCl_3$) δ(ppm) 1.26 (3H, t, J=6.9), 1.4-1.8 (6H, m), 2.34 (2H, t, J=7.4), 3.24 (2H, q, J=6.9), 4.14 (2H, q, J=6.9), 4.38 (1H, m), 6.29 (2H, brs), 6.47 (1H, s), 7.16 (1H, t, J=10.4), 7.53 (1H, dd, 12.9, 2.0), 7.60 (1H, dd, J=8.4, 2.0)

MS (M/Z) 448 ($M^+$)

Molecular formula $C_{23}H_{23}F_3N_2O_4$=448

Example 57

Sodium salt of 5-amino-2-[4-(5-carboxypentylamino)-3-fluorophenyl]-6,8-difluoro-4H-1-benzopyran-4-one (Compound 57)

367 mg (0.818 mmol) of Compound 56 obtained in Example 56 was dissolved in 10 ml of ethanol, 1.23 ml of a 2N aqueous solution of sodium hydroxide was added and the mixture was stirred at room temperature for 17 hours. The precipitated crystals were filtered to give 261 mg (69%) of Compound 57.

NMR (270MHz, DMSO-$d_6$) δ(ppm) 1.2-1.7 (6H, m), 1.90 (2H, t, J=7.4), 3.16 (2H, q, J=6.4), 6.43 (1H, m), 6.70 (1H, s), 6.81 (1H, t, J=8.4), 7.04 (2H, brs), 7.6-7.8 (3H, m)

FAB-MS (M/Z) 421 ($M^+$+1)

Molecular formula $C_{21}H_{19}F_3N_2O_4$=420

Example 58

6,8-Difluoro-2-[4-(N,N-dimethyl)amino-3-fluorophenyl]-5-hexylamino-4H-1-benzopyran-4-one (Compound 58)

500 mg (1.50 mmol) of Compound 38 obtained in Example 38 was dissolved in 5 ml of dimethylformamide under argon atmosphere, 120 mg of sodium hydride (60% oil suspension) and 0.441 ml of 1-iodohexane were added under ice-cooling and the mixture was stirred at room temperature for 3 hours. An aqueous saturated solution of ammonium chloride was added to the reaction solution and the mixture was extracted with ethyl acetate. The organic layer was washed with an aqueous saturated solution of sodium chloride, dried over anhydrous sodium sulfate and the solvent was distilled off under reduced pressure. The residue was purified by silica gel column chromatography (n-hexane: ethyl acetate=6:1) and recrystallized from n-hexane/ethyl acetate to give Compound 58 which was converted into hydrochloride according to the same manner as that in Example 16 to give 494 mg (79%).

NMR (270MHz, DMSO-$d_6$) δ(ppm) 0.86 (3H, t, J=6.7), 1.2-1.5 (6H, m), 1.54 (2H, quint., J=6.9), 2.96 (6H, d, J=1.5), 3.2-3.5 (2H, m), 6.81 (1H, s), 7.00 (1H, t, J=9.2), 7.6-7.8 (2H, m), 7.68 (1H, t, J=11.1), 8.82 (1H, brs)

MS (M/Z) 418 ($M^+$)

Molecular formula $C_{23}H_{25}F_3N_2O_2$=418

Example 59

5-(6-Bromohexylamino)-6,8-difluoro-2-[4-(N,N-dimethyl)amino-3-fluorophenyl]-4H-1-benzopyran-4-one (Compound 59)

100 mg (1.50 mmol) of Compound 38 obtained in Example 38 was dissolved in 2 ml of dimethylformamide under argon atmosphere, 24 mg of sodium hydride (60% oil suspension) and 2 ml of 1,6-dibromohexane were added under ice-cooling and the mixture was stirred at 0 °C for 2 hours. An aqueous saturated solution of ammonium chloride was added to the reaction solution and the mixture was extracted with ethyl acetate. The organic layer was washed with an aqueous saturated solution of sodium chloride, dried over anhydrous sodium sulfate and the solvent was distilled off under reduced pressure. The residue was purified by silica gel column chromatography (n-hexane:ethyl acetate=4:1) and recrystallized from n-hexane/ethyl acetate to give 92mg (62%) of Compound 59.

NMR (270MHz, $CDCl_3$) δ(ppm) 1.4-1.8 (8H, m), 3.01 (6H, d, J=1.7), 3.4-3.6 (2H, m), 3.41 (2H, t, J=6.8), 6.47 (1H, s), 6.86 (1H, t, J=8.9), 7.12 (1H, dd, J=13.4, 10.4), 7.4-7.6 (2H, m), 8.82 (1H, brs)

MS (M/Z) 496/498 ($M^+$)

Molecular formula $C_{23}H_{24}BrF_3N_2O_2$=497

Example 60

6,8-Difluoro-2-[4-(N,N-dimethyl)amino-3-fluorophenyl]-5-(6-hydroxyhexylamino)-4H-l-benzopyran-4-one (Compound 60)

1.0 g (2.99 mmol) of Compound 38 obtained in Example 38 was dissolved in 20 ml of dimethylformamide under argon atmosphere, 239 mg of sodium hydride (60% oil suspension) and 1.87 g of tetrahydropyranyl ether of 6-iodo-1-hexanol were added under ice-cooling and the mixture was stirred at 0 °C for 1.3 hours. An aqueous saturated solution of ammonium chloride was added to the reaction solution and the mixture was extracted with ethyl acetate. The organic layer was washed with an aqueous saturated solution of sodium chloride, dried over anhydrous sodium sulfate and the solvent was distilled off under reduced pressure. 18 ml of ethanol and 2 ml of concentrated hydrochloric

acid were added to the residue and the mixture was stirred at room temperature for 18 hours. A 10N aqueous solution of sodium hydroxide was added to the reaction solution to adjust to pH 7 and the mixture was extracted with ethyl acetate. The organic layer was washed with an aqueous saturated solution of sodium chloride, dried over anhydrous sodium sulfate and the solvent was distilled off under reduced pressure. The residue was purified by silica gel column chromatography (chloroform:methanol=40:1) and recrystallized from n-hexane/ethyl acetate to give 912 mg (70%) of Compound 60.

NMR (270MHz, $CDCl_3$) δ(ppm) 1.3-1.8 (8H, m), 3.02 (6H, d, J=1.5), 3.45 (2H, m), 3.65 (2H, t, J=6.4), 6.48 (1H, s), 6.89 (1H, t, J=8.9), 7.13 (1H, dd, J=13.4, 9.9), 7.5-7.7 (2H, m), 8.82 (1H, brs)

MS (M/Z) 434 ($M^+$)

Molecular formula $C_{23}H_{25}F_3N_2O_3$=434

Example 61

6,8-Difluoro-2-[4-(N,N-dimethyl)amino-3-fluorophenyl]-5-(6-methoxyhexylamino)-4H-1-benzopyran-4-one (Compound 61)

500 mg (1.15 mmol) of Compound 60 obtained in Example 60 was dissolved in 20 ml of tetrahydrofuran under argon atmosphere, 92 mg of sodium hydride (60% oil suspension) and 0.14 ml of iodomethane were added under ice-cooling and the mixture was stirred at room temperature for 1 hour. Thereafter, 0.22 ml of iodomethane was added and the mixture was heated at reflux for 3.5 hours. Additional 0.36 ml of iodomethane was added and the mixture was heated at reflux for 1 hour. The solvent was distilled off under reduced pressure, an aqueous saturated solution of ammonium chloride was added to the residue and the mixture was extracted with ethyl acetate. The organic layer was washed with an aqueous saturated solution of sodium chloride, dried over anhydrous sodium sulfate, purified by silica gel column chromatography (chloroform:acetone=40:1) and recrystallized from n-hexane/ethyl acetate to give 300 mg (58%) of Compound 61.

NMR (270MHz, $CDCl_3$) δ(ppm) 1.3-1.8 (8H, m), 3.01 (6H, d, J=1.5), 3.33 (3H, s), 3.37 (2H, t, J=6.7), 3.4-3.6 (2H, m), 6.47 (1H, s), 6.86 (1H, t, J=8.9), 7.12 (1H, dd, J=13.4, 10.4), 7.5-7.8 (2H, m), 8.82 (1H, brs)

MS (M/Z) 448 ($M^+$)

Molecular formula $C_{24}H_{27}F_3N_2O_3$=448

Example 62

6,8-Difluoro-2-[4-(N,N-dimethyl)amino-3-fluorophenyl]-5-[6-(N',N'-dimethylamino)hexylamino]-4H-1-benzopyran-4-one (Compound 62)

500 mg (0.01 mmol) of Compound 59 obtained in Example 59 was dissolved in 25 ml of dimethylformamide, 1.39 g of potassium carbonate and 820 mg of dimethylamine hydrochloride were added and the mixture was stirred at room temperature for 5.5 hours. 971 mg of potassium carbonate and 410 mg of dimethylamine hydrochloride were added and the mixture was stirred at room temperature for 16 hours. The reaction solution was poured in water, the precipitated crystals were filtered, purified by silica gel column chromatography (chloroform:methanol:triethylamine=90:10:1) and recrystallized from chloroform/ethyl acetate to give Compound 62 which was converted into hydrochloride according to the same manner as that in Example 16 to give 283 mg (53%).

NMR (270MHz, $CDCl_3$) δ(ppm) 1.3-1.8 (8H, m), 2.21 (6H,s), 2.24 (2H, t, J=6.9), 3.01 (6H, d, J=1.3), 3.4-3.5 (2H, m), 6.46 (1H, s), 6.86 (1H, t, J=8.7), 7.12 (1H, dd, J=13.5, 10.2), 7.5-7.7 (2H, m), 8.81 (1H, brt)

MS (M/Z) 461 ($M^+$)

Molecular formula $C_{25}H_{30}F_3N_3O_2$=461

Example 63

6,8-Difluoro-2-[4-(N,N-dimethyl)amino-3-fluorophenyl]-5-[6-(pyrrolidin-1-yl)hexylamino]-4H-1-benzopyran-4-one (Compound 63)

500 mg (1.01 mmol) of Compound 59 obtained in Example 59 was dissolved in 5 ml of tetrahydrofuran, 0.17 ml of pyrrolidine was added and the mixture was heated at reflux. At 1 hour and 1.7 hours after the reaction was started, each 0.17 ml of pyrrolidine was added. At 3.5 hours and 4.5 hours, each 0.08 ml of pyrrolidine was added. The mixture was heated at reflux for total 5.5 hours. Water was added to the reaction solution and the mixture was extracted with chloroform. The organic layer was washed with an aqueous saturated solution of sodium chloride, dried over anhydrous sodium sulfate and the solvent was distilled off under reduced pressure. The residue was purified by silica gel column

chromatography (chloroform:methanol:triethylamine=90:10:1) and triturated with n-hexane to give Compound 63 which was converted into hydrochloride according to the same manner as that in Example 16 to give 465 mg (83%).

NMR (270MHz, $CDCl_3$) δ(ppm) 1.3-2.0 (12H, m), 2.4-2.7 (6H, m), 3.01 (6H, d, J=1.5), 3.4-3.5 (2H, m), 6.47 (1H, s), 6.86 (1H, t, J=8.7), 7.12 (1H, dd, J=13.4, 9.9), 7.5-7.7 (2H, m), 8.81 (1H, brt)

MS (M/Z) 487 ($M^+$)

Molecular formula $C_{27}H_{32}F_3N_3O_2$=487

Example 64

6,8-Difluoro-2-[4-(N,N-dimethyl)amino-3-fluorophenyl]-5-[6-(imidazol-1-yl)hexylamino]-4H-1-benzopyran-4-one (Compound 64)

500 mg (1.01 mmol) of Compound 59 obtained in Example 59 was dissolved in 10 ml of tetrahydrofuran, 137 mg of imidazole and 80 mg of sodium hydride (60% oil suspension) were added and the mixture was heated at reflux. At 1.5 hours after the reaction was started, 40 mg of sodium hydride (60% oil suspension) was added and the mixture was heated at reflux for total 2.3 hours. An aqueous solution of ammonium chloride was added to the reaction solution and the mixture was extracted with chloroform. The organic layer was washed with an aqueous saturated solution of sodium chloride, dried over anhydrous sodium sulfate and the solvent was distilled off under reduced pressure. The residue was purified by silica gel column chromatography (chloroform:methanol:triethylamine=200:10:1) and triturated with n-hexane to give Compound 64 which was converted into hydrochloride according to the same manner as that in Example 16 to give 447 mg (73%).

NMR (270MHz, DMSO-$d_6$) δ(ppm) 1.2-1.5 (4H, m), 1.5-1.7 (2H, m), 1.7-1.9 (2H, m), 2.98 (6H, d, J=1.5), 3.3-3.5 (2H, m), 4.19 (2H, t, J=7.2), 6.80 (1H, s), 7.01 (1H, t, J=9.2), 7.6-7.8 (5H, m), 9.15 (1H, s)

MS (M/Z) 484 ($M^+$)

Molecular formula $C_{26}H_{27}F_3N_4O_2$=484

Example 65

5-(6-Aminohexylamino)-6,8-difluoro-2-[4-(N,N-dimethyl)amino-3-fluorophenyl]-4H-1-benzopyran-4-one (Compound 65)

500 mg (1.01 mmol) of Compound 59 obtained in Example 59 was dissolved in 10 ml of tetrahydrofuran under argon atmosphere, 372 mg of potassium phthalimide was added under ice-cooling and the mixture was stirred at room temperature for 45 minutes and at 60-70 °C for 1.5 hours. 10 ml of dimethylformamide was added and the mixture was stirred at 70-80 °C for 2 hours. Water was added to the reaction solution and the mixture was extracted with ethyl acetate. The organic layer was washed with an aqueous saturated solution of sodium chloride, dried over anhydrous sodium sulfate and the solvent was distilled off under reduced pressure.

The above product was dissolved in a mixed solvent of 10 ml of methanol and 10 ml of dimethylformamide, 0.59 ml of hydrazine monohydrate was added and the mixture was stirred at 60-70 °C for 1.7 hours. The solvent was distilled off under reduced pressure and the residue was purified by silica gel column chromatography (chloroform-chloroform: methanol:ammonia=90:5:5) to give Compound 65 which was converted into hydrochloride according to the same manner as that in Example 16 to give 275 mg (two stages 54%).

NMR (270MHz, DMSO-$d_6$) δ(ppm) 1.3-1.7 (8H, m), 2.7-2.9 (2H, m), 2.98 (6H, d, J=1.6), 3.3-3.5 (2H, m), 6.82 (1H, s), 7.01 (1H, t, J=9.1), 7.6-7.8 (2H, m), 7.71 (1H, dd, J=13.9, 10.9), 7.88 (1H, brs)

MS (M/Z) 433 ($M^+$)

Molecular formula $C_{23}H_{26}F_3N_3O_2$=433

Example 66

2-(4-Amino-3-fluorophenyl)-6,8-difluoro-5-pentylamino-4H-1-benzopyran-4-one (Compound 66)

5.00 g (16.3 mmol) of Compound 26 obtained in Example 26 was dissolved in 50 ml of pyridine, 2.2 ml of pivaloyl chloride was added under ice-cooling and the mixture was stirred at room temperature for 1.5 hours. Water was added to the reaction solution and the precipitated crystals were filtered to give 6.35 g (100%) of 5-amino-6,8-difluoro-2-(3-fluoro-4-pivaloylaminophenyl)-4H-1-benzopyran-4-one.

NMR (90MHz, $CDCl_3$) δ(ppm) 1.36 (9H, s), 6.19 (2H, brs), 6.58 (1H, s), 7.20 (1H, dd, J=10.8, 10.3), 7.5-7.9 (3H, m), 8.59 (1H, t, J=8.6)

MS (M/Z) 390 ($M^+$)

Molecular formula $C_{20}H_{17}F_3N_2O_3$=390

505 mg (1.29 mmol) of the resulting 5-amino-6,8-difluoro-2-(3-fluoro-4-pivaloylaminophenyl)-4H-1-benzopyran-4-one was dissolved in 15 ml of dimethylformamide under argon atmosphere, 155 mg of sodium hydride (60% oil suspension) and 0.35 ml of 1-iodopentane were added and the mixture was stirred at the same temperature for 30 minutes. 0.17 ml of 1-iodopentane was added and the mixture was stirred for additional 2 hours. An aqueous saturated solution of ammonium chloride was added to the reaction solution and the mixture was extracted twice with ethyl acetate. The organic layer was washed with an aqueous saturated solution of sodium chloride, dried over anhydrous sodium sulfate and the solvent was distilled off under reduced pressure. The residue was purified by silica gel column chromatography (chloroform) to give 333 mg (56%) of 6,8-difluoro-2-(3-fluoro-4-pivaloylaminophenyl)-5-pentylamino-4H-1-benzopyran-4-one.

NMR (90MHz, $CDCl_3$) δ(ppm) 0.8-1.0 (3H, m), 1.36 (9H, s), 1.2-1.9 (6H, m), 3.38-3.49 (2H, m), 6.66 (1H, s), 7.29 (1H, t, J=10.1), 7.5-7.9 (3H, m), 8.52 (1H, t, J=8.6)

MS (M/Z) 460 ($M^+$)

Molecular formula $C_{25}H_{27}F_3N_2O_3$=460

6 ml of dioxane and 6 ml of concentrated hydrochloric acid were added to 390 mg (0.85 mmol) of the resulting 6,8-difluoro-2-(3-fluoro-4-pivaloylaminophenyl)-5-pentylamino-4H-1-benzopyran-4-one and the mixture was heated at reflux for 1 hour. The reaction solution was cooled on ice, water was added to adjust the solution to neutral. The mixture was extracted twice with ethyl acetate, the organic layer was washed with an aqueous saturated solution of sodium chloride, dried over anhydrous sodium sulfate and the solvent was distilled off under reduced pressure. The residue was purified by silica gel column chromatography (n-hexane:ethyl acetate=5:1) to give 292 mg (92%) of Compound 66.

NMR (270MHz, $CDCl_3$) δ(ppm) 0.91 (3H, t, J=7.2), 1.3-1.5 (4H, m), 1.6-1.8 (2H, m), 3.41-3.48 (2H, m), 6.47 (1H, s), 6.84 (1H, t, J=8.7), 7.15 (1H dd, J=12.9, 9.9), 7.50 (1H, dd, J=7.9, 2.0), 7.54 (1H, dd, J=13.4, 2.0)

MS (M/Z) 376 ($M^+$)

Molecular formula $C_{20}H_{19}F_3N_2O_2$=376

Example 67

2-(4-Amino-3-fluorophenyl)-6,8-difluoro-5-hexylamino-4H-1-benzopyran-4-one (Compound 67)

505 mg (1.29 mmol) of 5-amino-6,8-difluoro-2-(3-fluoro-4-pivaloylaminophenyl)-4H-1-benzopyran-4-one was dissolved in 15 ml of dimethylformamide under argon atmosphere, 156 mg of sodium hydride (60% oil suspension) and 0.38 ml of 1-iodohexane were added under ice-cooling and the mixture was stirred at the same temperature for 30 minutes. 0.19 ml of 1-iodohexane was added and the mixture was stirred for additional 2 hours. An aqueous saturated solution of ammonium chloride was added to the reaction solution and the mixture was extracted twice with ethyl acetate. The organic layer was washed with an aqueous saturated solution of sodium chloride, dried over anhydrous sodium sulfate and the solvent was distilled off under reduced pressure. The residue was purified by silica gel column chromatography (n-hexane:ethyl acetate=5:1) to give 487 mg (79%) of 6,8-difluoro-2-(3-fluoro-4-pivaloylaminophenyl)-5-hexylamino-4H-1-benzopyran-4-one.

NMR (90MHz, $CDCl_3$) δ(ppm) 0.8-1.0 (3H, m), 1.36 (9H, s),1.2-1.9 (8H, m), 3.38-3.49 (2H, m), 6.66 (1H, s), 7.29 (1H, t, J=10.1), 7.5-7.9 (2H, m), 8.52 (1H, t, J=8.6)

MS (M/Z) 474 ($M^+$)

Molecular formula $C_{26}H_{29}F_3N_2O_3$=474

6 ml of dioxane and 6 ml of concentrated hydrochloric acid were added to 470 mg (0.99 mmol) of the resulting 6,8-difluoro-2-(3-fluoro-4-pivaloylaminophenyl)-5-hexylamino-4H-1-benzopyran-4-one and the mixture was heated at reflux for 1 hour. The reaction solution was cooled on ice, water was added to adjust the solution to neutral. The mixture was extracted twice with ethyl acetate, the organic layer was washed with an aqueous saturated solution of sodium chloride, dried over anhydrous sodium sulfate and the solvent was distilled off under reduced pressure. The residue was purified by silica gel column chromatography (n-hexane:ethyl acetate=4:1) to give 377 mg (87%) of Compound 67.

NMR (270MHz, $CDCl_3$) δ(ppm) 0.89 (3H, t, J=6.7), 1.2-1.6 (6H, m), 1.66 (2H, quint., J=7.2), 3.41-3.48 (2H, m), 6.47 (1H, s), 6.84 (1H, t, J=8.7), 7.15 (1H, dd, J=13.1, 10.1), 7.50 (1H, dd, J=7.9, 2.0), 7.54 (1H, dd, J=13.9, 2.0)

MS (M/Z) 390 ($M^+$)

Molecular formula $C_{21}H_{21}F_3N_2O_2$=390

Example 68

2-(4-Amino-3-fluorophenyl)-6,8-difluoro-5-heptylamino-4H-1-benzopyran-4-one (Compound 68)

505 mg (1.29 mmol) of 5-amino-6,8-difluoro-2-(3-fluoro-4-pivaloylaminophenyl)-4H-1-benzopyran-4-one obtained

in Example 66 was dissolved in 15 ml of dimethylformamide under argon atmosphere, 156 mg of sodium hydride (60% oil suspension) and 0.42 ml of 1-iodoheptane were added under ice-cooling and the mixture was stirred for 2 hours. An aqueous saturated solution of sodium chloride was added to the reaction solution and the mixture was extracted twice with ethyl acetate. The organic layer was washed with an aqueous saturated solution of sodium chloride, dried over anhydrous sodium sulfate and the solvent was distilled off under reduced pressure. The residue was purified by silica gel column chromatography (n-hexane:ethyl acetate=5:1) to give 460 mg (73%) of 6,8-difluoro-2-(3-fluoro-4-pivaloylaminophenyl)-5-heptylamino-4H-1-benzopyran-4-one.

NMR (90MHz, $CDCl_3$) δ(ppm) 0.8-1.9 (13H, m), 1.36 (9H, s), 3.40-3.60 (2H, m), 6.57 (1H, s), 7.17 (1H, dd, J=13.1, 10.2), 7.58-7.75 (3H, m), 8.59 (1H, t, J=8.6)

MS (M/Z) 488 ($M^+$)

Molecular formula $C_{27}H_{31}F_3N_2O_3$=488

6 ml of dioxane and 6 ml of concentrated hydrochloric acid were added to 460 mg (0.94 mmol) of the resulting 6,8-difluoro-2-(3-fluoro-4-pivaloylaminophenyl)-5-heptylamino-4H-1-benzopyran-4-one and the mixture was heated at reflux for 1.5 hours. The reaction solution was cooled on ice, water was added, and the precipitated crystals were filtered and purified by silica gel column chromatography (n-hexane:ethyl acetate=6:1) to give 358 mg (94%) of Compound 68.

NMR (270MHz, $CDCl_3$) δ(ppm) 0.88 (3H, t, J=6.9), 1.2-1.5 (8H, m), 1.5-1.7 (2H, m), 3.39-3.48 (2H, m), 4.17 (2H, brs), 6.45 (1H, s), 6.83 (1H, t, J=8.7), 7.12 (1H, dd, J=13.4, 10.4), 7.50-7.58 (2H, m), 8.81 (1H, brs)

MS (M/Z) 404 ($M^+$)

Molecular formula $C_{22}H_{23}F_3N_2O_2$=404

Example 69

2-(4-Amino-3-fluorophenyl)-5-(5-chlorohexylamino)-6,8-difluoro-4H-1-benzopyran-4-one (Compound 69)

512 mg (1.31 mmol) of 5-amino-6,8-difluoro-2-(3-fluoro-4-pivaloylaminophenyl)-4H-1-benzopyran-4-one obtained in Example 66 was dissolved in 15 ml of dimethylformamide under argon atmosphere, 160 mg of sodium hydride (60% oil suspension) and 0.35 ml of 6-bromo-1-hexene were added under ice-cooling and the mixture was stirred at room temperature for 2 hours. An aqueous saturated solution of ammonium chloride was added to the reaction solution and the mixture was extracted three times with ethyl acetate. The organic layer was washed with an aqueous saturated solution of sodium chloride, dried over anhydrous sodium sulfate and the solvent was distilled off under reduced pressure. The residue was purified by silica gel column chromatography (n-hexane:ethyl acetate=6:1) to give 409 mg (66%) of 6,8-difluoro-2-(3-fluoro-4-pivaloylaminophenyl)-5-(5-hexenylamino)-4H-1-benzopyran-4-one.

NMR (90MHz, $CDCl_3$) δ(ppm) 1.36 (9H, s), 1.3-2.3 (6H, m), 3.3-3.6 (2H, m), 4.88-5.11 (2H, m), 5.6-6.0 (1H, m), 6.64 (1H, s), 7.26 (1H, t, J=11.1), 7.5-7.9 (3H, m), 8.62 (1H, t, J=8.6)

MS (M/Z) 472 ($M^+$)

Molecular formula $C_{26}H_{27}F_3N_2O_3$=472

6 ml of dioxane and 6 ml of concentrated hydrochloric acid were added to 440 mg (0.93 mmol) of the resulting 6,8-difluoro-2-(3-fluoro-4-pivaloylaminophenyl)-5-(5-hexenylamino)-4H-1-benzopyran-4-one and the mixture was heated at reflux for 1.5 hours. The reaction solution was cooled on ice, the precipitated crystals were filtered, purified by silica gel column chromatography (n-hexane:ethyl acetate=8:1) and recrystallized from n-hexane/ethyl acetate and subsequently n-hexane/chloroform to give 291 mg (74%) of Compound 69.

NMR (270MHz, $CDCl_3$) δ(ppm) 1.51 (3H, d, J=6.4), 1.4-1.8 (6H, m), 3.42-3.50 (2H, m), 4.04 (1H, sextet., J=6.4), 4.18 (2H, brs), 6.46 (1H, s), 6.84 (1H, t, J=8.7), 7.13 (1H, dd, J=13.4, 10.4), 7.50-7.58 (2H, m), 8.83 (1H, brs) MS (M/Z) 424 ($M^+$)

Molecular formula $C_{21}H_{20}ClF_3N_2O_2$=424

Example 70

2-(4-Amino-3-fluorophenyl)-6,8-difluoro-5-isopentylamino-4H-1-benzopyran-4-one (Compound 70)

510 mg (1.32 mmol) of 5-amino-6,8-difluoro-2-(3-fluoro-4-pivaloylaminophenyl)-4H-1-benzopyran-4-one obtained in Example 66 was dissolved in 15 ml of dimethylformamide under argon atmosphere, 160 mg of sodium hydride (60% oil suspension) and 0.35 ml of isoamyl bromide were added under ice-cooling and the mixture was stirred at room temperature for 1.5 hours. 0.16 ml of isoamyl bromide was added and the mixture was stirred for additional 1 hour. An aqueous saturated solution of ammonium chloride was added to the reaction solution and the mixture was extracted twice with ethyl acetate. The organic layer was washed with an aqueous saturated solution of sodium chloride, dried over anhydrous sodium sulfate and the solvent was distilled off under reduced pressure. The residue was purified by

silica gel column chromatography (n-hexane:ethyl acetate=5:1) to give 310 mg (51%) of 6,8-difluoro-2-(3-fluoro-4-pivaloylaminophenyl)-5-isopentylamino-4H-1-benzopyran-4-one.

NMR (90MHz, $CDCl_3$) δ(ppm) 0.94 (6H, d, J=5.9), 1.36 (9H, s), 1.2-2.1 (3H, m), 3.38-3.53 (2H, m), 6.54 (1H, s), 7.15 (1H, dd, J=13.3, 10.2), 7.5-7.8 (3H, m), 8.57 (1H, t, J=8.6)

MS (M/Z) 460 ($M^+$)

Molecular formula $C_{25}H_{27}F_3N_2O_3$

6 ml of dioxane and 6 ml of concentrated hydrochloric acid were added to 283 mg (0.61 mmol) of the resulting 6,8-difluoro-2-(3-fluoro-4-pivaloylaminophenyl)-5-isopentylamino-4H-1-benzopyran-4-one and the mixture was heated at reflux for 1 hour. The reaction solution was cooled on ice, water was added to adjust the solution to neutral, and the precipitated crystals were filtered and purified by silica gel column chromatography (n-hexane:ethyl acetate=4:1-3:1) to give 207 mg (89%) of Compound 70.

NMR (270MHz, $CDCl_3$) δ(ppm) 0.94 (6H, d, J=6.4), 1.53 (2H, td, J=7.4, 6.9), 1.72 (1H, m), 3.46 (2H, m), 4.17 (2H, brs), 6.45 (1H, s), 6.83 (1H, t, J=8.9), 7.12 (1H, dd, J=13.4, 10.4), 7.50-7.58 (2H, m), 8.77 (1H, brs)

MS (M/Z) 376 ($M^+$)

Molecular formula $C_{20}H_{19}F_3N_2O_2$=376

Example 71

2-(4-Amino-3-fluorophenyl)-6,8-difluoro-5-(4-methylpentylamino)-4H-1-benzopyran-4-one (Compound 71)

515 mg (1.32 mmol) of 5-amino-6,8-difluoro-2-(3-fluoro-4-pivaloylaminophenyl)-4H-1-benzopyran-4-one obtained in Example 66 was dissolved in 15 ml of dimethylformamide under argon atmosphere, 160 mg of sodium hydride (60% oil suspension) and 0.38 ml of 1-bromo-4-methylpentane were added under ice-cooling and the mixture was stirred at room temperature for 2 hours. An aqueous saturated solution of ammonium chloride was added to the reaction solution and the mixture was extracted twice with ethyl acetate. The organic layer was washed with an aqueous saturated solution of sodium chloride, dried over anhydrous sodium sulfate and the solvent was distilled off under reduced pressure. The residue was purified by silica gel column chromatography (n-hexane:ethyl acetate=6:1) to give 334 mg (53%) of 6,8-difluoro-2-(3-fluoro-4-pivaloylaminophenyl)-5-(4-methylpentylamino)-4H-1-benzopyran-4-one.

NMR (90MHz, $CDCl_3$) δ(ppm) 0.90 (6H, d, J=6.2), 1.2-1.7 (5H, m), 1.34 (9H, s), 3.38-3.49 (2H, m), 6.54 (1H, s), 7.14 (1H, dd, J=14.4, 9.8), 7.5-7.8 (3H, m), 8.57 (1H, t, J=8.4), 8.8 (1H, brs)

MS (M/Z) 474 ($M^+$)

Molecular formula $C_{26}H_{29}F_3N_2O_3$=474

6 ml of dioxane and 6 ml of concentrated hydrochloric acid were added to 321 mg (0.68 mmol) of the resulting 6,8-difluoro-2-(3-fluoro-4-pivaloylaminophenyl)-5-(4-methylpentylamino)-4H-1-benzopyran-4-one and the mixture was heated at reflux for 1 hour. The reaction solution was cooled on ice, water was added to adjust the solution to neutral, and the precipitated crystals were filtered and purified by silica gel column chromatography (n-hexane:ethyl acetate=5:1) to give 230 mg (87%) of Compound 71.

NMR (270MHz, $CDCl_3$) δ(ppm) 0.90 (6H, d, J=6.9), 1.24-1.32 (2H, m), 1.55-1.66 (3H, m), 3.41-3.47 (2H, m), 4.17 (2H, brs), 6.46 (1H, s), 6.84 (1H, t, J=8.7), 7.12 (1H, dd, J=13.4, 10.4), 7.32-7.58 (2H, m), 8.80 (1H, brs)

MS (M/z) 390 ($M^+$)

Molecular formula $C_{21}H_{21}F_3N_2O_2$=390

Example 72

2-(4-Amino-3-fluorophenyl)-5-(3-aminopropylamino)-6,8-difluoro-4H-1-benzopyran-4-one (Compound 72)

814 mg (2.09 mmol) of 5-amino-6,8-difluoro-2-(3-fluoro-4-pivaloylaminophenyl)-4H-1-benzopyran-4-one obtained in Example 66 was dissolved in 15 ml of dimethylformamide under argon atmosphere, 256 mg of sodium hydride (60% oil suspension) and 0.42 ml of 1-bromo-3-chloropropane were added under ice-cooling and the mixture was stirred at room temperature for 3 hours. Water was added to the reaction solution and the mixture was extracted twice with ethyl acetate. The organic layer was washed twice with an aqueous saturated solution of sodium chloride, dried over anhydrous sodium sulfate and the solvent was distilled off under reduced pressure. The residue was purified by silica gel column chromatography (n-hexane:ethyl acetate=6:1) to give 193 mg (20%) of 5-(3-chloropropylamino)-6,8-difluoro-2-(3-fluoro-4-pivaloylaminophenyl)-4H-1-benzopyran-4-one.

NMR (90MHz, $CDCl_3$), δ(ppm) 1.36 (9H, s), 2.09 (2H, quint., J=6.3), 3.5-3.8 (2H, m), 3.65 (2H, t, J=6.4), 6.56 (1H, s), 7.17 (1H, dd, J=14.0, 10.3), 7.5-7.9 (3H, m), 8.58 (1H, t, J=8.2), 8.24 (1H, brs)

MS (M/Z) 466 ($M^+$)

Molecular formula $C_{23}H_{22}CF_3N_2O_3$=466

177 mg (0.38 mmol) of the resulting 5-(3-chloropropylamino)-6,8-difluoro-2-(3-fluoro-4-pivaloylaminophenyl)-4H-1-benzopyran-4-one was dissolved in 10 ml of dimethylformamide under argon atmosphere, 124 mg of sodium azide was added and the mixture was stirred at 70 °C for 18 hours. Water was added to the reaction solution, and the precipitated crystals were filtered and purified by silica gel column chromatography (n-hexane:ethyl acetate=4:1) to give 179 mg (100%) of 5-(3-azidopropylamino)-6,8-difluoro-2-(3-fluoro-4-pivaloylaminophenyl)-4H-1-benzopyran-4-one.

NMR (90MHz, $CDCl_3$) δ(ppm) 1.36 (9H, s), 1.91 (2H, quint., J=6.6), 3.4-3.7 (2H, m), 3.43 (2H, t, J=6.7), 6.56 (1H, s), 7.14 (1H, dd, J=13.4, 10.4), 7.5-7.9 (3H, m), 8.59 (1H, t, J=9.0), 8.80 (1H, brs)

MS (M/Z) 473 ($M^+$)

Molecular formula $C_{23}H_{22}ClF_3N_5O_3$=473

161 mg (0.34 mmol) of the resulting 5-(3-azidopropylamino)-6,8-difluoro-2-(3-fluoro-4-pivaloylaminophenyl)-4H-1-benzopyran-4-one was dissolved in 10 ml of dioxane and 10 ml of concentrated hydrochloric acid and the mixture was heated at reflux for 1 hour. The reaction solution was cooled on ice, water was added and the mixture was extracted twice with ethyl acetate. The organic layer was washed with an aqueous saturated solution of sodium chloride, dried over anhydrous sodium sulfate and the solvent was distilled off under reduced pressure to give 142 mg of crude product of 2-(4-amino-3-fluorophenyl)-5-(3-azidopropylamino)-6,8-difluoro-4H-1-benzopyran-4-one.

NMR (90MHz, $CDCl_3$) δ(ppm) 1.90 (2H, quint., J=6.7), 3.43 (2H, t, J=7.0), 3.5-3.8 (2H, m), 4.17 (2H, brs), 6.46 (1H, s), 6.83 (1H, t, J=8.4), 7.14 (1H, dd, J=13.4, 10.1), 7.4-7.7 (2H, m), 8.80 (1H, brs)

FAB-MS (M/Z) 390 ($M^+$+1)

Molecular formula $C_{18}H_{14}F_3N_5O_2$=389

114 mg (0.29 mmol) of the resulting crude 2-(4-amino-3-fluorophenyl)-5-(3-azidopropylamino)-6,8-difluoro-4H-1-benzopyran-4-one was dissolved in 5 ml of tetrahydrofuran, 117 mg of triphenylphosphine was added, the mixture was stirred at room temperature for 1 hour, 5 ml of water was added and the mixture was stirred for 2 hours. The mixture was extracted twice with ethyl acetate, the organic layer was washed with an aqueous saturated solution of sodium chloride and dried over anhydrous sodium sulfate. The solvent was distilled off under reduced pressure and the residue was purified by preparative thin layer chromatography (chloroform:methanol:aqueous ammonia=9:1:1) to give 82 mg (77%) of Compound 72.

NMR (270MHz, DMSO-$d_6$) δ(ppm) 1.62 (2H, quint., J=6.4), 2.62 (2H, t, J=6.9), 3.3-3.5 (2H, m), 6.11 (2H, brs), 6.71 (1H, s), 6.87 (1H, t, J=8.9), 7.5-7.8 (3H, m), 8.82 (1H, m)

MS (M/Z) 363 ($M^+$)

Molecular formula $C_{18}H_{16}F_3N_3O_2$=363

Example 73

5-(4-Aminobutylamino)-2-(4-amino-3-fluorophenyl)-6,8-difluoro-4H-1-benzopyran-4-one (Compound 73)

1.01 g (2.59 mmol) of 5-amino-6,8-difluoro-2-(3-fluoro-4-pivaloylaminophenyl)-4H-1-benzopyran-4-one obtained in Example 66 was dissolved in 20 ml of dimethylformamide under argon atmosphere, 228 mg of sodium hydride (60% oil suspension) and 1.5 ml of 1-bromo-4-chlorobutane were added under ice-cooling and the mixture was stirred at room temperature for 3 hours. 228 mg of sodium hydride (60% oil suspension) and 1.5 ml of 1-bromo-4-chlorobutane were further added and the mixture was stirred for additional 1 hour. Water was added to the reaction solution and the mixture was extracted three times with ethyl acetate. The organic layer was washed twice with an aqueous saturated solution of sodium chloride, dried over anhydrous sodium sulfate and the solvent was distilled off under reduced pressure. The residue was purified by silica gel column chromatography (n-hexane:ethyl acetate=6:1) to give 396 mg (32%) of 5-(4-chlorobutylamino)-6,8-difluoro-2-(3-fluoro-4-pivaloylaminophenyl)-4H-1-benzopyran-4-one.

NMR (90MHz, $CDCl_3$) δ(ppm) 1.35 (9H, s), 1.7-2.1 (4H, m), 3.4-3.7 (2H, m), 3.57 (2H, t, J=6.3), 6.55 (1H, s), 7.16 (1H, dd, J=13.3, 10.2), 7.5-7.9 (3H, m), 8.58 (1H, t, J=8.4), 8.80 (1H, brs)

FAB-MS (M/Z) 481 ($M^+$+1)

Molecular formula $C_{24}H_{24}ClF_3N_2O_3$=480

516 mg (1.07 mmol) of the resulting 5-(4-chlorobutylamino)-6,8-difluoro-2-(3-fluoro-4-pivaloylaninophenyl)-4H-1-benzopyran-4-one was dissolved in 30 ml of dimethylformamide under argon atmosphere, 350 mg of sodium azide was added and the mixture was stirred at 70 °C for 17 hours. Water was added to the reaction solution and the precipitated crystals were filtered.

457 mg (0.94 mmol) of the above product was dissolved in 40 ml of dioxane and 20 ml of concentrated hydrochloric acid and the mixture was heated at reflux for 7 hours. The reaction solution was cooled on ice and the precipitated crystals were filtered.

303 mg (0.76 mmol) of the above product was dissolved in 20 ml of tetrahydrofuran, 297 mg of triphenylphosphine was added under ice-cooling, the mixture was stirred at room temperature for 1 hour, 20 ml of water was added and

the mixture was stirred for 6 hours. The mixture was extracted twice with ethyl acetate, the organic layer was washed with an aqueous saturated solution of sodium chloride and dried over anhydrous sodium sulfate. The solvent was distilled off under reduced pressure and the residue was purified by column chromatography (chloroform:methanol: aqueous ammonia=9:1:1) to give 184 mg (65%) of Compound 73.

NMR (270MHz, DMSO-$d_6$) δ(ppm) 1.5-1.8 (4H, m), 2.7-2.9(2H, m), 3.3-3.5 (2H, m), 6.72 (1H, s), 6.88 (1H, t, J=8.9), 7.5-7.8 (3H, m)

MS (M/Z) 377 ($M^+$)

Molecular formula $C_{19}H_{18}F_3N_3O_2$=377

Example 74

6,8-Difluoro-2-(4-ethoxycarbonylmethylamino-3-fluorophenyl)-5-hexylamino-4H-1-benzopyran-4-one (Compound 74)

1.20 g (2.76 mmol) of 2-[4-(N-acetyl-N-ethoxycarbonylmethylamino)-3-fluorophenyl]-5-amino-6,8-difluoro-4H-1-benzopyran-4-one obtained in Example 44 was dissolved in 20 ml of dimethylformamide under argon atmosphere, 111 mg of sodium hydride (60% oil suspension) and 0.82 ml of 1-iodohexane were added under ice-cooling and the mixture was stirred at room temperature for 1.7 hours. 33 mg of sodium hydride was added and the mixture was stirred for additional 40 minutes. A 10% aqueous solution of citric acid was added to the reaction solution and the mixture was extracted with ethyl acetate. The organic layer was washed with an aqueous saturated solution of sodium chloride, dried over anhydrous sodium sulfate and the solvent was distilled off under reduced pressure. The residue was purified by silica gel column chromatography (chloroform:acetonitrile=10:1) to give 433 mg (28%) of 2-[4-(N-acetyl-N-ethoxycarbonylmethylamino)-3-fluorophenyl]-6,8-difluoro-5-hexylamino-4H-1-benzopyran-4-one.

NMR (90MHz, $CDCl_3$) δ(ppm) 0.8-1.0 (3H, m), 1.1-1.9 (8H, m), 1.28 (3H, t, J=7.0), 1.99 (3H, s), 3.3-3.6 (2H, m), 4.20 (2H, q, J=7.0), 4.37 (2H, brs), 6.63 (1H, s), 7.23 (1H, dd, J=13.2, 10.2), 7.5-7.9 (3H, m)

MS (M/Z) 518 ($M^+$)

Molecular formula $C_{27}H_{29}F_3N_2O_5$=518

422 mg (0.815 mmol) of the resulting 2-[4-(N-acetyl-N-ethoxycarbonylmethylamino)-3-fluorophenyl]-6,8-difluoro-5-hexylamino-4H-1-benzopyran-4-one was dissolved in 9 ml of ethanol, 6 ml of concentrated hydrochloric acid was added and the mixture was stirred at reflux for 2 hours. The reaction solution was cooled on ice, water was added and the mixture was extracted with ethyl acetate. The organic layer was washed with an aqueous saturated solution of sodium chloride, dried over anhydrous sodium sulfate and the solvent was distilled off under reduced pressure. The residue was purified by silica gel column chromatography (chloroform:acetone=40:1) and recrystallized from ethyl acetate/n-hexane to give 162 mg of Compound 74.

NMR (270MHz, DMSO-$d_6$) δ(ppm) 0.89 (3H, t, J=6.7), 1.2-1.5 (6H, m), 1.33 (3H, t, J=6.9), 1.66 (2H, quint., J=7.4), 3.45 (2H, td, J=7.2, 3.5), 4.01 (2H, s), 4.29 (2H, q, J=6.9), 6.48 (1H, s), 6.64 (1H, t, J=8.4), 7.16 (1H, dd, J=12.9, 9.9), 7.5-7.7 (2H, m)

MS (M/Z) 476 ($M^+$)

Molecular formula $C_{25}H_{27}F_3N_2O_4$=476

Example 75

2-(4-Carboxymethylamino-3-fluorophenyl)-6,8-difluoro-5-hexylamino-4H-1-benzopyran-4-one (Compound 75)

143 mg (0.276 mmol) of Compound 74 obtained in Example 74 was dissolved in 12 ml of dioxane, 8 ml of concentrated hydrochloric acid was added and the mixture was heated at reflux for 10 minutes. The reaction solution was cooled on ice, water was added and the mixture was extracted with ethyl acetate. The organic layer was washed twice with water and once with an aqueous saturated solution of sodium chloride, dried over anhydrous sodium sulfate and the solvent was distilled off under reduced pressure. The residue was recrystallized from ethyl acetate/n-hexane to give 95 mg (77%) of Compound 75.

NMR (270MHz, DMSO-$d_6$) δ(ppm) 0.89 (3H, t, J=6.7), 1.2-1.5 (6H, m), 1.61 (2H, quint., J=6.9), 3.42 (2H, td, J=6.9, 4.5), 3.98 (2H, s), 6.47 (1H, s), 6.68 (1H, t, J=8.7), 7.20 (1H, dd, J=13.4, 10.4), 7.54 (1H, dd, J=12.6, 2.0), 7.60 (1H, dd, J=8.4, 2.0)

FAB-MS (M/Z) 449 ($M^+$+1)

Molecular formula $C_{23}H_{23}F_3N_2O_4$=448

Example 76

6,8-Difluoro-2-(3-fluoro-4-methylaminophenyl)-5-(4-pentenylamino)-4H-1-benzopyran-4-one (Compound 76)

1.99 g (4.73 mmol) of Compound 51 obtained in Example 51 was dissolved in 80 ml of dimethylformamide under argon atmosphere, 212 mg of sodium hydride (60% oil suspension) and 0.60 ml of iodomethane were added and the mixture was stirred at room temperature for 1 hour. An aqueous saturated solution of ammonium chloride was added to the reaction solution, the precipitated crystals were filtered, the resulting crystals were dissolved in ethanol and the solvent was distilled off under reduced pressure. The residue was purified by silica gel column chromatography (chloroform) to give 2.09 g of 5-amino-2-[4-[N-(tert-butoxycarbonyl)-N-methylamino]-3-fluorophenyl)-6,8-difluoro-4H-1-benzopyran-4-one.

NMR (90MHz, $CDCl_3$) δ(ppm) 1.44 (9H, s), 3.25 (3H, d, J=0.7), 6.20 (2H, brs), 6.60 (1H, s), 7.21 (1H, t, J=10.6), 7.38 (1H, t, J=7.9), 7.58-7.72 (2H, m)

MS (M/Z) 420 ($M^+$)

Molecular formula $C_{21}H_{19}F_3N_2O_4$=420

852 mg (2.03 mmol) of the resulting 5-amino-2-[4-[N-(tert-butoxycarbonyl)-N-methylamino]-3-fluorophenyl)-6,8-difluoro-4H-1-benzopyran-4-one was dissolved in 30 ml of dimethylformamide under argon atmosphere, 165 mg of sodium hydride (60% oil suspension) and 0.48 ml of 5-bromo-1-pentene were added under ice-cooling and the mixture was stirred at room temperature for 2 hours. 85 mg of sodium hydride (60% oil suspension) and 0.24 ml of 5-bromo-1-pentene were added and the mixture was stirred for additional 1 hour. An aqueous saturated solution of ammonium chloride was added to the reaction solution and the mixture was extracted twice with ethyl acetate. The organic layer was washed with an aqueous saturated solution of sodium chloride, dried over anhydrous sodium sulfate and the solvent was distilled off under reduced pressure. The residue was purified by silica gel column chromatography (n-hexane:ethyl acetate=7:1) to give 239 mg (24%) of 2-[4-[N-(tert-butoxycarbonyl)-N-methylamino]-3-fluorophenyl]-6,8-difluoro-5-(4-pentenylamino)-4H-1-benzopyran-4-one.

NMR (90MHz, $CDCl_3$) δ(ppm) 1.44 (9H, s), 1.5-1.9 (2H, m), 2.0-2.4 (2H, m), 3.24 (3H, d, J=0.7), 3.49 (2H, m), 4.93-5.13 (2H, m), 5.6-6.0 (1H, m), 6.58 (1H, s), 7.16 (1H, dd, J=13.3, 10.2), 7.46 (1H, t, J=7.7), 7.59-7.71 (2H, m), 8.80 (1H, brs)

FAB-MS (M/Z) 489 ($M^+$+1)

Molecular formula $C_{26}H_{27}F_3N_2O_4$=488

212 mg (0.434 mmol) of the resulting 2-[4-[N-(tert-butoxycarbonyl)-N-methylamino]-3-fluorophenyl]-6,8-difluoro-5-(4-pentenylamino)-4H-1-benzopyran-4-one was dissolved in 5 ml of tetrahydrofuran, 5 ml of trifluoroacetic acid was added under ice-cooling and the mixture was stirred at room temperature for 17 hours. The solvent was distilled off under reduced pressure, water was added and the mixture was extracted with ethyl acetate. The organic layer was washed once with an aqueous saturated solution of sodium bicarbonate and once with an aqueous saturated solution of sodium chloride, dried over anhydrous sodium sulfate and the solvent was distilled off under reduced pressure. The residue was purified by silica gel column chromatography (chloroform:n-hexane=3:2) to give 143 mg (81%) of Compound 76.

NMR (270MHz, $CDCl_3$) δ(ppm) 1.73 (2H, m), 2.17 (2H, m), 2.97 (3H, d, J=5.4), 3.41-3.56 (2H, m), 4.45 (1H, brs), 4.97-5.10 (2H, m), 5.82 (1H, ddt, J=17.1, 10.1, 6.7), 6.45 (1H, s), 6.72 (1H, t, J=8.7), 7.11 (1H, dd, J=13.4, 10.4), 7.52 (1H, dd, J=12.4, 2.0), 7.62 (1H, dd, J=8.7, 2.0), 8.85 (1H, brs)

MS (M/Z) 388 ($M^+$)

Molecular formula $C_{21}H_{19}F_3N_2O_2$=388

Example 77

6,8-Difluoro-2-(3-fluoro-4-methylaminophenyl)-5-(5-hexenylamino)-4H-1-benzopyran-4-one (Compound 77)

828 mg (1.97 mmol) of 5-amino-2-[4-[N-(tert-butoxycarbonyl)-N-methylamino]-3-fluorophenyl]-6,8-difluoro-4H-1-benzopyran-4-one obtained in Example 76 was dissolved in 40 ml of dimethylformamide under argon atmosphere, 160 mg of sodium hydride (60% oil suspension) and 0.53 ml of 6-bromo-1-hexene were added under ice-cooling and the mixture was stirred at room temperature for 2 hours. Additional 80 mg of sodium hydride (60% oil suspension) and 0.27 ml of 6-bromo-1-hexene were added and the mixture was stirred for additional 1.5 hours. An aqueous saturated solution of ammonium chloride was added to the reaction solution and the mixture was extracted three times with ethyl acetate. The organic layer was washed with an aqueous saturated solution of sodium chloride, dried over sodium sulfate and the solvent was distilled off under reduced pressure. The residue was purified by silica gel column chromatography (n-hexane:ethyl acetate=8:1) to give 315 mg (32%) of 2-[4-[N-(tert-butoxycarbonyl)-N-methylamino]-3-fluorophenyl]-6,8-difluoro-5-(5-hexenylamino)-4H-1-benzopyran-4-one.

NMR (270MHz, $CDCl_3$) δ(ppm) 1.44 (9H, s), 1.40-1.71 (4H, m), 2.10 (2H, bq, J=7.1), 3.25 (3H, s), 3.48 (2H, m), 4.93-5.06 (2H, m), 5.81 (1H, ddt, J=17.2, 10.2, 6.6), 6.56 (1H, s), 7.16 (1H, dd, J=13.5, 10.2), 7.38 (1H, brt, J=8.1), 7.62-7.68 (2H, m), 8.80 (1H, brs)

MS (M/Z) 502 ($M^+$)

Molecular formula $C_{27}H_{29}F_3N_2O_4$=502

295 mg (0.605 mmol) of the resulting 2-[4-[N-(tert-butoxycarbonyl)-N-methylamino]-3-fluorophenyl]-6,8-difluoro-5-(5-hexenylamino)-4H-1-benzopyran-4-one was dissolved in 6 ml of tetrahydrofuran, 5 ml of trifluoroacetic acid was added under ice-cooling and the mixture was stirred at room temperature for 12 hours. The solvent was distilled off reduced pressure, water was added and the mixture was extracted with ethyl acetate. The organic layer was washed once with an aqueous saturated solution of sodium bicarbonate and once with an aqueous saturated solution of sodium chloride, dried over anhydrous sodium sulfate and the solvent was distilled off under reduced pressure. The residue was purified by silica gel column chromatography (chloroform:n-hexane=3:2) to give 226 mg (96%) of Compound 77.

NMR (270MHz, $CDCl_3$) δ(ppm) 1.42-1.70 (6H, m), 2.10 (2H, q, J=6.9), 2.97 (3H, d, J=5.4), 3.40-3.49 (2H, m), 4.45 (1H, brs), 4.92-5.05 (2H, m), 5.81 (1H, ddt, J=17.1, 10.1, 6.7), 6.44 (1H, s), 6.72 (1H, t, J=8.7), 7.11 (1H, dd, J=13.4, 10.4), 7.52 (1H, dd, J=12.4, 2.0), 7.62 (1H, dd, J=8.7, 2.0), 8.83 (1H, brs)

MS (M/Z) 402 ($M^+$)

Molecular formula $C_{22}H_{21}F_3N_2O_2$=402

Example 78

6,8-Difluoro-2-(3-fluoro-4-methylaminophenyl)-5-(3-methoxypropylamino)-4H-1-benzopyran-4-one (Compound 78)

1.03 g (2.64 mmol) of 5-amino-6,8-difluoro-2-(3-fluoro-4-pivaloylaminophenyl)-4H-1-benzopyran-4-one obtained in Example 66 was dissolved in 20 ml of dimethylformamide, 310 mg of sodium hydride (60% oil suspension) and 1.04 g of 3-(tetrahydropyran-2-yloxy)-1-iodopropane were added under ice-cooling and the mixture was stirred for 2 hours. Water was added to the reaction solution and the mixture was extracted twice with ethyl acetate. The organic layer was washed once with water and once with an aqueous saturated solution of sodium chloride, dried over anhydrous magnesium sulfate and the solvent was distilled off under reduced pressure. The residue was purified by silica gel column chromatography (n-hexane:ethyl acetate=5:1) to give 1.11 g of a mixture of the desired 6,8-difluoro-2-(3-fluoro-4-pivaloylaminophenyl)-5-[3-(tetrahydropyran-2-yloxy)propylamino]-4H-1-benzopyran-4-one and the raw material.

The above mixture was dissolved in a mixed solvent of 20 ml of methanol and 10 ml of tetrahydrofuran, 79 mg of p-toluenesulfonic acid was added and the mixture was stirred at room temperature for 24 hours. Water was added to the reaction solution, the precipitated crystals were filtered and purified by silica gel column chromatography (n-hexane: ethyl acetate=3:1) to give 502 mg (two stages 42%) of 6,8-difluoro-2-(3-fluoro-4-pivaloylaminophenyl)-5-(3-hydroxypropylamino)-4H-1-benzopyran-4-one.

NMR (90MHz, $CDCl_3$) δ(ppm) 1.36 (9H, s), 1.90 (2H, quint., J=6.3), 3.4-3.8 (2H, m), 3.80 (2H, t, J=6.2), 6.55 (1H, s), 7.15 (1H, dd, J=13.4, 10.3), 7.57-7.81 (3H, m), 8.58 (1H, t, J=8.2), 8.80 (1H, brs)

FAB-MS (M/Z) 449 ($M^+$+1)

Molecular formula $C_{23}H_{23}F_3N_2O_4$=448

483 mg (1.08 mmol) of the resulting 6,8-difluoro-2-(3-fluoro-4-pivaloylaminophenyl)-5-(3-hydroxypropylamino)-4H-1-benzopyran-4-one was dissolved in 10 ml of dimethylformamide under argon atmosphere, 0.34 ml of iodomethane and 140 mg of sodium hydride (60% oil suspension) were added under ice-cooling and the mixture was stirred at room temperature for 2 hours. Water was added to the reaction solution and the mixture was extracted twice with ethyl acetate. The organic layer was washed twice with an aqueous saturated solution of sodium chloride, dried over anhydrous sodium sulfate and the solvent was distilled off under reduced pressure. The residue was purified by silica gel column chromatography (n-hexane:ethyl acetate=8:1) to give 284 mg (55%) of 6,8-difluoro-2-[3-fluoro-4-(N-methyl-N-pivaloylaminolphenyl]-5-(3-methoxypropylamino)-4H-1-benzopyran-4-one.

NMR (90MHz, $CDCl_3$) δ(ppm) 1.12 (9H, s), 1.90 (2H, m), 3.23 (3H, s), 3.35 (3H, s), 3.4-3.8 (2H, m), 3.50 (2H, t, J=6.2), 6.61 (1H, s), 7.18 (1H, dd, J=13.4, 10.3), 7.41 (1H, t, J=8.0), 7.64-7.76 (3H, m), 8.80 (1H, brs)

MS (M/Z) 476 ($M^+$)

Molecular formula $C_{25}H_{27}F_3N_2O_4$=476

10 ml of dioxane and 10 ml of concentrated hydrochloric acid were added to 270 mg (0.567 mmol) of the resulting 6,8-difluoro-2-[3-fluoro-4-(N-methyl-N-pivaloylamino)phenyl]-5-(3-methoxypropylamino)-4H-1-benzopyran-4-one and the mixture was heated at reflux for 30 minutes. The reaction solution was cooled on ice, water was added to adjust the solution to neutral, and the precipitated crystals were filtered and purified by silica gel column chromatography (n-hexane:ethyl acetate=3:1) to give 190 mg (85%) of Compound 78.

NMR (270MHz, $CDCl_3$) δ(ppm) 1.89 (2H, quint., J=6.3), 2.97 (3H, d, J=5.4), 3.35 (3H, s), 3.50 (2H, t, J=6.2), 3.54 (2H, m), 4.45 (1H, brs), 6.45 (1H, s), 6.72 (1H, t, J=8.7), 7.12 (1H, dd, J=13.4, 10.4), 7.52 (1H, dd, J=12.9, 2.0), 7.62

(1H, dd, J=8.4, 2.0)

MS (M/Z) 392 ($M^+$)

Molecular formula $C_{20}H_{19}F_3N_2O_3$=392

Example 79

2-[4-(3-Aminopropylamino)-3-fluorophenyl]-6,8-difluoro-5-pentylamino-4H-1-benzopyran-4-one (Compound 79)

810 mg (1.91 mmol) of 2-[4-[N-acetyl-N-(3-chloropropyl)amino]-3-fluorophenyl]-5-amino-6,8-difluoro-4H-1-benzopyran-4-one obtained in Example 50 was dissolved in 40 ml of dimethylformamide under argon atmosphere, 622 mg of sodium azide was added at room temperature and the mixture was stirred at 70 °C for 4 hours. Water was added to the reaction solution, the mixture was extracted with twice with ethyl acetate, the organic layer was washed once with water and once with an aqueous saturated solution of sodium chloride, dried over anhydrous sodium sulfate and the solvent was distilled off under reduced pressure to give 870 mg of 2-[4-[N-acetyl-N-(3-azidopropyl)amino]-3-fluorophenyl]-5-amino-6,8-difluoro-4H-1-benzopyran-4-one.

NMR (90MHz, $CDCl_3$) δ(ppm) 1.82 (2H, m), 1.91 (3H, s), 3.37 (2H, t, J=6.7), 3.8 (2H, m), 6.22 (2H, brs), 6.64 (1H, s), 7.23 (1H, t, J=10.4), 7.40 (1H, t, J=8.0), 7.72-7.84 (2H, m)

MS (M/Z) 431 ($M^+$)

Molecular formula $C_{20}H_{16}F_3N_5O_3$=431

751 mg (1.74 mmol) of the resulting 2-[4-[N-acetyl-N-(3-azidopropyl)amino]-3-fluorophenyl]-5-amino-6,8-difluoro-4H-1-benzopyran-4-one was dissolved in 25 ml of dimethylformamide under argon atmosphere, 141 mg of sodium hydride (60% oil suspension) and 0.45 ml of 1-iodopentane were added under ice-cooling and the mixture was stirred at room temperature for 1 hour. Water was added to the reaction solution, the mixture was extracted twice with ethyl acetate, a 10% aqueous solution of citric acid was added to the aqueous layer and the mixture was further extracted with ethyl acetate. The organic layer was washed once with an aqueous saturated solution of sodium chloride, dried over anhydrous sodium sulfate and the solvent was distilled off under reduced pressure. The residue was purified by silica gel column chromatography (n-hexane:ethyl acetate=3:1) to give 659 mg (75%) of 2-[4-[N-acetyl-N-(3-azidopropyl)amino]-3-fluorophenyl]-6,8-difluoro-5-pentylamino-4H-1-benzopyran-4-one.

NMR (90MHz, $CDCl_3$) δ(ppm) 0.8-1.0 (3H, m), 1.2-2.0 (8H, m), 1.90 (3H, s), 3.36 (2H, t, J=6.8), 3.3-3.6 (2H, m), 3.6-4.0 (2H, m), 6.60 (1H, s), 7.17 (1H, dd, J=13.3, 10.2), 7.39 (1H, t, J=7.7), 7.70-7.81 (2H, m)

MS (M/Z) 501 ($M^+$)

Molecular formula $C_{25}H_{26}F_3N_5O_3$=501

51 mg (0.10 mmol) of the resulting 2-[4-[N-acetyl-N-(3-azidopropyl)amino]-3-fluorophenyl]-6,8-difluoro-5-pentylamino-4H-1-benzopyran-4-one was dissolved in a mixed solvent of 1.5 ml of dioxane and a mixed solvent of 1.5 ml of concentrated hydrochloric acid and the mixture was heated at reflux for 3 hours. The reaction solution was cooled, water was added to adjust the solution to weak alkaline and the mixture was extracted twice with ethyl acetate. The organic layer was washed once with water and once with an aqueous saturated solution of sodium chloride, dried over anhydrous sodium sulfate and the solvent was distilled off under reduced pressure. The residue was purified by preparative thin layer chromatography (chloroform) to give 26 mg (56%) of 2-[4-(3-azidopropylamino)-3-fluorophenyl]-6,8-difluoro-5-pentylamino-4H-1-benzopyran-4-one.

NMR (90MHz, $CDCl_3$), δ(ppm) 0.8-1.0 (3H, m), 1.2-1.8 (6H, m), 1.94 (2H, quint., J=6.6), 3.41 (2H, t, J=6.2), 3.48 (2H, t, J=6.6), 4.45 (1H, brs), 6.44 (1H, s), 6.75 (1H, t, J=8.7), 7.11 (1H, dd, J=13.4, 10.3), 7.45-7.67 (2H, m), 8.80 (1H, brs)

MS (M/Z) 459 ($M^+$)

Molecular formula $C_{23}H_{24}F_3N_5O_2$=459

330 mg (0.72 mmol) of the resulting 2-[4-(3-azidopropylamino)-3-fluorophenyl]-6,8-difluoro-5-pentylamino-4H-1-benzopyran-4-one was dissolved in 20 ml of tetrahydrofuran, 284 mg of triphenylphosphine was added under ice-cooling, the reaction was warmed to room temperature, 10 ml of water was added and the mixture was stirred overnight. The solvent was distilled off under reduced pressure and the residue extracted with ethyl acetate. The organic layer was washed with an aqueous saturated solution of sodium chloride, dried over anhydrous sodium sulfate and the solvent was distilled off under reduced pressure. The residue was purified by silica gel column chromatography (chlorofom:methanol=50:1-chloroform:methanol:aqueous ammonia=50:1:1) to give 278 mg (89%) of Compound 79.

NMR (270MHz, $CDCl_3$) δ(ppm) 0.91 (3H, t, J=6.9), 1.25-1.75 (6H, m), 1.83 (2H, quint., J=6.4), 2.91 (2H, t, J=6.4), 3.30-3.37 (2H, m), 3.39-3.48 (2H, m), 5.10 (1H, brs), 6.43 (1H, s), 6.73 (1H, t, J=8.4), 7.11 (1H, dd, J=13.4, 10.4), 7.51 (1H, dd, J=12.4, 2.0), 7.59 (1H, dd, J=8.4, 1.5), 8.82 (1H, brs)

MS (M/Z) 433 ($M^+$)

Molecular formula $C_{23}H_{26}F_3N_3O_2$=433

Reference Example 1

Ethyl 2-(N-ethoxycarbonyl-N-pivaloylamino)-3-fluoro-6-methoxymethoxybenzoate (Compound A)

200 g of ethyl 6-(N-ethoxycarbonyl-N-pivaloylamino)-2-(2-tetrahydropyranyloxy)benzoate obtained according to the known method (USP 4,571,405) was dissolved in 900 ml of ethanol, 300 ml of concentrated hydrochloric acid was added and the mixture was heated at reflux for 3.5 hours. The reaction solution was cooled on ice, 500 ml of water was added and the precipitated crystals were filtered to give 89.1 g (74%) of ethyl 6-(N-ethoxycarbonylamino)-2-hydroxybenzoate (Compound D).

NMR (90MHz, $CDCl_3$) δ(ppm) 1.31 (3H, t, J=7.0), 1.50 (3H, t, J=7.0), 4.22 (2H, q, J=7.0), 4.53 (2H, q, J=7.0), 6.65 (1H, dd, J=8.2, 1.2), 7.37 (1H, t, J=8.4), 7.86 (1H, dd, J=8.4, 1.1), 9.48 (1H, brs), 10.74 (1H, s)

MS (M/Z) 253 ($M^+$)

Molecular formula $C_{12}H_{15}NO_5$=253

12.7 g of the above Compound D was dissolved in 100 ml of dichloroethane, 17.4 g of N-fluoro-3,5-pyridinium triflate [Onoda Florinate FP-T700 (Wakojunyaku)] was added and the mixture was stirred at 60-70 °C for two hours. Additional 4.74 g of FP-T700 was added and the mixture was stirred at the same temperature for 2.5 hours. 1N hydrochloric acid was added to the reaction solution to adjust to acidic, the mixture was extracted with ether, the organic layer was washed with water and an aqueous saturated solution of sodium chloride, dried over anhydrous sodium sulfate and the solvent was distilled off under reduced pressure. The residue was subjected to silica gel column chromatography (n-hexane:ethyl acetate=8:1-4:1) to give 5.08 g (37%) of ethyl 2-(N-ethoxycarbonylamino)-3-fluoro-6-hydroxybenzoate.

NMR (90MHz, $CDCl_3$) δ(ppm) 1.30 (3H, t, J=7.1), 1.42 (3H, t, J=7.1), 4.21 (2H, q, J=7.0), 4.44 (2H, q, J=7.1), 6.80 (1H, dd, J=9.2, 4.4), 7.05 (1H, brs), 7.20 (1H, t, J=9.3), 10.4 (1H, s)

MS (M/Z) 271 ($M^+$)

Molecular formula $C_{12}H_{14}FNO_5$=271

26.6 g of the resulting ethyl 2-(N-ethoxycarbonylamino)-3-fluoro-6-hydroxybenzoate was dissolved in 300 ml of dichloromethane under argon atmosphere, 20.5 ml of diisopropylethylamine and 9.0 ml of chloromethyl methyl ether were added under ice-cooling and the mixture was stirred at room temperature for 1.5 hours. Additional 20.5 ml of diisopropylethylamine and 9.0 ml of chloromethyl methyl ether were added and the mixture was stirred for 1.6 hours. Additional 5.1 ml of diisopropylethylamine and 2.2 ml of chloromethyl methyl ether were added and the mixture was stirred for 1 hour. An aqueous dilute hydrochloric acid solution was added to the reaction solution, the mixture was extracted with dichloromethane, the organic layer was washed with water and an aqueous saturated solution of sodium chloride, dried over anhydrous sodium sulfate and the solvent was distilled off under reduced pressure to give ethyl 2-(N-ethoxycarbonylamino)-3-fluoro-6-methoxymethoxybenzoate.

NMR (90MHz, $CDCl_3$) δ(ppm) 1.28 (3H, t, J=7.2), 1.37 (3H, t, J=7.2), 3.48 (3H, s), 4.20 (2H, q, J=7.2), 4.38 (2H, q, J=7.1), 5.13 (2H, s), 6.71 (1H, brs), 6.97 (1H, dd, J=9.0, 4.6), 7.13 (1H, t, J=9.0)

MS (M/Z) 315 ($M^+$)

Molecular formula $C_{14}H_{18}FNO_6$=315

The resulting ethyl 2-(N-ethoxycarbonylamino)-3-fluoro-6-methoxymethoxybenzoate was dissolved in 200 ml of tetrahydrofuran under ice-cooling, 3.92 g of sodium hydride (60% oil suspension) and 8.39 ml of pivaloyl chloride were added and the mixture was stirred at 0 °C for 45 minutes. Additional 785 mg of sodium hydride (60% oil suspension) and 1.68 ml of pivaloyl chloride were added and the mixture was stirred at 0 °C for 40 minutes. An aqueous saturated solution of ammonium chloride was added to the reaction solution, the mixture was extracted with ether, the organic layer was washed with an aqueous saturated solution of sodium chloride, dried over sodium sulfate and the solvent was distilled off under reduced pressure. The residue was subjected to silica gel column chromatography (n-hexane: ethyl acetate=4:1-3:1) to give 32.2 g (two stages 82%) of Compound A.

NMR (90MHz, $CDCl_3$) δ(ppm) 1.20 (3H, t, J=7.0), 1.32 (3H, t, J=7.2), 1.39 (9H, s), 3.48 (3H, s), 4.19 (2H, q, J=6.8), 4.33 (2H, q, J=7.2), 5.14 (2H, s), 6.9-7.3 (2H, m)

MS (M/Z) 399 ($M^+$)

Molecular formula $C_{19}H_{26}FNO_7$=399

Reference Example 2

Ethyl 6-(N-ethoxycarbonyl-N-pivaloylamino)-3-fluoro-2-methoxymethoxybenzoate (Compound B)

12.7 g of Compound D obtained in Reference Example 1 was dissolved in 100 ml of dichloroethane, 17.4 g of Onoda Florinate FP-T700 (Wakojunyaku) was added and the mixture was stirred at 60-70 °C for 2 hours. Additional 4.74 g of FP-T700 was added and the mixture was stirred at the same temperature for 2.5 hours. 1N hydrochloric acid

was added to adjust the solution to acidic, the mixture was extracted with ether, the organic layer was washed with water and an aqueous saturated solution of sodium chloride, dried over sodium sulfate and the solvent was distilled off under reduced pressure. The residue was subjected to silica gel column chromatography (n-hexane:ethyl acetate=8:1-4:1) to give 3.26 g (24%) of ethyl 6-(N-ethoxycarbonylamino)-3-fluoro-2-hydroxybenzoate.

NMR (90MHz, $CDCl_3$) δ(ppm) 1.31 (3H, t, J=7.1), 1.51 (3H, t, J=7.1), 4.22 (2H, q, J=7.1), 4.56 (2H, q, J=7.2), 7.22 (1H, t, J=9.7), 7.79 (1H, dd, J=9.2, 4.4), 9.22 (1H, brs)

MS (M/Z) 271 ($M^+$)

Molecular formula $C_{12}H_{14}FNO_5$=271

11.2 g of the resulting ethyl 6-(N-ethoxycarbonylamino)-3-fluoro-2-hydroxybenzoate was dissolved in 100 ml of dichloromethane under argon atmosphere, 14.4 ml of diisopropylethylamine and 6.3 ml of chloromethyl methyl ether were added under ice-cooling and the mixture was stirred at room temperature for 40 minutes. An aqueous dilute hydrochloric acid solution was added to the reaction solution, the mixture was extracted with dichloromethane, the organic layer was washed with water and an aqueous saturated solution of sodium chloride, dried over anhydrous sodium sulfate and the solvent was distilled off under reduced pressure to give ethyl 6-(N-ethoxycarbonylamino)-3-fluoro-2-methoxymethoxybenzoate.

NMR (90MHz, $CDCl_3$) δ(ppm) 1.30 (3H, t, J=7.1), 1.41 (3H, t, J=7.1), 3.55 (3H, s), 4.20 (2H, q, J=7.0), 4.43 (2H, q, J=7.0), 5.12 (2H, d, J=0.7), 7.18 (1H, t, J=9.8), 7.89 (1H, dd, J=9.3, 4.3), 8.45 (1H, brs)

MS (M/Z) 315 ($M^+$)

Molecular formula $C_{14}H_{18}FNO_6$=315

The resulting ethyl 6-(N-ethoxycarbonylamino)-3-fluoro-2-methoxymethoxybenzoate was dissolved in 80 ml of tetrahydrofuran under ice-cooling, 1.98 g of sodium hydride (60% oil suspension) and 4.25 ml of pivaloyl chloride were added and the mixture was stirred at 0 °C for 3.8 hours. Additional 1.65 g of sodium hydride (60% oil suspension) and 3.54 ml of pivaloyl chloride were added and the mixture was stirred at 0 °C for 35 minutes. An aqueous saturated solution of ammonium chloride was added to the reaction solution, the mixture was extracted with ether, the organic layer was washed with an aqueous saturated solution of sodium chloride, dried over anhydrous sodium sulfate and the solvent was distilled off under reduced pressure. The residue was subjected to silica gel column chromatography (n-hexane:ethyl acetate=4:1) to give 15.7 g (two stages 95%) of Compound B.

NMR (90MHz, $CDCl_3$) δ(ppm) 1.19 (3H, t, J=7.0), 1.35 (3H, t, J=7.0), 1.35 (9H, s), 3.55 (3H, s), 4.17 (2H, q, J=7.1), 4.32 (2H, q, J=7.2), 5.17 (2H, s), 6.85 (1H, dd, J=8.8, 4.4), 7.16 (1H, dd, J=10.3, 9.0)

MS (M/Z) 399 ($M^+$)

Molecular formula $C_{19}H_{26}FNO_7$=399

Reference Example 3

Ethyl 3,5-difluoro-6-(N-ethoxycarbonyl-N-pivaloylamino)-2-methoxymethoxybenzoate (Compound C)

2.0 g of Compound D obtained in Reference Example 1 was dissolved in 30 ml of dichloroethane, 5.0 g of Onoda Florinate FP-T700 (Wakojunyaku) was added and the mixture was heated at reflux for 3.7 hours. Additional 1.25 g of FP-T700 was added and the mixture was heated at reflux for 50 minutes. Additional 1.25 g of FP-T700 was added and the mixture was heated at reflux for 1 hour. 1N hydrochloric acid was added to the reaction solution to adjust to acidic, the mixture was extracted with ether, the organic layer was washed with water and an aqueous saturated solution of sodium chloride, dried over anhydrous sodium sulfate and the solvent was distilled off under reduced pressure. The residue was subjected to silica gel column chromatography (n-hexane:ethyl acetate=9:1-3:1) to give 690 mg (30%) of ethyl 3,5-difluoro-6-(N-ethoxycarbonylamino)-2-hydroxybenzoate.

NMR (90MHz, $CDCl_3$) δ(ppm) 1.29 (3H, t, J=7.0), 1.43 (3H, t, J=7.1), 4.21 (2H, q, J=7.2), 4.47 (2H, q, J=7.1), 6.83 (1H, brs), 7.13 (1H, t, J=9.9), 10.54 (1H, s)

MS (M/Z) 289 ($M^+$)

Molecular formula $C_{12}H_{13}F_2NO_5$=289

5.72 g of the resulting ethyl 3,5-difluoro-6-(N-ethoxycarbonylamino)-2-hydroxybenzoate was dissolved in 70 ml of dichloromethane under argon atmosphere, 4.13 ml of diisopropylethylamine and 1.80 ml of chloromethyl methyl ether were added under ice-cooling and the mixture was stirred at 0 °C for 20 minutes. An aqueous dilute hydrochloric acid solution was added to the reaction solution, the mixture was extracted with ether, the organic layer was washed with water and an aqueous saturated solution of sodium chloride, dried over anhydrous sodium sulfate and the solvent was distilled off under reduced pressure to give ethyl 3,5-difluoro-6-(N-ethoxycarbonylamino)-2-methoxymethoxybenzoate.

NMR (90MHz, $CDCl_3$) δ(ppm) 1.27 (3H, t, J=7.1), 1.38 (3H, t, J=7.3), 3.55 (3H, s), 4.19 (2H, q, J=7.1), 4.39 (2H, q, J=7.1), 5.11 (2H, s), 6.56 (1H, brs), 7.01 (1H, t, J=10.0)

MS (M/Z) 333 ($M^+$)

Molecular formula $C_{14}H_{17}F_2NO_6$=333

The resulting ethyl 3,5-difluoro-6-(N-ethoxycarbonylamino)-2-methoxymethoxybenzoate was dissolved in 35 ml of tetrahydrofuran under ice-cooling, 792 mg of sodium hydride (60% oil suspension) and 1.69 ml of pivaloyl chloride were added and the mixture was stirred at 0 °C for 20 minutes. Additional 396 mg of sodium hydride (60% oil suspension) and 0.85 ml of pivaloyl chloride were added and the mixture was stirred at 0 °C for 20 minutes. Additional 158 mg of sodium hydride (60% oil suspension) and 0.34 ml of pivaloyl chloride were added and the mixture was stirred at 0 °C for 1.2 hours. An aqueous saturated solution of ammonium chloride was added to the reaction solution, the mixture was extracted with ether, the organic layer was washed with an aqueous saturated solution of sodium chloride, dried over anhydrous sodium sulfate and the solvent was distilled off under reduced pressure to give 6.63 g (two stages 80%) of Compound C.

NMR (90MHz, $CDCl_3$) δ(ppm) 1.20 (3H, t, J=7.0), 1.33 (3H, t, J=7.0), 1.39 (9H, s), 3.55 (3H, s), 4.18 (2H, q, J=7.0), 4.33 (2H, q, J=7.0), 5.12 (2H, s), 7.00 (1H, dd, J=10.2, 9.1)

MS (M/Z) 417 ($M^+$)

Molecular formula $C_{19}H_{25}F_2NO_7$=417

Reference Example 4

Ethyl 3,5-difluoro-6-pivaloylamino-2-(2-tetrahydropyranyloxy)benzoate (Compound E)

104 g (796 mmol) of 2,4-difluorophenol was dissolved in 800 ml of dichloromethane, 132 ml of triethylamine and 92.0 ml of ethyl chloroformate were added under ice-cooling and the mixture was stirred at -10-0 °C for 2 hours. The reaction solution was washed with an aqueous saturated solution of sodium chloride, dried over anhydrous magnesium sulfate and the solvent was distilled off under reduced pressure to give 156 g (97%) of 2,4-difluoro-O-ethoxycarbonylphenol.

NMR (90MHz, $CDCl_3$) δ(ppm) 1.39 (3H, t, J=7.0), 4.33 (2H, q, J=7.0), 6.7-7.3 (3H, m)

MS (M/Z) 202 ($M^+$)

Molecular formula $C_9H_8F_2O_3$=202

50.5 g (250 mmol) of the resulting 2,4-difluoro-O-ethoxycarbonylphenol was dissolved in 115 ml of concentrated sulfuric acid, 15.9 ml of fuming nitric acid was added while maintaining the internal temperature at from 10 to 20 °C and the mixture was stirred at the same temperature for 1 hour. The reaction solution was poured into ice water and the mixture was extracted with 500 ml of ethyl acetate. The organic layer was washed twice with an aqueous saturated solution of sodium chloride, dried over anhydrous sodium sulfate and the solvent was distilled off under reduced pressure. The residue was dissolved in 1.0 liter of methanol, 50 ml of water and 40 g of sodium bicarbonate were added and the mixture was stirred at room temperature for 16 hours. The reaction solution was filtered, methanol was distilled off under reduced pressure, 200 ml of water was added to adjust to pH 5 and the mixture was extracted twice with 200 ml of ethyl acetate. The organic layer was washed once with 400 ml of water and once with 400 ml of an aqueous saturated solution was sodium chloride and dried over anhydrous sodium sulfate. The solvent was distilled off under reduced pressure to give 41.6 g (95%) of 2,4-difluoro-5-nitrophenol.

NMR (90MHz, $CDCl_3$) δ(ppm) 7.23 (1H, t, J=9.9), 7.76 (1H, dd, J=8.6, 7.3)

MS (M/Z) 175 ($M^+$)

Molecular formula $C_6H_3F_2NO_3$=175

24.9 g (142 mmol) of the resulting 2,4-difluoro-5-nitrophenol was dissolved in 150 ml of ethyl acetate, 5.0 g of 10% palladium-carbon was added and the mixture was stirred at 50-60 °C for 27 hours under a stream of hydrogen. The reaction vessel was filled with nitrogen, the reaction solution was filtered by means of suction, the solvent was distilled off under reduced pressure, and the residue was triturated with n-hexane to give 19.8 g (96%) of 5-amino-2,4-difluorophenol.

NMR (90MHz, $CDCl_3$) δ(ppm) 4.75 (2H, brs), 6.37 (1H, t, J=9.1), 6.87 (1H, t, J=11.1), 9.21 (1H, s)

MS (M/Z) 145 ($M^+$)

Molecular formula $C_6H_5F_2NO$=145

18.9 g (130 mmol) of the resulting 5-amino-2,4-difluorophenol was dissolved in 45 ml of pyridine, 16.0 ml of pivaloyl chloride was added dropwise over 8 minutes under ice-cooling, and the mixture was stirred at the same temperature for 30 minutes. 1N hydrochloric acid was added to the reaction solution and the mixture was extracted with ether. The organic layer was washed once with a 1N aqueous hydrochloric acid solution, water and an aqueous saturated solution of sodium chloride, respectively, and dried over magnesium sulfate. The solvent was distilled off under reduced pressure and the residue was triturated with n-hexane to give 27.0 g (91%) of 2,4-difluoro-5-pivaloylaminophenol.

NMR (90MHz, $CDCl_3$) δ(ppm) 1.35 (9H, s), 6.90 (1H, t, J=10.4), 7.65 (1H, brs), 7.94 (1H, brs), 8.24 (1H, dd, J=9.1, 8.0)

MS (M/Z) 229 ($M^+$)

Molecular formula $C_{11}H_{13}F_2NO_2$=229

2.15 g (9.39 mmol) of the resulting 2,4-difluoro-5-pivaloylaminophenol was dissolved in 40 ml of dichloromethane, 4.3 ml of 3,4-dihydro-2H-pyrane and 44 mg of camphorsulfonic acid were added and the mixture was stirred at room temperature for 4.3 hours. The reaction solution was added to a 5% aqueous solution of potassium carbonate and the mixture was extracted with chloroform. The organic layer was washed once with water and once with an aqueous saturated solution of sodium chloride and dried over anhydrous magnesium sulfate. The solvent was distilled off under reduced pressure and the residue was triturated with n-hexane to give 2.51 g (85%) of 2,4-difluoro-5-pivaloylamino-O-(2-tetrahydropyranyl)phenol.

NMR (90MHz, $CDCl_3$) δ(ppm) 1.31 (9H, s), 1.4-2.2 (6H, m), 3.4-4.2 (2H, m), 5.43 (1H, brs), 6.89 (1H, t, J=10.4), 7.44 (1H, brs), 8.25 (1H, t, J=8.5)

MS (M/Z) 313 ($M^+$)

Molecular formula $C_{16}H_{21}F_2NO_3$=313

31.3 g (100 mmol) of the resulting 2,4-difluoro-5-pivaloylamino-O-(2-tetrahydropyranyl)phenol was dissolved in 300 ml of tetrahydrofuran under argon atmosphere, 40 ml of hexamethylphosphoric triamide was added and the mixture was cooled to -78 °C. 140 ml of n-butyl lithium (1.6M solution in n-hexane) was added to the reaction solution at an internal temperature of not higher than -60 °C and the mixture was stirred at the same temperature for 1 hour. 19 ml of ethyl chloroformate was added, the mixture was stirred while rising the temperature gradually. After 2.2 hours at which the internal temperature became -15 °C, water was added and the mixture was extracted with ethyl acetate. The organic layer was washed once with water and once with an aqueous saturated solution of sodium chloride, dried over anhydrous magnesium sulfate. The solvent was distilled off under reduced pressure and the residue was triturated with n-hexane to give 23.4 g (61%) of Compound E.

NMR (90MHz, $CDCl_3$) δ(ppm) 1.28 (9H, s), 1.38 (3H, t, J=7.0), 1.4-2.0 (6H, m), 3.3-4.1 (2H, m), 4.36 (2H, q, J=7.0), 5.32 (1H, brs), 7.00 (1H, dd, J=10.7, 9.6), 7.57 (1H, brs)

FAB-MS (M/Z) 386 ($M^+$+1)

Molecular formula $C_{19}H_{25}F_2NO_5$=385

Reference Example 5

Ethyl 3,5-difluoro-6-pivaloylamino-2-(2-tetrahydropyranyloxy)-4-trimethylsilylbenzoate (Compound F)

161 ml (1.15 mol) of diisopropylamine was dissolved in 500 ml of tetrahydrofuran under a stream of nitrogen, 690 ml (1.10 mol) of n-butyl lithium (1.6M solution in n-hexane) was added over 30 minutes while maintaining an internal temperature at from -20 to 0 °C and the mixture was cooled to an internal temperature of not higher than -60 °C. A solution of 156 g (0.50 mol) of 2,4-difluoro-5-pivaloylamino-O-(2-tetrahydropyranyl)phenol obtained in Reference Example 4 dissolved in 900 ml of tetrahydrofuran was added thereto over 40 minutes while maintaining an internal temperature at not higher than -55 °C and the mixture was stirred for 10 minutes. 114 ml (0.90 mol) of trimethylsilyl chloride was added thereto over 15 minutes while maintaining an internal temperature at not higher than -60 °C and the mixture was stirred at the same temperature for 1 hour. 50 ml of water was added to stop the reaction, the temperature was risen to room temperature, 1000 ml of water and 1000 ml of ethyl acetate were further added and the mixture was stirred vigorously. The organic layer was separated, washed once with water and once with an aqueous saturated solution of sodium chloride and dried over anhydrous magnesium sulfate. The solvent was distilled off under reduced pressure to give 189 g (98%) of 2,4-difluoro-5-pivaloylamino-3-trimethylsilyl-O-(2-tetrahydropyranyl)phenol.

NMR (90MHz, $CDCl_3$) δ(ppm) 0.37 (9H, m), 1.31 (9H, s), 1.4-2.1 (6H, m), 3.4-3.8 (2H, m), 5.41 (1H, brs), 7.50 (1H, brs), 8.27 (1H, t, J=8.8)

FAB-MS (M/Z) 386 ($M^+$+1)

Molecular formula $C_{19}H_{29}F_2NO_3Si$=385

268 g (0.70 mol) of the resulting 2,4-difluoro-5-pivaloylamino-3-trimethylsilyl-O-(2-tetrahydropyranyl)phenol was dissolved in 2 ℓ of tetrahydrofuran under a stream of nitrogen and the mixture was cooled to an internal temperature of not higher than -60 °C. 1.0 ℓ (1.6 mol) of n-butyl lithium (1.6M solution in n-hexane) was added thereto at a temperature of not higher than -50 °C over 1 hour. The reaction solution was warmed to -45 °C, stirred for 5 minutes and cooled again to not higher than -60 °C. 133 ml (1.4 mol) of ethyl chloroformate was added thereto at an internal temperature of not higher than -55 °C and the mixture was stirred at the same temperature for 10 minutes. 50 ml of water was added to stop the reaction, the mixture was warmed to room temperature, 1 ℓ of water and 1 ℓ of ethyl acetate were added and the mixture was stirred vigorously. The organic layer was separated, washed once with water and once with an aqueous saturated solution of sodium chloride, dried over magnesium sulfate and the solvent was distilled off under reduced pressure. About 1 ℓ of petroleum ether (b.p.30-60 °C) was added to the solidified residue to form slurry which was cooled on ice and filtered to give 245 g (77%) of Compound F. The mother liquor was concentrated and the similar operations were carried out to give additional 11.4 g (3.6%) of Compound F.

NMR (90MHz, $CDCl_3$) δ(ppm) 0.37 (9H, t, J=1.7), 1.28 (9H, s), 1.37 (3H, t, J=7.0), 1.4-2.1 (6H, m), 3.4-4.1 (2H,

m), 4.33 (2H, q, J=7.0), 5.26 (1H, brs), 7.46 (1H, brs)
FAB-MS (M/Z) 458 ($M^+$+1)
Molecular formula $C_{22}H_{33}F_2NO_5Si$=457

## Claims

1. A compound represented by the formula:

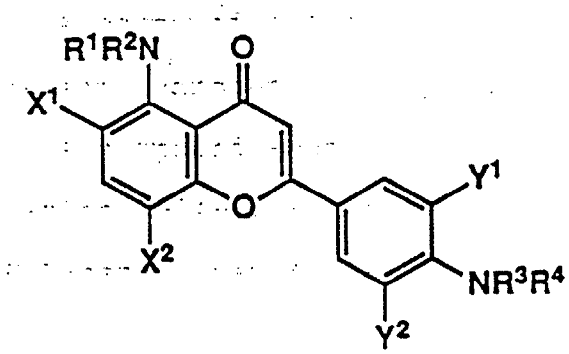

wherein $R^1$, $R^2$, $R^3$ and $R^4$ are the same or different and represent hydrogen, substituted or unsubstituted lower alkyl, lower alkenyl, halogen-substituted or unsubstituted lower alkanoyl or lower alkoxycarbonyl; $X^1$, $X^2$, $Y^1$ and $Y^2$ are the same or different and represent hydrogen or halogen; provided that at least one of $X^1$ and $X^2$ represents halogen.

2. A compound according to claim 1, wherein $X^1$ and $X^2$ are the same or different and represent hydrogen or fluorine provided that at least one of $X^1$ and $X^2$ represents fluorine.

3. A compound according to claim 2, wherein $X^1$ and $X^2$ represent fluorine.

4. A compound according to claim 3, wherein $R^1$, $R^2$ and $R^3$ represent hydrogen.

5. A compound according to claim 4 which is:

5-amino-2-[4-[(3-aminopropyl)amino]phenyl]-6,8-difluoro-4H-1-benzopyran-4-one,
5-amino-2-[4-(3-aminopropylamino)-3-fluorophenyl]-6,8-difluoro-4H-1-benzopyran-4-one,
5-amino-6,8-difluoro-2-[3-fluoro-4-[2-(pyrrolidin-1-yl)ethylamino]phenyl]-4H-1-benzopyran-4-one,
5-amino-6,8-difluoro-2-[3-fluoro-4-[2-morpholinoethylamino)phenyl]-4H-1-benzopyran-4-one,
5-amino-6,8-difluoro-2-[4-[3-(N',N'-dimethylamino)propylamino]-3-fluorophenyl]-4H-1-benzopyran-4-one,
5-amino-6,8-difluoro-2-[3-fluoro-4-[3-(pyrrolidin-1-yl)propylamino]phenyl]-4H-1-benzopyran-4-one,
5-amino-6,8-difluoro-2-[3-fluoro-4-[3-(imidazol-1-yl)propylamino]phenyl]-4H-1-benzopyran-4-one,
5-amino-6,8-difluoro-2-[3-fluoro-4-[6-(imidazol-1-yl)hexylamino]phenyl]-4H-1-benzopyran-4-one,
5-amino-6,8-difluoro-2-[4-[6-(N',N'-dimethylamino)hexylamino]-3-fluorophenyl]-4H-1-benzopyran-4-one,
5-amino-6,8-difluoro-2-[3-fluoro-4-[6-(pyrrolidin-1-yl)hexylamino]phenyl]-4H-1-benzopyran-4-one, or
5-amino-6,8-difluoro-2-[3-fluoro-4-(6-morpholinohexylamino)phenyl]-4H-1-benzopyran-4-one.

6. A compound according to claim 4, wherein $R^4$ represents hydrogen.

7. A compound according to claim 6 which is:

5-amino-2-(4-aminophenyl)-6,8-difluoro-4H-1-benzopyra-4-one,
5-amino-2-(4-amino-3-fluorophenyl)-6,8-difluoro-4H-1-benzopyran-4-one,
5-amino-2-(4-amino-3,5-difluorophenyl)-6,8-difluoro-4H-1-benzopyran-4-one,
5-amino-2-(4-amino-3,5-dichlorophenyl )-6,8-difluoro-4H-1-benzopyran-4-one, or
5-amino-2-(4-amino-3,5-dibromophenyl)-6,8-difluoro-4H-1-benzopyran-4-one.

**8.** A compound according to claim 3, wherein $R^1$ represents hydrogen.

**9.** A compound according to claim 8, wherein $Y^1$ represents fluorine; and $Y^2$ represents hydrogen.

**Patentansprüche**

**1.** Verbindung der Formel:

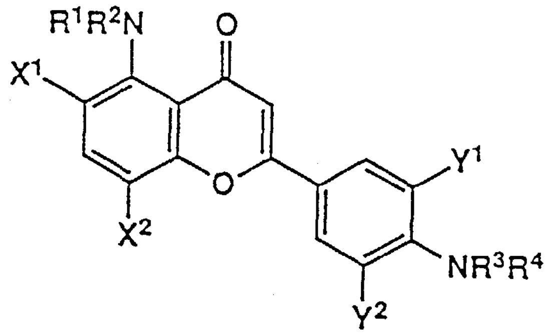

in der $R^1$, $R^2$, $R^3$ und $R^4$ gleich oder verschieden sind und ein Wasserstoffatom, einen substituierten oder unsubstituierten Niederalkylrest, Niederalkenylrest, einen halogensubstituierten oder unsubstituierten Niederalkanoylrest oder Niederalkoxycarbonylrest darstellen; $X^1$, $X^2$, $Y^1$ und $Y^2$ gleich oder verschieden sind und ein Wasserstoffatom oder Halogenatom darstellen; mit der Maßgabe, daß mindestens einer der Reste $X^1$ und $X^2$ ein Halogenatom darstellt.

**2.** Verbindung nach Anspruch 1, in der $X^1$ und $X^2$ gleich oder verschieden sind und ein Wasserstoff- oder Fluoratom darstellen, mit der Maßgabe, daß mindestens einer der Reste $X^1$ und $X^2$ ein Fluoratom darstellt.

**3.** Verbindung nach Anspruch 2, in der $X^1$ und $X^2$ ein Fluoratom darstellen.

**4.** Verbindung nach Anspruch 3, in der $R^1$, $R^2$ und $R^3$ ein Wasserstoffatom darstellen.

**5.** Verbindung nach Anspruch 4, nämlich

5-Amino-2-[4-[(3-aminopropyl)amino]phenyl]-6,8-difluor-4H-1-benzopyran-4-on,
5-Amino-2-[4-(3-aminopropylamino)-3-fluorphenyl]-6, 8-difluor-4H-1-benzopyran-4-on,
5-Amino-6,8-difluor-2-[3-fluor-4-[2-(pyrrolidin-1-yl)ethylamino]phenyl]4H-1-benzopyran-4-on,
5-Amino-6,8-difluor-2-[3-fluor-4-[2-morpholinoethylamino)phenyl]-4H-1-benzopyran-4-on,
5-Amino-6,8-difluor-2-[4-[3-(N',N'-dimethylamino)propylamino]-3-fluorphenyl]-4H-1-benzopyran-4-on,
5-Amino-6,8-difluor-2-[3-fluor-4-[3-(pyrrolidin-1-yl)propylamino]phenyl]-4H-1-benzopyran-4-on,
5-Amino-6,8-difluor-2-[3-fluor-4-[3-(imidazol-1-yl)propylamino]phenyl]-4H-1-benzopyran-4-on,
5-Amino-6,8-difluor-2-[3-fluor-4-[6-(imidazol-1-yl)hexylamino]phenyl]-4H-1-benzopyran-4-on,
5-Amino-6,8-difluor-2-[4-[6-(N',N'-dimethylamino)hexylamino]-3-fluorphenyl]-4H-1-benzopyran-4-on,
5-Amino-6,8-difluor-2-[3-fluor-4-[6-(pyrrolidin-1-yl)hexylamino]phenyl]-4H-1-benzopyran-4-on oder
5-Amino-6,8-difluor-2-[3-fluor-4-(6-morpholinohexylamino)phenyl]-4H-1-benzopyran-4-on.

**6.** Verbindung nach Anspruch 4, in der $R^4$ ein Wasserstoffatom darstellt.

**7.** Verbindung nach Anspruch 6, nämlich

5-Amino-2-(4-aminophenyl)-6, 8-difluor-4H-1-benzopyran-4-on,
5-Amino-2-(4-amino-3-fluorphenyl)-6, 8-difluor-4H-1-benzopyran-4-on,
5-Amino-2-(4-amino-3,5-difluorphenyl)-6,8-difluor-4H-1-benzopyran-4-on,
5-Amino-2-(4-amino-3,5-dichlorphenyl)-6,8-difluor-4H-1-benzopyran-4-on oder
5-Amino-2-(4-amino-3,5-dibromphenyl)-6,8-difluor-4H-1-benzopyran-4-on.

**8.** Verbindung nach Anspruch 3, in der $R^1$ ein Wasserstoffatom darstellt.

**9.** Verbindung nach Anspruch 8, in der $Y^1$ ein Fluoratom darstellt und $Y^2$ ein Wasserstoffatom darstellt.

**Revendications**

**1.** Composé représenté par la formule :

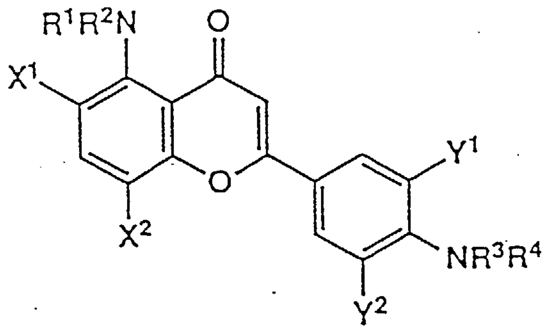

dans laquelle :

- $R^1$, $R^2$, $R^3$ et $R^4$ sont identiques ou différents et. représentent hydrogène, alkyle inférieur substitué ou non-substitué, alcényle inférieur, alcanoyle inférieur substitué par halogène ou non-substitué ou alcoxycarbonyle inférieur;
- $X^1$, $X^2$, $Y^1$ et $Y^2$ sont identiques ou différents et représentent hydrogène ou halogène ; à la condition qu'au moins l'un parmi $X^1$ et $X^2$ représente halogène.

**2.** Composé selon la revendication 1, dans lequel $X^1$ et $X^2$ sont identiques ou différents et représentent hydrogène ou fluor, à la condition qu' au moins l'un parmi $X^1$ et $X^2$ représente fluor.

**3.** Composé selon la revendication 2, dans lequel $X^1$ et $X^2$ représentent fluor.

**4.** Composé selon la revendication 3, dans lequel $R^1$, $R^2$ et $R^3$ représentent hydrogène.

**5.** Composé selon la revendication 4 qui est :

. la 5-amino-2-[4-[(3-aminopropyl)amino]phényl]-6,8-difluoro-4H-1-benzopyran-4-one ;

. la 5-anino-2-[4-(3-aminopropylamino)-3-fluorophényl]-6,8-difluoro-4H-1-benzopyran-4-one ;

. la 5-amino-6,8-difluoro-2-[3-fluoro-4-[2-(pyrrolidin-1-yl)éthylamino]phényl]-4H-1-benzopyran-4-one ;

. la 5-amino-6,8-difluoro-2-[3-fluoro-4-[2-morpholinoéthylamino)phényl]-4H-1-benzopyran-4-one ;

. la 5-amino-6,8-difluoro-2-[4-[3-(N',N'-diméthylanino)-propylamino]-3-fluorophényl]-4H-1-benzopyran-4-one ;

. la 5-amino-6,8-difluoro-2-[3-fluoro-4-[3-(pyrrolidin-1-yl)propylamino]phényl]-4H-1-benzopyran-4-one ;

. la 5-amino-6,8-difluoro-2-[3-fluoro-4-[3-(imidazol-1-yl)propylamino]phényl])-4H-1-benzopyran-4-one ;

. la 5-amino-6,8-difluoro-2-[3-fluoro-4-[6-(imidazol-1-yl)hexylamino]phényl]-4H-1-benzopyran-4-one ;

. la 5-amino-6,8-difluoro-2-[4-[6-(N',N'-diméthylamino)-hexylamino]-3-fluorophényl]-4H-1-benzopyran-4-one ;

- la 5-amino-6,8-difluoro-2-[3-fluoro-4-[6-(pyrrolidin-1-yl)hexylamino]phényl]-4H-1-benzopyran-4-one ; ou
- la 5-amino-6,8-difluoro-2-[3-fluoro-4-(6-morpholinohexylamino)phényl]-4H-1-benzopyran-4-one.

**6.** Composé selon la revendication 4, dans lequel $R^4$ représente hydrogène.

**7.** Composé selon la revendication 6, qui est :

- la 5-amino-2- (4-aminophényl)-6,8-difluoro-4H-1-benzopyran-4-one ;
- la 5-amino-2-(4-amino-3-fluorophényl)-6,8-difluoro-4H-1-benzopyran-4-one ;
- la 5-amino-2-(4-amino-3,5-difluorophényl)-6,8-difluoro-4H-1-benzopyran-4-one ;
- la 5-amino-2-(4-amino-3,5-dichlorophényl)-6,8-difluoro-4H-1-benzopyran-4-one ; ou
- la 5-amino-2-(4-amino-3,5-dibromophényl)-6,8-difluoro-4H-1-benzopyran-4-one.

**8.** Composé selon la revendication 3, dans lequel $R^1$ représente hydrogène.

**9.** Composé selon la revendication 8, dans lequel :

- $Y^1$ représente fluor ; et
- $Y^2$ représente hydrogène.